# EUROPÄISCHE PATENTSCHRIFT

(11) **EP 1 158 995 B1**
(45) Veröffentlichungstag und Bekanntmachung des Hinweises auf die Patenterteilung: **18.06.2003**
(21) Anmeldenummer: 00915172.1
(22) Anmeldetag: 08.03.2000
(51) Int. Cl.: A61K 35/84, C07C 69/732, C07C 59/52, C07C 69/734, C07C 59/64, C07C 69/80, C07C 69/738, C07C 59/84, C07F 9/655, C07F 9/40, C07H 19/067, C07D 501/28, C07D 499/36, C07J 13/00, C07D 498/06, A61P 31/00

(54) **BIOLOGISCH AKTIVE VERBINDUNGEN AUS GANODERMA PFEIFFERI DMS 13239**
BIOLOGICALLY ACTIVE COMPOUNDS OF GANODERMA PFEIFFERI DMS 13239
COMPOSES ISSUS DE GANODERMA PFEIRRERI DMS 13239, A ACTIVITE BIOLOGIQUE

(30) Priorität: 09.03.1999 DE 19911679; 09.03.1999 DE 19911680
(43) Veröffentlichungstag der Anmeldung: 05.12.2001
(73) Patentinhaber: Ganomycin Gesellschaft für Biomedizinische Forschung mbH, 17489 Greifswald (DE)
(72) Erfinder: JÜLICH, Wolf, D-17498 Greifswald (DE); LINDEQUIST, Ulrike, D-17491 Greifswald (DE); JANSEN, Rolf, D-38124 Braunschweig (DE); MOTHANA, Ramzi Institut für Pharmazie, D-17487 Greifswald (DE)
(74) Vertreter: Meyers, Hans-Wilhelm, Dr.Dipl.-Chem.
(86) Internationale Anmeldenummer: EP0002026
(87) Internationale Veröffentlichungsnummer: WO00053207

(56) Entgegenhaltungen:
- EP-A- 0 591 603

## Beschreibung

Die Erfindung betrifft biologisch aktive Verbindungen aus Pilzen der Spezies Ganoderma pfeifferi DSM 13239, Verfahren zu ihrer Herstellung und ihre Verwendung. Aus dem Fruchtkörper und dem Mycel der Spezies Ganoderma pfeifferi DSM 13239 werden Extrakte erhältlich, die antimikrobiell wirksam sind und sich als Konservierungsmittel, für pharmazeutische und kosmetische Zubereitungen, zur Bekämpfung von Infektionen sowie zum Einsatz in der Fischzucht eignen.

### Stand der Technik

Biologisch aktive Verbindungen, die durch Extraktion mit Lösungsmitteln aus bestimmten Pilzen der Gattung Ganoderma erhältlich werden, sind seit langem beschrieben und wurden von uns in einer Monographie zusammengefasst (U. Lindequist: Ganoderma. In: Hagers Handbuch der pharmazeutischen Praxis/Hrsg F. von Bruchhausen, 5. vollständig neubearbeitete Auflage, Springer-Verlag Berlin-Heidelberg New York 1998, Folgeband 2 Drogen A-K (Hrsg W. Blaschek). S. 750-761. Im Folgenden wird auf die dort aufgeführte Literatur Bezug genommen.

Kommerziell verwertete drogenliefernde Arten der Gattung Ganoderma sind bisher ausschließlich *Ganoderma applanatum,* (Ganoderma - applanatum - Fruchtkörper) und *Ganoderma lucidum* (Ganoderma - lucidum - Fruchtkörper). Auf Grund seiner wertvollen Inhaltstoffe wird G. lucidum in verschiedenen asiatischen Ländern (Japan, China, Korea u.a.) in großen Mengen kommerziell auf künstlichem Substrat angebaut (Hager, Zitat 20,25).

Zur Extraktion der Wirkstoffe aus G. lucidum und G. applanatum wurden verschiedene Lösungsmittel herangezogen. Dichlormethan und Ethylacetat wurden bisher nicht verwendet.

Wichtigste Wirkstoffgruppe dieser Pilze sind die Triterpene, Polysaccharide und Sterole. Mehr als 100 Triterpene sind strukturell aufgeklärt. Sie werden u.a. als Ganodersäuren, Ganodermsäuren, Ganoderensäuren, Ganolucidinsäuren, Ganosporersäuren, Lucidensäuren, Ganoderiole, Epoxyganoderiole, Ganoderale, Ganoderole, Lucidone, Ganodermanondiol, Ganodermanontriol und Ganodermatriol bezeichnet. Auf Grund der unterschiedlichen Bezeichnung einer Verbindung in verschiedenen Arbeitsgruppen gibt es zahlreiche Dopplungen und Unklarheiten. Grundkörper ist ein meistens ungesättigtes Lanostan, oft mit zwei konjugierten Doppelbindungen im Ringsystem in den Positionen 7 und 9(11) oder einer Doppelbindung in Position 8, die mit zwei Oxogruppen in den Positionen 7 und 11 in Konjugation steht. Die Säuren besitzen eine Carboxylgruppe am Ende der Seitenkette in Position 26. Bei den Trinor-Triterpensäuren (C27-Verbindungen), den Lucidensäuren, befindet sich die Carboxylgruppe in Position 24. Auch Methylester der Säuren kommen vor. Die Hydroxylgruppen sind teilweise acetyliert oder zu Oxogruppen dehydriert. Bei einigen Vertretern hat ein Abbau der Seitenkette bis auf zwei C-Atome (C23-Verbindungen).

Während der Fruchtkörperentwicklung kommt es zu Veränderungen im Triterpenmuster der Pilze. Im Mycelstadium überwiegen die Ganodersäuren, mit fortschreitender Entwicklung der Fruchtkörper treten zunehmend Lucidensäuren auf (Hager, Zitat 22). Bei den Polysacchariden kann man Glucane, Heteropolysaccharide und protein-gebundene Polysaccharide unterscheiden.

Außerdem wurden in Ganoderma lucidum verschiedene Proteine, Steroide, Nucleotide und einige weitere kleinmolekulare Verbindungen charakterisiert.

Als Hauptsterole wurden Ergosterol und Ergosta-7,22-dienol identifiziert (Hager Zitate 15,56,65). Daneben wurden Ergosterolperoxid, Ergosta-7,22-dien-3β-yl-palmitat 56,66), 6α- und 6β-Hydroxy-ergosta-4,7,22-trien-3-on 52), Ergosta-7,22-dien-3β-yl-linoleat, 5α,8α-Epidioxyergosta-6,22-dien-3β-yllinoleat und Ergosta-7,22-dien-2β-3α,9α-triol 66) identifiziert.

Weitere Inhaltsstoffe sind Adenosin in Konzentrationen ab 40 mg/100 g getrocknete Fruchtkörper (Hager, Zitat 67) und 5'-Deoxy-5'-methylsulfinyladenosin (Hager, Zitat 68).

Aus Extrakten des kultivierten Mycels wurde ein als Ling Zhi-8 (LZ-8) bezeichnetes, aus 110 Aminosäuren bestehendes Polypeptid mit einer Molekülmasse von 12 420 Dalton isoliert, dessen Aminoende acetyliert ist (Hager, Zitat 69,70). Außerdem wurde ein Lectin, bestehend aus 2 Proteinuntereinheiten (Molekülmasse 55 600 und 59 800 Dalton) und einem Kohlenhydratanteil von 2,56%, gefunden (Hager, Zitat 72).

Extrakte von G. lucidum werden seit langem arzneilich verwandt. Die Wirkungen von Extrakten aus *Ganoderma lucidum* sind sehr komplex und vielfältig und bis jetzt nur selten eindeutig einzelnen Wirkstoffen zuzuordnen. Folgende Wirkungen von Extrakten aus G. lucidum gehören zum Stand der Technik.

**Extrakte mit Wirkungen auf das ZNS:** Ein wässriger Extrakt der Fruchtkörper wirkt bei Mäusen zentral hemmend und muskelrelaxierend. 30, 100 und 300 mg des lyophilisierten Extraktes/kg, KG, s.c., supprimieren dosisabhängig die Aktivität der Tiere in einem Laufrad, die lokomotorische Aktivität und den zentral stimulierenden Effekt von Koffein. Die Schmerzschwelle im Wärmeplattentest wird erhöht. Beim Schwanzpresstest kommt es zu analgetischen Effekten. 300 mg/kg verlängern die Zeit bis zum Todeseintritt nach Gabe von Strychnin (1,5 mg/kg, i.p.) oder Koffein (400 mg/kg, i.p.), wobei die durch die Alkaloide induzierten Krämpfe nicht beeinflusst werden (Hager, Zitat 73). Bei Ratten sollen Ganoderma-Extrakte schlaffördernd wirken (Hager, Zitat 74).

**Extrakte mit Wirkungen auf das Herz-Kreislauf-System:** Hexan- und Methanolextrakte der Fruchtkörper (1 mg/ml) reduzieren in vitro signifikant die Kontraktionsrate kultivierter Myocardzellen von Mäusen um 25 bzw. 80%. Der Hexan- und der wässrige Extrakt erhöhen in der genannten Konzentration die Kontraktionsamplitude um ca. 15%. Verantwortliche Verbindungen für den erstgenannten Effekt sind Ganodersäure S , Ganoderal A und Ganodermanontriol. 0,1 mg/ml dieser Verbindung führen zum kompletten Stop der Kontraktionen. Die Erhöhung der Amplitude wird durch Ganodersäure S, Portensterol und Ganodermanontriol bewirkt (Hager, Zitat 5).

In vivo wurde ein lyophilisierter wässriger Extrakt Patienten mit essentiellem Hypertonus (Gruppe I) und Patienten mit mildem bzw. ohne Hypertonus (Gruppe II) p.o. in Form von Tabletten über einen Zeitraum von 6 Monaten (6 Tabletten mit 240 mg Extrakt/Tag) verabreicht. Bei den Patienten der Gruppe I kam es zu einer signifikanten Abnahme des erhöhten Blutdrucks, der von Patienten der Gruppe II wurde nicht beeinflusst. Nebenwirkungen traten nicht auf (Hager, Zitat 76).

Spontan hypertensive Ratten, deren Futter 5% Ganoderma-lucidum-Pulver enthielt, zeigten nach 4-wöchiger entsprechender Ernährung einen gegenüber der Kontrollgruppe signifikant reduzierten systolischen Blutdruck und einen erniedrigten Gesamtcholesterolgehalt in Plasma und Leber (Hager, Zitat 77).

MORIGIWA et al. (Hager, Zitat 39) fanden bei 10 Triterpenen in vitro eine Hemmung des Angiotensin Converting Enzyms und damit eine andere Erklärungsmöglichkeit für die antihypertensiven Effekte. Am stärksten wirksam war Ganodersäure F (IC₅₀ 4,7 x 10 -6 M). Mit einer durchschnittlichen IC₅₀von 10⁻⁵ M waren Ganoderal A, Ganoderol A und B sowie die Ganodersäuren, B, D (C), H, K, S und Y aktiv.

**Extrakte mit Wirkungen auf das Blut:** Ein wässriger Extrakt (50 µl des Extraktes, 5 g des Pilzes wurden mit 300 ml extrahiert) hemmt in vitro die durch Thrombin induzierte Aggregation von Rinderthrombozyten. Als verantwortlicher Wirkstoff wurde Adenosin identifiziert (Hager, Zitat 67). Andererseits weisen Rohextrakte trotz hoher Adenosinkonzentrationen bei HIVpositiven Hämophilie-Patienten keine Antiplatelet-Wirkung auf (Hager, Zitat 78). Weitere Wirkstoffe mit in vitro nachgewiesener thrombozytenaggregationsbeeinflussender Wirkung sind 5-Desoxy-5-methylsulfinyladenosin (Hager, Zitat 79) und Ganodermsäure S. Das Adenosinderivat hemmt in einer Konzentration von 50 µg/ml die ADP-induzierte Aggregation von Kaninchenthrombozyten, nicht jedoch die PAF-stimulierte Aggregation (Hager, Zitat 79). Ganodermsäure S besitzt Membranaktivität und beeinflusst in vitro den Phosphoinositol-Stoffwechsel. In Abhängigkeit von der Konzentration fördert oder hemmt sie in vitro die durch verschiedene Induktoren bewirkte Aggregation humaner Thrombozyten (Hager, Zitat 80,81).

Im Tierexperiment hemmt ein wässriger Extrakt (500 mg/kg) den Abfall von Thrombozyten und Fibrinogen und die Verlängerung der Prothrombinzeit in Ratten, bei denen durch Endotoxineinwirkung disseminierte intravaskuläre Coagulationen erzeugt worden waren. In vitro reduziert dieser Extrakt in Konzentrationen von 500 bis 1000 µg/ml die Wirkung von Thrombin und die Kollagen-induzierte Plättchenaggregation (Hager, Zitat 82).

Außerdem wurde der Einfluss wässriger Extrakte auf die Aggregation von Thrombozyten von 15 gesunden Spendern und 33 Patienten mit Arteriosklerose untersucht. In vitro Gabe des Extraktes zu den Thrombozyten gesunder Menschen reduziert konzentrationsabhängig die Aggregation. In vivo führt die perorale Gabe von 1 g Extrakt 3 mal täglich 2 Wochen lang an die Patienten zu einer signifikanten Hemmung der ADP-induzierten Plateletaggregation (Hager, Zitat 83).

**Extrakte mit Wirkungen auf den Verdauungstrakt:** Einige Triterpene (Lucidensäure A, Lucidensäure D1, Ganodersäure A, Ganodersäure C1, Ganodersäure J, Lucidon A, Lucidon C) schmecken intensiv bitter und dürften somit verdauungs- und appetitsanregende Wirkung besitzen. Der bittere Geschmack von Lucidensäure D1 ist noch in einer Verdünnung von 5 x 10⁻¹⁰ wahrnehmbar (Hager, Zitat 84).

**Extrakte mit Wirkungen auf die Leber:** In vitro wurde eine starke Hemmung der Teilung von humanen Hepatomzellen (PLC/PRF/5 Zellen) und von KB Zellen durch Ganoderaldehyd A ( ED 50 bei den Hepatomzellen 10,99 µg/ml, bei KB-Zellen 9,75 µg/ml) und durch Ergosta-7,22-dien-2β,3alpha, 9alpha-triol (ED ₅₀ bei Hepatomzellen 1,17 mg/ml, bei KB-Zellen 0,89 µg/ml) erzielt (Hager, Zitat 66).

Die Ganodersäuren T,U,V,W,X,Z besitzen in vitro in Dosen von 10⁻⁴ mol ebenfalls cytotoxische Wirksamkeit auf Hepatomzellen (Hager, Zitat 47).

Dagegen wirken die Ganodersäuren R und S in vitro antihepatotoxisch. Sie verringern die galactosamin-induzierte Cytotoxizität in kultivierten Rattenhepatocyten (Hager, Zitat 45).

Wässrige Extrakte der Fruchtkörper (30 und 100 mg/kg, i.p.) vermindern in Ratten die durch Chloroform induzierte Lebertoxizität (Bestimmung von GOT und LDH) (Hager, Zitat 85). Alkoholische Extrakte antagonisieren in Mäusen die ebenfalls durch Chloroform verursachte Lipidakkumulation in der Leber. Darüber hinaus senken sie die Mortalitätsrate der Tiere nach hohen Dosen von Digitoxin und Indomethacin. Sie fördern die Leberregeneration in partiell hepatektomisierten Tieren (Hager, Zitat 6).

Zwei aus dem Mycel durch Alkalibehandlung und Ethanolfällung erhältliche Polysaccharidfraktionen (0,5 bis 5 mg/Ratte/Tag für 4 Wochen) zeigen in Ratten mit experimentell erzeugter Lebercirrhose antifibrotische Effekte (Hager, Zitat 87).

**Extrakte mit Wirkungen auf den Stoffwechsel:** Wässrige und ethanolische Extrakte der Fruchtkörper wurden auf ihren Einfluss auf Glucose- und Insulinspiegel im oralen Glucosetoleranztest in Ratten getestet. Der wässrige Extrakt (50 mg/ 200-250 g schwere Ratte) verminderte den Glucosespiegel im Blut 10 min nach p.o. Glucoseapplikation im Vergleich zu den Kontrollratten signifikant. Der Insulinspiegel war nach 10 min im Vergleich zur Kontrollgruppe ebenfalls reduziert, er lag jedoch 30 und 60 min nach Glucosezufuhr auf einem im Vergleich zur Kontrollgruppe signifikant höherem Niveau. Darüber hinaus reduzierte der wässrige Extrakt die durch i.v. Adrenalingabe ausgelöste Erhöhung des Blutglucosespiegels und die adrenalin-induzierte Upolyse in Rattenadipozyten. Die Glucoseabsorption aus dem kleinen Intestinum wurde nicht beeinflusst. Es wird angenommen, dass der Effekt des wässrigen Extraktes auf einer Verbesserung der Glucoseverwertung im peripheren Gewebe beruht. Die ethanolischen Extrakte waren kaum wirksam (Hager, Zitat 88).

HIKINO et al. (Hager, Zitat 62,63,89)) fanden hypoglykämische Effekte bei den als Ganoderane bezeichneten Polysacchariden. Es bestehen keine Überschneidungen mit den Polysacchariden mit Antitumorwirksamkeit (Hager, Zitat 90). Die aktivste Verbindung ist Ganoderan B. 30 mg/kg der Verbindung, i.p., reduzieren 3 bis 7 Stunden nach Applikation signifikant den Plasmaglucosespiegel in glucose-belasteten Mäusen. In normalen Tieren ist der Effekt nicht so klar ausgeprägt. Die hypoglykämische Wirksamkeit wird zurückgeführt auf eine Erhöhung der Insulinkonzentration im Blutplasma und eine Steigerung des Glucosemetabolismus (Hager, Zitat 63).

Andere Stoffwechselwirkungen betreffen den Cholesterolspiegel im Blut. Wässrige und alkoholische Extrakte von *Ganoderma lucidum* hemmen die Cholesterolakkumulation in kultivierten humanen Aortenintimazellen und die Proliferation der Zellen. Es werden daher antiarteriosklerotische Eigenschaften des Pilzes angenommen (Hager, Zitat 91).

Einige oxygenierte Triterpene mit einer Carboxylgruppe am C-26 (3α,15α-Diacetoxy-5α-Ianosta-7,9(11),24-trien-26-säure, 3α,15α-Dihydroxy-5α-lanosta-7,9(11),24-trien-26-on-säure) werden im Gastrointestinaltrakt von Ratten kaum resorbiert. Sie reduzieren auf diese Weise ähnlich wie Sitosterol die Resorption von mit der Nahrung zugeführtem Cholesterol aus dem Magen-Darm-Trakt und wirken so hypolipidämisch (Hager, Zitat 92,93). Partialsynthetisch veränderte Ganodersäure-Derivate mit 7-Oxo- und 15α-Hydroxygruppen hemmen die 14-Demethylierung während der Biosynthese von Cholesterol aus 24,25-Dihydrolanosterol und sollen ebenfalls in der Lage sein, den Cholesterolspiegel im Blut zu senken (Hager, Zitat 94).

**Extrakte mit Wirkungen auf das Immunsystem:** Wässrige Extrakte der Fruchtkörper zeigen antiallergische Effekte. Sie hemmen in Konzentrationen von 20 bis 500 µg/ml die durch die Verbindung 48/80 (10 µg/ml) induzierte Histaminfreisetzung aus peritonealen Rattenmastzellen um 14 bis 70% 95). In vivo reduzieren sie Symptome des experimentellen Asthmas beim Meerschweinchen (Störungen der Atemrate und des Verhältnisses Expiration/Inspiration), der durch Picrylchlorid induzierten Kontaktdermatitis bei der Maus (Ohrschwellung) und der durch Immunkomplexe verursachten Serumnephritis bei Ratten (Proteinausscheidung, Blutdruck, mikroskopische Veränderungen der Glomeruli). Die p.o. verabreichte Dosis betrug jeweils 500 mg/kg 96).

Die aus einem methanolischen Extrakt der Fruchtkörper isolierten Ganodersäuren C und D hemmen ebenso wie die Ethylacetatphase des Ausgangsextraktes in vitro die durch Concanavalin A oder die Verbindung 48/80 induzierte Histaminfreisetzung aus peritonealen Mastzellen von Ratten. 2 mg/ml der genannten Verbindungen reduzieren die durch Con A induzierte Freisetzung um 85% bzw. 80%, die durch 48/80 veranlasste um 70 bzw. 80% (Hager, Zitat 31).

Ein nur aus den Sporen hergestellter Extrakt (wasserlöslicher Anteil des ethanolischen Extraktes der Sporen) hemmt in Dosen von 50, 100 und 200 mg/kg/Tag an 9 Tagen, i.p., die durch Schaferythrozyten, 2,4-Dinitrochlorobenzen oder allotypische Splenozyten induzierte DTH-Reaktion in Mäusen (Hager, Zitat 97).

Das aus dem Mycel isolierte Protein Ling Zhi-8 wirkt in vitro mitogen auf T-Lymphozyten (Hager, Zitat 98). In Mäusen hemmt es in Dosen von 6,9 mg/kg, 2mal pro Woche, i.p., die durch Rinderserumalbumin ausgelösten anaphylaktischen Reaktionen 99).

Auch ein Chloroform Extrakt aus G. lucidum zeigt eine antiallegische Wirkung ((JP 0630 280 028 AA).

**Extrakte mit Antitumorwirksamkeit:** Extrakte aus G. lucidum sowie die daraus isolierten Ganodersäuren A-Z haben eine Antitumorwirkung (JP 0040304890 AA). Ein lyophilisierter heiß-wässriger Extrakt der Fruchtkörper verlängert nach i.p. Gabe allein oder in Kombination mit Cytostatika (Adriamycin, Methotrexat oder andere) signifikant die Überlebenszeit von syngenen C57BL/6 Mäusen, denen i.p. ein Lewis Lungen Carcinom implantiert worden war. Vorbehandlung der Mäuse mit Cyclosporin hemmte die Aktivität. Cytotoxizität war nicht nachweisbar. 10 mg/kg, i.p., an den Tagen 1,3,5,7 und 9 nach Tumorzellinokulation (Tag 0), verlängerten die Überlebenszeit um 95%. Bei Kombination mit Cytostatika waren die Effekte teilweise noch deutlicher. Die Aktivität wird auf eine Anregung des Immunsystems durch im Extrakt enthaltene Polysaccharide zurückgeführt (Hager, Zitat 100). Ein heiß-wässriger Extrakt der Fruchtkörper reduziert in einer Dosis von 10 mg/kg, i.p., 10 Tage, das Gewicht von Sarcom 180-Tumoren in Mäusen (ICR) um 99%. Bei einem von drei Tieren kam es zur kompletten Regression. Verantwortlich waren Fraktionen mit einer Molekülmasse von mehr als 10000 Dalton. Im Gegensatz zum wässrigen Extrakt waren methanolische Extrakte nach i.p. oder p.o. Appl. nicht wirksam (Hager, Zitat 101). Auch das Wachstum eines Fibrosarkoms in C3H-Mäusen wird durch wässrige Extrakte gehemmt (Hager, Zitat 102,103).

Durch Extraktion mit verschiedenen Lösungsmitteln (heißes Wasser, 3% Ammoniumoxalat-Lösung, 100°C, 5% NaOH, 30°C und 80°C u.a.) aus den Fruchtkörpern erhältliche Polysaccharidfraktionen reduzieren nach i.p. Appl. in Dosen von 5 bis 100 mg/kg in ICR/JCL-Mäusen das Wachstum von s.c. implantierten Sarcom 180-Tumoren (Hager, Zitat 57,58,59). 10tägige i.p. Appl. von 20 mg/kg eines wasser-löslichen Polysaccharids hemmt das Wachstum von soliden Sarcom 180-Tumoren in Mäusen um mehr als 90%. Als für die Wirkung essentielle Struktur wurde ein Glucan mit β-1-3, β-1-4 und β-1-6-Verknüpfungen identifiziert (Hager, Zitat 102). Eine von SONE et al. 1985 isolierte Glucanfraktion führte in Dosen von 10 mg/kg/d, i.p., nach 10 Tagen zur kompletten Regression von Sarcom 180-Tumoren bei vier von fünf Mäusen. Die Antitumorwirksamkeit ist höher bei den stärker verzweigten Glucanen (Hager, Zitat 61). Eine wasserlösliche Polysaccharidfraktion hemmte das Tumorwachstum in BDF1-Mäusen mit Leukämie P388 oder L1210 und verlängerte die Überlebenszeit der Tiere (Hager, Zitat 103).

Der Polysaccharid-Protein-Komplex Lucidan inhibiert nach i.p. Appl. (20 mg/kg/Tag) an ICR-Mäuse das Wachstum von Sarcom 180-Tumoren um ca. 70% 64).

Aus dem Kulturfiltrat wurde ein alkalilösliches Glucan (1-3 verknüpfte Glucoseeinheiten mit 1-2 und 1-6-Verzweigungen) extrahiert, das nach i.p. Appl. in Dosen von 10 mg/kg/Tag an 10 Tagen das Wachstum von Sarcom 180-Tumoren in Mäusen um mehr als 90% hemmt (Hager, Zitat 61).

Nach 5-tägiger wiederholter p.o. Gabe von Polysacchariden an Sarcom 180-tragende Mäuse kommt es zu einer beständigen Erhöhung des Phagozytose-Index (Hager, Zitat 105). Die Sauerstoffradikalbildung in isolierten Makrophagen wird stimuliert (Hager, Zitat 103);

Polysaccharide aus Ganoderma lucidum Extrakten fördern konzentrationsabhängig in der gemischten Lymphozytenkultur die IL-2 Produktion 12 Stunden nach Initiierung der Kultur und steigern die Wiederherstellung von Lyt2+ und L3T4+ Zellen nach 4 Tagen (Hager, Zitat 107); andere Extrakte haben einen Scavening Effekte auf Radikale und damit antioxidative Wirksamkeit (Hager, Zitat 85, 109) und bewirken eine Verzögerung von Alterungsprozessen (Hager, Zitat 60, 112). Eine Förderung der Proliferation und Differenzierung einer humanen leukämischen Monozytenzellinie (U 937) in reife Monozyten/Makrophagen durch 50 µg/ml Polysaccharide nach 24 Stunden Inkubation wurde beschrieben (Hager, Zitat 111); 25 und 50 mg/kg/Tag einer Polysaccharidfraktion, i.p., 4 Tage, restaurieren die Aktivität der DNA-Polymerase α in 24 Monate alten Mäusen auf mehr als die Höhe der Aktivität des Enzyms von nur 3 Monate alten Tieren. Auch die im Vergleich zu jüngeren Tieren reduzierte IL-2 Produktion von Splenocyten und die Aktivität in der gemischten Lymphozyten-Kultur wird in den alten Tieren wieder auf das Niveau der jungen Mäuse erhöht (Hager, Zitat 113).

Ganodersäuren enthaltende Extrakte, die aus Schüttelkulturen von G. lucidum nach Überführung des Mycels in eine stationäre Kultur durch Extraktion erhältlich werden, haben eine Antitumor-Wirkung (JP 4-304890 AA). Als besonders vorteilhaft hat sich herausgestellt, Bestimmte Fraktion aus G. lucidum mit Wasser, andere mit Ethanol zu extrahieren und beide Extrakte zu vereinen (JP 0600222423 AA).

**Extrakte mit antimikrobieller Wirksamkeit:** 0,01 bis 1 mg/ml eines methanolischen Rohextraktes hemmen in vitro die durch die cariogenen Bakterien *Streptococcus mutans* ausgelöste Plaquebildung. (Hada, S., Hattori, M., Namba, T. Pharmakology 113 (1990) 73. Ein aus dem Pileus von Ganoderma lucidum erhältlicher Extrakt hemmt das Wachstum von Staphylokokken ((DE 693 18 921 T 2). Es gehört zum Stand der Technik, Extrakte aus G. lucidum mit Antibiotika zu kombinieren, wobei aber die antimikrobielle Wirksamkeit nicht vorhergesagt werden kann, da häufig auch Antagonismus auftritt (Chemical Abstracts Vol 131, Nr. 182 146).

Eine vermutlich Ganodersäuren enthaltende, als F-III-4 bezeichnete Fraktion eines methanolischen Extraktes der Fruchtkörper reduziert in einer Konzentration von 20 µg/ml die Aktivität der reversen Transkriptase im Kulturüberstand von verschiedenen HIV-infizierten Zellen um 40% und verdient daher weiteres Interesse als potentieller Anti-AIDS-Wirkstoff (Hager, Zitat 114). Ein aus Pilzen der Familien Ganodermataceae, Polyporaceae oder Lycoperdaceae durch Extraktion erhältliches Triterpen hemmt DNA-Polymerase (JP 0090-124690 AA).

**Extrakte mit antiphlogistische Wirksamkeit:** Wässrige Extrakte (2 g/kg, s.c.) wirken signifikant anti-inflammatorisch beim carrageenin-induzierten Ohrödem der Maus (Hager, Zitat 115). Beim crotonöl-induzierten Ohrödem (Mäuse) sind 500 µg/Ohr des Ethylacetatextraktes bei topischer Applikation nach 6 Stunden ebenso effektiv wie 200 µg/Ohr Hydrocortison. Wässrige Extrakte (500 µg/Ohr) waren hier nicht wirksam. Sechs Stunden nach p.o. Appl. (1 Stunde vor der topischen Gabe von Crotonöl) waren 500 mg/kg des Ethylacetat- bzw. des wässrigen Extraktes ebenso stark antiinflammatorisch wirksam wie 18 mg/kg Hydrocortison. Die Wirkung der Ganoderma-Extrakte hielt länger an (24 Stunden) als die von Hydrocortison (Hager, Zitat 23). Die antimikrobielle Wirkung von G. lucidum Extrakten wurden zur Imprägnierung von Textilien genutzt (JP 0110060424 AA). Auch von anderen Basidomyceten z.B. Lentinus eodes wurden nach Autolyse heißwässrige Extrakte erhältlich, die eine Wirksamkeit gegen HIV aufweisen (DE 689 06 245 T 2).

**Extrakte mit radioprotektiver Wirksamkeit:** Kontinuierliche i.p. Appl. eines wässrigen Extraktes von *Ganoderma lucidum* an 6 bis 7 Wochen alte männliche ICR Mäuse vor und nach Bestrahlung der Tiere mit Röntgenstrahlen (500 und 650 cGy) verringerte die schädlichen Auswirkungen der Strahlung und vergrößerte die Zahl der überlebenden Tiere (Hager, Zitat 116).

Ein Vorkommen von Hydrochinonen bei Pilzen der Gattung Ganoderma war bisher nicht bekannt. Hydrochinone sind u.a. Bestandteil der Atmungskette und der Photosynthese und daher in der Natur weit verbreitet. Die Radikalfängereigenschaft der Hydrochinone ist pharmakologisch interessant und wird arzneilich genutzt.

Bei der Totalsynthese der aus Ganordema isolierten Wirkstoffe vom Hydrochinon-Typ kann von folgendem Stand der Technik ausgegangen werden: Die Synthese des als Zwischenprodukt benötigten [2,5-Bis-[(*tert*.butyl-dimethylsilyl)-oxy]-phenyl]-acetaldehyds erfolgt ausgehend von 2,5-Dihydroxyphenethylalkohol nach D. G. Hahngauer (*Tetrahedron Lett.,* **1986,** *27,* 5799-5802).

Bei der Synthese der terpenoiden Seitenketten wurde auf L. Chen; G. B. Gill; G. Pattenden; H. Simonian (*J. Chem. Soc., Perkin Trans*. *1,* **1996,** 31-44), F. Kido; Y. Noda; T. Maruyama; C. Kabuto; A. Yoshikoshi (*J. Org. Chem.,* **1981,** *46*, 4264-4266), M. A. Avery; M. S. Verlander; M. Goodman (*J. Org. Chem.,* **1980,** *45,* 2750-2753), E. J. Corey; M. A. Tius; J. Das *(J. Amer. Chem. Soc.,* **1980,** *102,* 1742-1744), E. J. Corey; A. Venktateswarin (*J. Amer. Chem. Soc.,* **1972,** *94,* 6190-6191), J. A. Marshall; D. G. Cleary (*J. Org. Chem.,* **1986**, *51,* 858-863), T. R. Hoye; M. J. Kurth (*J. Org. Chem.,* **1980,** *45,* 3549-3554), G. Beck; D. Günther (*Chem. Ber.,* **1973,** *106,* 2758-2766) und D. Grassi; V. Lippuner; M. Aebi; J. Brunner; A. Vasella (*J*. *Amer. Chem. Soc.,* **1997,** *119,* 10992-10999) zurückgegriffen.

Zum Stand der Technik gehört ferner die Oxidation von Hydrochinonen zu Chinonen, die mit verschiedenen Oxidationsmitteln erfolgen kann (Owton, W. M., J.Chem Soc., Perkin Trans. 1, 1999, 2409-2420. Es ist ferner möglich, 4-substituierte Phenole als Ausgangsmaterial einzusetzen. Bei der Ruthenium katalysierten Oxidation mit tert.Butyl-hydroperoxid in Ethylacetat erfolgt eine Umlagerung, so dass 2 substituierte Chinone erhalten werden (Owton, W. M., J.Chem Soc., Perkin Trans. 1, 1999, 2409-2420. Von keinem der bisher nach verschiedenen Verfahren hergestellten Hydrochinone und Chinone ist eine Wirksamkeit gegen multiresistente Bakterien bekannt. Das gilt in gleichem Umfang für bisher bekannte Umsetzungsprodukte dieser Hydrochinone und Chinone.

Mono- und Di-Hydrochinonether leiten sich durch Alkylierung bzw. Arylierung vom Hydrochinon ab. Hydrochinonbenzylether werden in der Medizin gegen Hyperpigmentierungen der Haut (Leberflecke, Sommersprossen) eingesetzt (RÖMPP - Chemie-Lexikon, Georg Thieme Verlag Stuttgart - New York, 9. Aufl., 1995). Aus dem u.a. in den Blättern von Arctostaphylos uvae ursi Ericaceae, Bärentraube) vorkommenden Glucosid des Hydrochinons, Arbutin, wird im Organismus unter bestimmten Bedingungen Hydrochinon freigesetzt. Dieses wird für die harndesinfizierende Wirksamkeit der Droge verantwortlich gemacht (E.Teuscher: Biogene Arzneimittel, Wiss. Verlagsgesellschaft mbH Stuttgart 1997). Eine Kopplung von aus Ganoderma-Arten erhältlichen Wirkstoffen mit anderen Antibiotika wurde bisher nicht beschrieben. Prinzipiell sind Kopplungsreaktion an Antibiotika mit dem Ziel der Wirkungsverstärkung durch chemische Umsetzungen bekannt . Eine Veresterung des Ofloxacins mit Ethanol ist bereits bekannt: J. S. Kiely; E. Laborde; L. E. Lesheski; R. A. Busch (*J*. *Heterocycl. Chem.,* **1991,** *28*, 541-543).

Veresterungen von Penicillin G mit einfachen Alkoholen gehören ebenfalls zum Stand der Technik, z.B.: M. Murakami; M. Hajima; F. Takami; M. Yoshioka (*Heterocycles*, **1990,** *31*, 2055-264). Zum Stand der Technik gehört der Einsatz von Enzymen, um eine Umsetzung unter schonenden Bedingungen zu ermöglichen.

Die Synthese von Cephalosporinen erfolgt beispielsweise durch Acylierung von 7-Aminocephalosporinsäure und 7-Aminodesacetoxycephalosporinsäure durch die katalytische Wirkung von Acylasen der Stämme B.megaterium oder E.coli (Fujii, T, Hanamitsu, K, Izumi, R. Yamguchi, T. and Watanabe, T (1973) Japanese Patent 7399393, SNAM, Proetti (1972) Belgian Patent 782646). Lipasen katalysieren die Veresterung einer breiten Palette von Substraten (Ching et al., Angew. Chem. 101 (1989) 711-724). Die Derivatisierung neuer Wirkstoffe aus Ganoderma-Arten kann sich deshalb auf diese zum Stand der Technik gehörende Syntheseprinzipien stützen, um zu prinzipiell neuen Hybrid-Antibiotika zu gelangen.

Das Vorkommen von Ganoderma pfeifferi als Baumpilz ist schon lange bekannt. Aus dem Pilz Ganoderma pfeifferi sind jedoch bisher keine biologisch aktiven Verbindungen bekannt. Extrakte aus G. Pfeifferi wurden bisher in der Literatur nicht beschrieben und wurden schon gar nicht arzneilich eingesetzt. Eine Kultivierung von Ganoderma pfeifferi, in Pilzfarmen bzw. in Laborkulturen wurde bisher nicht beschrieben.

### Nachteile des Standes der Technik

Obwohl erwartet werden konnte, dass auch andere Ganoderma-Arten ähnliche Verbindungen enthalten, wie sie aus G. lucidum isoliert wurden, ist im Gegensatz zur intensiven arzneilichen Nutzung von G. applanatum und insbesondere von G. lucidum eine Nutzung weiterer Ganoderma-Arten und insbesondere von G. pfeifferi bisher nicht möglich gewesen. Der Pilz ist damit öffentlich zugänglich, eine kommerzielle Verwertung der Wildvorkommen ist jedoch bis jetzt nicht erfolgt. Damit blieb das wertvolle Potential dieses Pilzes bisher ungenutzt.

Zu den Antibiotika gehören von Pilzen gebildete Stoffe sowie daraus gebildete Derivate, die Infektionen bei intrasomatischer Anwendung bekämpfen können.

Die Inhaltsstoffe von G. lucidum und G. applanatum sind bisher nicht zur Entwicklung von Antibiotika genutzt worden, obwohl die antimikrobielle Wirksamkeit einiger Extrakte bekannt war. Das ist darauf zurückzuführen, dass die vielfältigen Wirkungen der Extrakte aus G. lucidum nur sehr schwer einem spezifischen Wirkstoff zuzuordnen sind. Gerade die Vielfalt der im Stand der Technik angeführten Wirkstoffe aus G. lucidum erweist sich als ein Hemmschuh bei der Entwicklung eines Antibiotikums, da aus G. lucidum und G. applanatum kein Stoff mit einer dominierenden antimikrobiellen Wirksamkeit isoliert wurde, bei dem die Gewinnung als Einzelstoff bzw. die Totalsynthese aussichtsreich erschien. Die bis jetzt isolierten Wirkstoffe aus G. lucidum bzw. G. applanatum haben als Einzelstoff keine ausreichende antimikrobielle Wirksamkeit. Die Extrakte sind schwer zu standardisieren (JP 0610225649 AA), in ihrer antimikrobiellen Aktivität unbestimmt und deshalb als Antibiotikum ungeeignet.

Der derzeitige Stand der Anforderungen an ein Antibiotikum setzt jedoch einen gut charakterisierten Wirkstoff mit definierter Pharmakokinetik und reproduzierbaren antimikrobiellen Eigenschaften für den Einsatz als Antibiotikum voraus. Diese Voraussetzung waren bisher bei den aus G. lucidum bzw. G. applanatum nicht gegeben. Trotz der intensiven Suche nach für pharmazeutische Zwecke verwertbaren Wirkstoffen aus Pilzen der Gattung Ganoderma ist eine Isolierung von Verbindungen, die auf Grund ihrer Eigenschaften für die Entwicklung von Antibiotika aussichtsreich sind, die Herstellung dieser Wirkstoffe durch chemische Synthese und die Derivatisierung nicht gelungen. Eine Totalsynthese eines aus Pilzen isolierten Wirkstoffes ist zwar für die Entwicklung eines Antibiotikums nicht unerlässlich, durch die Lösung dieser Aufgabe werden jedoch die Voraussetzungen für die Entwicklung eines Antibiotikums wesentlich verbessert.

Durch die zunehmende Resistenzproblematik besteht nach wie vor ein großer Bedarf an Antibiotika, der durch die im Stand der Technik beschriebenen Extrakte und Wirkstoffe nicht gedeckt wird.

Insbesondere bei schweren grampositiven Infektionen sind z.Z. nur noch Glykopeptid-Antibiotika ausreichend wirksam. Auch gegen Antibiotika dieser Stoffgruppe wird jedoch durch Staphylokokken und Enterokokken in zunehmenden Maße eine Resistenz entwickelt. Aber auch gramnegative Erreger z.B. der Gattung Pseudomonas entwickeln in zunehmenden Maß eine Mehrfachresistenz, so dass auch Infektionen mit gramnegativen Keimen in Zukunft klinisch nicht mehr sicher beherrscht werden können. Auch in der Veterinärmedizin werden Infektionen von Nutztieren mit multiresistenten Staphylokokken immer bedrohlicher. Durch eine mögliche Übertragung resistent gewordener Keime auf den Menschen droht eine zusätzliche Gefahr. Darüber hinaus ist die Bekämpfung von Infektionen in vielen anderen Bereichen, z.B. in der Fischzucht bisher nur unbefriedigend gelöst. Die zunehmende Verbreitung von Aufzuchtanlagen hat hier zu großen Schwierigkeiten geführt. Auf Grund der ökologischen Probleme sind bisher nur wenige Mittel für die Behandlung in Fischzuchtanlagen zugelassen. Die Probleme können mit den im Stand der Technik beschriebenen Wirkstoffen nicht gelöst werden.

Zur Überwindung der Resistenzproblematik in der Human- und Veterinärmedizin besteht eine dringende Notwendigkeit zur Entwicklung neuer Antibiotika. Die Aufgabe, die durch die Erfindung gelöst werden sollte; bestand darin, weitere Ganoderma-Arten für eine arzneiliche Verwendung nutzbar zu machen, Extrakte aus diesen zu gewinnen sowie aus diesen Extrakten Wirkstoffe zu isolieren und durch chemische Umsetzungen weiterzuentwickeln. Der Erfindung liegt insbesondere die Aufgabe zugrunde, den durch die zunehmende Resistenzentwicklung von Bakterien gegenüber herkömmlichen Antibiotika bestehenden Handlungsbedarf, insbesondere bei der Therapie von Infektionskrankheiten in der Human- und Veterinärmedizin, durch bisher unbekannte Wirkstoffe aus der Gattung Ganoderma und von diesen abgeleitete Derivate zu decken.

Um die Voraussetzungen für die Entwicklung eines Antibiotikums zu verbessern, sollten Lösungen gefunden werden, um die isolierten Wirkstoffe sowie daraus abgeleitete Produkte auch durch chemische Synthese herzustellen.

Die Aufgabe wurde durch Gewinnung von bisher unbekannten Wirkstoffen aus natürlich vorkommenden Pilzen der Gattung G. pfeifferi, Kultivierung von G. pfeifferi DSM 13239 in Pilzfarmen, Kultivierung von G. pfeifferi DSM 13239 in Fermentern, Herstellung verschiedener Extrakte aus dem kultivierten Pilz, Isolierung antimikrobieller Wirkstoffe aus diesen Extrakten, Herstellung dieser Wirkstoffe durch Totalsynthese sowie Derivatisierung dieser Wirkstoffe gelöst.

Der Pilz der Gattung G. pfeifferi wurde am 11. Januar 2000 von der Internationalen Hinterlegungsstelle DSMZ-Deutsche Sammlung von Mikroorganismen und Zellkulturen GmbH, Mascheroder Weg 1b, D-38124 Braunschweig hinterlegt gemäß den Bestimmungen des Budapester Vertrages. Die Hinterlegungsnummer ist DSM 13239.

Erfindungsgemäß sind biologisch aktive Extrakte aus Ganoderma pfeifferi DSM 13239 durch Behandlung mit Lösungsmitteln erhältlich.

Die erfindungsgemäßen Extrakte sind sowohl aus dem Fruchtkörper natürlich vorkommender Pilze der Spezies Ganoderma pfeifferi als auch aus den Fruchtkörpern von in Pilzfarmen gezüchteter Pilze erhältlich.

Die Extrakte sind vorteilhaft erhältlich aus dem Fruchtkörper der Spezies Ganoderma pfeifferi DSM 13239, wenn die Pilze in Pilzfarmen auf Holz-Substraten kultiviert werden. Damit wird eine bisher nicht verwirklichte technische Lösung zur arzneilichen Verwertung der wertvollen Inhaltsstoffe von G. pfeifferi erreicht.

Besonders vorteilhaft sind die Extrakte erhältlich aus dem Fruchtkörper der Spezies Ganoderma pfeifferi. DSM 13239, wenn die Pilzkultivierung nach Vorbehandlung des Holzes mit zelluloseabbauenden Enzymen erfolgte.

Die erfindungsgemäßen Extrakte sind erhältlich aus dem Mycel der Spezies Ganoderma pfeifferi DSM 13239 nach Anzucht in Fermentern. Damit wird eine weitere technische Lösung zur Gewinnung der Inhaltstoffe von Ganoderma-Arten, Insbesondere von G. pfeifferi DSM 13239 verwirklicht. Extrakte, erhältlich aus dem Mycel der Spezies Ganoderma pfeifferi DSM 13239 nach Anzucht in Fermentern, sind vorzugsweise einsetzbar, da damit eine von der langwierigen Fruchtkörpergewinnung unabhängige Möglichkeit zur Gewinnung biologische wirksamer Extrakte und Verbindungen eröffnet wird. Als vorteilhaft hat sich erwiesen, dass dem Flüssigmedium Ammoniumsuccinat zugesetzt wird. Die Extrakte sind erhältlich aus dem Mycel der Spezies Ganoderma pfeifferi DSM 13239, wobei die Pilze in Flüssigmedien mit Kohlenhydratquellen vorzugsweise in Malzmedium mit einem Gehalt an Malzextrakt von 20-40 g/1000 l und einem Ausgangs-pH von 4,5-7,5 unter Einhaltung eines Beleuchtungs- und Schüttelregimes kultiviert werden. Insbesondere ist vorteilhaft, dem Flüssigmedium Holzextrakt, insbesondere Dekokt aus Buche, allein oder in Kombination mit zeiluloseabbauenden Enzymen zuzusetzen.

Aus dem Fruchtkörper und/oder dem Mycel des natürlich vorkommenden oder nach diesem Verfahren gezüchteten Pilzes können mit Lösungsmitteln unterschiedlicher Polarität Extrakte erhältlich werden. Dabei können bekannte Extraktionsmittel wie kaltes und heißes Wasser, Methanol, Ethanol Aceton, Ethylether eingesetzt werden, besonders vorteilhaft sind jedoch Lösungsmittel, die bei der Extraktion von Ganoderma-Arten bisher nicht eingesetzt wurden wie Dichlormethan und Ethylacetat. Extrakte werden vorteilhaft erhalten durch eine Extraktion des Fruchtkörpers oder des Mycels mit einem liphophilen Lösungsmittel, insbesondere mit Dichlormethan. Erfindungsgemäße Extrakte werden ebenfalls erhalten durch eine Extraktion des Fruchtkörpers und/oder des Kulturmediums und/oder des Mycels mit Ethylacetat. Extrakte, erhältlich durch eine Extraktion mit einwertigen Alkoholen, werden in besonders hohen Ausbeuten gewonnen. Auch hierbei ist der Zusatz von Holzdekokt förderlich.

Extrakte sind durch eine Wasserextraktion zu gewinnen vorzugsweise bei Temperaturen von 10°C-80°C erhältlich, wobei die Extraktion vorteilhaft stufenweise mit Wasser mit ansteigender Temperatur ausgeführt wird.

Es ist möglich, die Extraktion in mehreren Schritten mit Lösungsmitteln unterschiedlicher Polarität durchzuführen. Extrakte werden erhalten durch eine Extraktion des Rückstandes, der bei der Extraktion mit lipophilen Lösungsmitteln erhalten wird.

Vorteilhaft sind Extrakte, erhalten durch eine Extraktion des Rückstandes der Dichlormethanextraktion mit einwertigen Alkoholen.

Extrakte, erhalten durch eine Extraktion des Rückstandes der Dichlormethanextraktion mit einwertigen Alkoholen, können durch Ausschütteln mit Ethytacetat und weiterer Reinigung an Kieselgel mittels Gradienten besonders vorteilhaft mit dem Laufmittel Dichlormethan:Ethylacetat / 4:1 oder an Sephadex weiter gereinigt werden.

Ebenso sind Extrakte erhältlich durch eine Wasserextraktion des Rückstandes der Dichlormethanextraktion und/oder des Rückstandes der Ethanolextraktion erhalten werden.

Aus dem Fruchtkörpern natürlich vorkommender oder in Pilzfarmen angezüchteter Pilze sowie aus dem Mycel der Spezies Ganoderma pfeifferi DSM nach Anzucht in Fermentern erhältliche Extrakte haben sich als biologisch wirksam erwiesen. Ethanolische und wässrige Extrakte aus dem Mycel sind überraschenderweise stark antiviral wirksam. Mit lipophilen Extraktionsmitteln werden Extrakte erhalten, die eine Wirksamkeit gegen grampositive Bakterien aufweisen. Nach Extraktion von Fruchtkörper oder Mycel der erfindungsgemäß kultivierten Stämmen von G. pfeifferi DSM 13239 mit einwertigen Alkoholen werden Extrakte mit einer Wirksamkeit gegen gramnegative Stäbchenbakterien erhalten. Alkoholische Extrakte zeigen insbesondere eine Wirksamkeit gegen multiresistente Bakterien der Gattung Pseudomonas, die mit den Dichlormethanextrakten nicht erreicht wird. Besonders aktive Extrakte werden erhalten durch eine Extraktion des Fruchtkörpers und/oder des Kulturmediums und/oder des Mycels und/oder des Rückstandes von Extraktionen mit Ethylacetat. Vorteilhaft kann der Rückstand von Extraktion mit einwertigen Alkoholen eingesetzt werden. Durch eine weitere Fraktionierung mit einem Dichlormethan-Ethylacetat-Gradienten lassen sich Fraktionen mit hoher antimikrobieller Aktivität gewinnen.

Es war nicht vorhersehbar, dass zum Beispiel aus dem Rückstand der Dichlormethanextraktion mit der ethanolischen Extraktion sowie aus dem Rückstand der Dichlormethanextraktion und der ethanolischen Extraktion mit einer Heißwasserextraktion jeweils ein weiterer Extrakt erhältlich ist, der in der Lage ist, Bakterien zu hemmen.

Die Extrakte können bei verschiedenen Anwendungsgebieten direkt eingesetzt werden. Die Extrakte, die aus Fruchtkörper oder Mycel von kultivierten Stämmen von G. pfeifferi DSM 13239 erhältlich sind, können mit chromatographischen Verfahren weiter verarbeitet werden und daraus können reine Wirkstoffe isolieret, werden.

Es können dabei auch aus G. lucidum oder G. applanatum bekannte Wirkstoffen wie Ganoderol B, Applanoxidinsäure G oder Triterpene gewonnen werden. Überraschenderweise können darüber hinaus aber auch weitere Wirkstoffe wie das bisher unbekannte Triterpern 3,26-Dihydroxylanosta-8,24-dien-7-on isoliert werden, das wir Ganoderon B genannt haben. Triperpene der Zusammensetzung 3,26-Dihydroxy-lanosta-8,24-dien-7-on sind aus den Extrakten von Fruchtkörper und Mycel von G. pfeifferi DSM 13239 durch Extraktion mit Lösungsmittel erhältlich und weisen eine bemerkenswerte biologische Aktivität auf. Ganoderon B besitzt eine antivirale Wirkung gegen Influenca Virus Typ A und hemmt die Lipopolysacharid-Bindung an CD-14-positive Zellen.

Im Rahmen der vielfältigen pharmakologischen Wirkungen der Inhaltsstoffe von G. lucidum und G. applanatum spielen antibakterielle Wirkstoffe bisher nur eine untergeordnete Rolle. Es war daher nicht vorhersehbar, dass aus der Vielzahl der möglichen Extrakte von G. pfeifferi DSM 13239 insbesondere aus dem Fruchtkörper solche mit antimikrobieller Aktivität hergestellt wurden, die Extrakte aus G. lucidum an Wirksamkeit deutlich übertreffen. Völlig überraschend werden biologisch aktive Wirkstoffe der allgemeinen Formel 1 erhältlich, die bisher weder bei anderen Ganoderma-Arten noch bei anderen Pilzen, Pflanzen oder Tieren beschrieben wurden. Die als Ganomycine bezeichneten Verbindungen der allgemeinen Formel 1 sind als Leitstrukturen für die Entwicklung von Antibiotika geeignet.

Wirkstoffe mit der Formel 1 mit den Resten R1-R5 sind mit den von uns entwickelten Anzucht- und Extraktionsverfahren erhältlich, wobei R1, R2 und R3 als Wasserstoff, vorliegen, R5 als CH₃ und CH₂OH und R4 als freie Säure vorliegt, ebenso sind diese Wirkstoffe durch chemische Synthese erhältlich, wobei Derivate erhalten werden, bei denen R1, R2 und R3 als Wasserstoff, Halogen, Alkyl- oder Alkoxygruppe vorliegen, R5 als -CH₂-Aryl-, -CH₂-Alkyl-,-CH₂-O-Aryl-, -CH₂-O-Alkyl-, -CH₂N₃ -CH₂-Carbonsäure-, -CH₂-Aldehyd- oder -CH₂-Alkoholgruppe, -CH₂NR9₂, -CX_{3,} -CHX₂, -CH₂X bzw. -CH₃ oder über Heteroatom mit einem Kohlenhydratderivat verbunden, vorliegt, wobei X = F, Cl, Br und R9 ein Wasserstoffatom, ein Alkyl- oder Arylrest sein kann und R4 so gewählt wird, dass die freie Säure, ein Säurehalogenid, ein -amid, ein Salz oder ein Ester mit aliphatischen oder aromatischen Alkoholen der Verbindung vorliegt und n eine Zahl von 1 bis 10 annimmt.

Wie bei den Nachteilen des Standes der Technik aufgeführt, ist es für die Entwicklung eines Antibiotikums von besonderem Vorteil, wenn der gefundene Naturstoff auch durch Totalsynthese hergestellt werden kann.

Wirkstoffe, die in der Formel 1 mit folgenden Substituenten versehen sind:R1, R2 und R3 = H, F, Cl, Br, I oder O-n-Alkyl ; R4 = MeI, MeII, Ammonium, Alkylammonium, Aryl oder Alkyl R5 = H, Alkyl-, Aryl-, R'OH, CHO, R'CHO, COOH oder R'COOH (R' = Aryl oder Alkyl) können durch chemische Synthese durch Variation des Syntheseweges und der Ausgangskomponenten erhalten werden.

Diese Aufgabe wurde erfindungsgemäß durch verschiedene chemische Verfahren gelöst.

Gegenstand der Erfindung sind insbesondere Verbindungen mit den Formelbildern 1 bis 12. 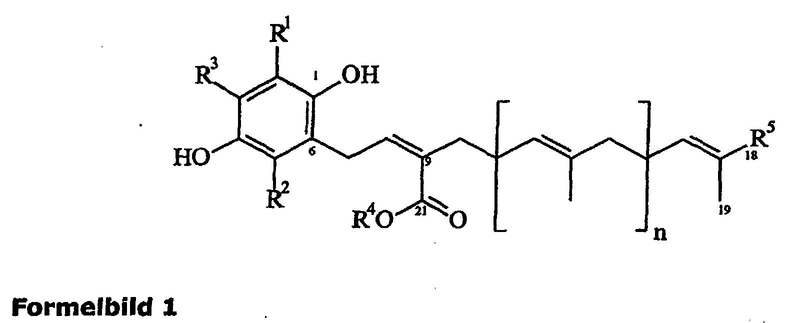 erhältlich aus den erfindungsgemäßen Extrakten mit den Resten R¹-R⁵, wobei R¹, R² und R³ als Wasserstoff, vorliegen, R⁵ als CH₃ und CH₂OH und R⁴ als freie Säure vorliegt, sowie durch chemische Synthese, wobei Derivate erhalten werden, bei denen R¹, R² und R³ als Wasserstoff, Halogen, Alkyl- oder Alkoxygruppe vorliegen, R⁵ als -CH₂-Aryl-, -CH₂-Alkyl-,-CH₂-O-Aryl-, -CH₂-O-Alkyl-, -CH₂-N₃ -CH₂-Carbonsäure-, -CH₂-Aldehyd- oder -CH₂-Alkoholgruppe, -CH₂NR⁹₂, -CX₃, -CHX₂, -CH₂X bzw. -CH₃ oder über Heteroatom mit einem Kohlenhydratderivat verbunden, vorliegt, wobei X = F, Cl, Br und R⁹ ein Wasserstoffatom, ein Alkyl- oder Arylrest sein kann und R⁴ so gewählt wird, dass die freie Säure, ein Säurehalogenid, ein -amid, ein Salz oder ein Ester mit aliphatischen oder aromatischen Alkoholen der Verbindung vorliegt und n eine Zahl von 1 bis 10 annimmt.

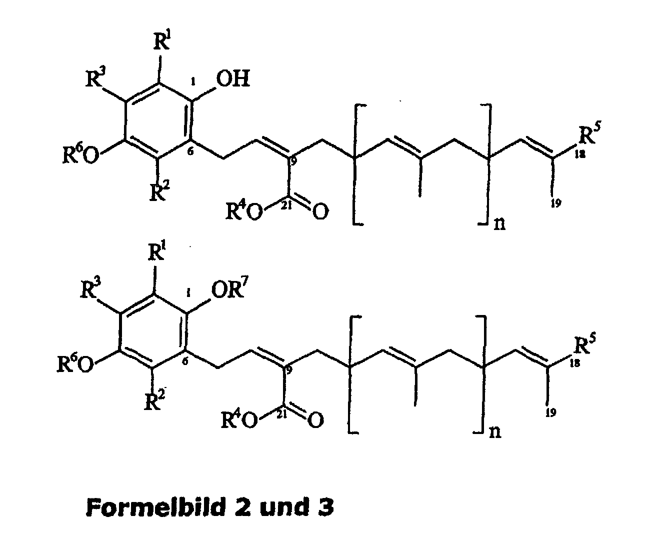

In diesen Verbindungen haben die Reste die weiter oben gegebenen Bedeutungen, wobei R⁶, R⁷ = Aryl, Alkyl ist.

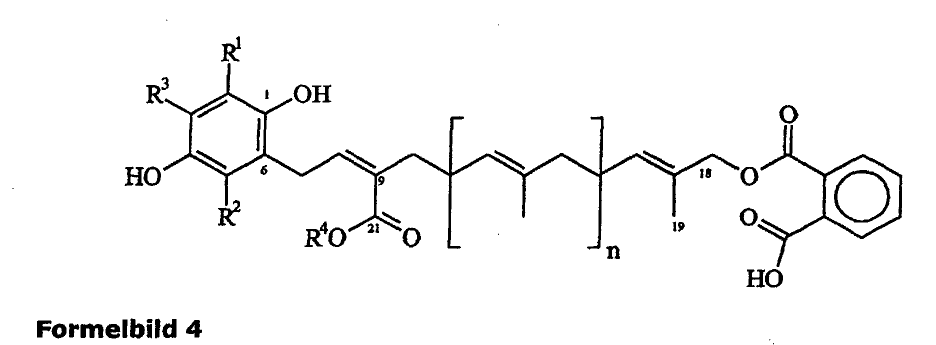

Diese Verbindung betrifft einen Substituenten am C-Atom 18, an dem Phtalsäureanhydrid eingeführt worden ist.

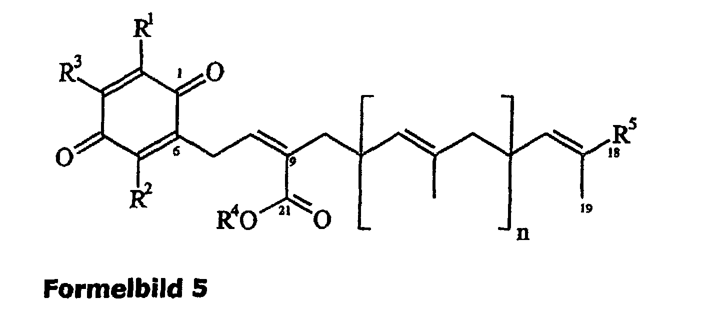

Die erfindungsgemäßen Wirkstoffe können auch In Form von Chinonen vorliegen.

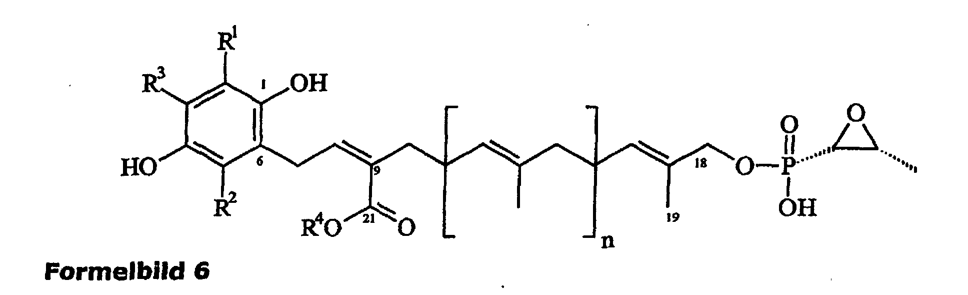

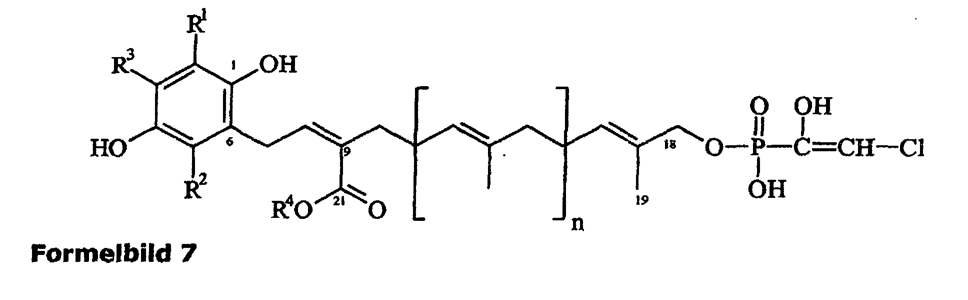

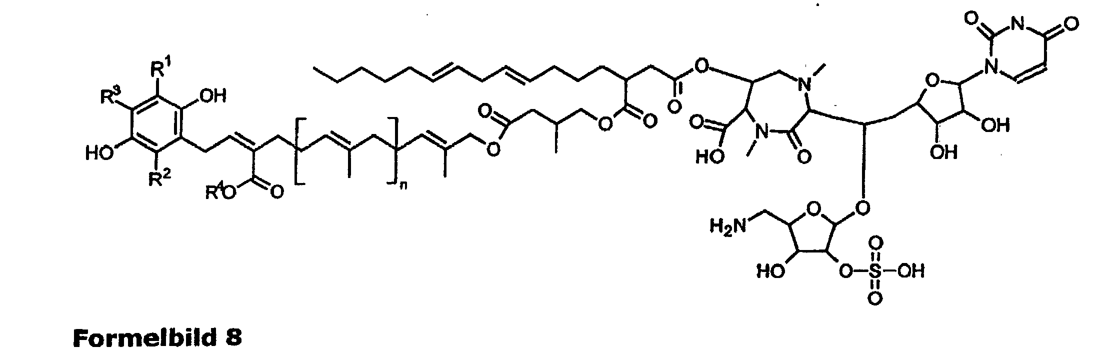

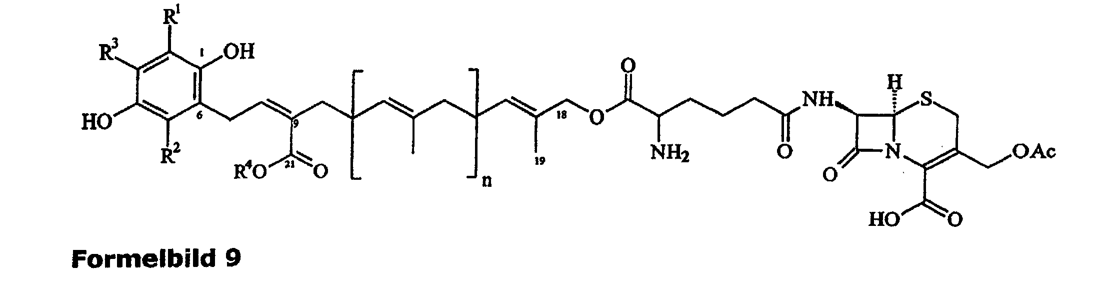

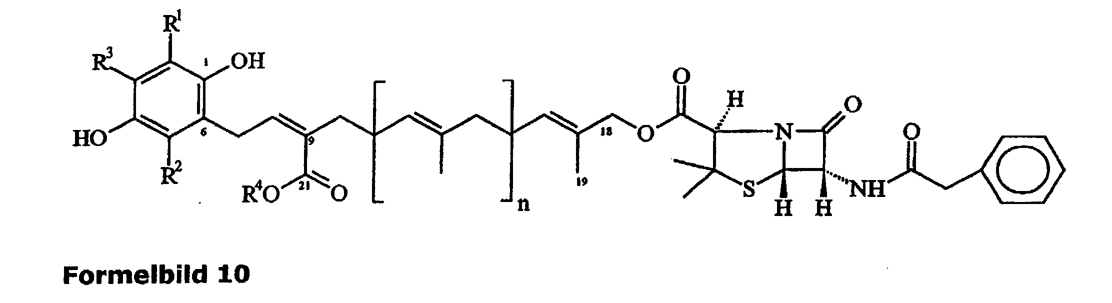

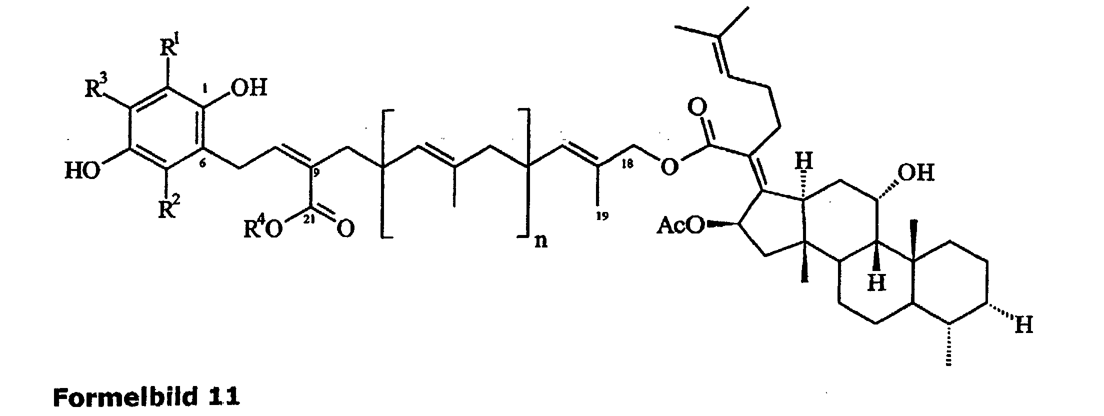

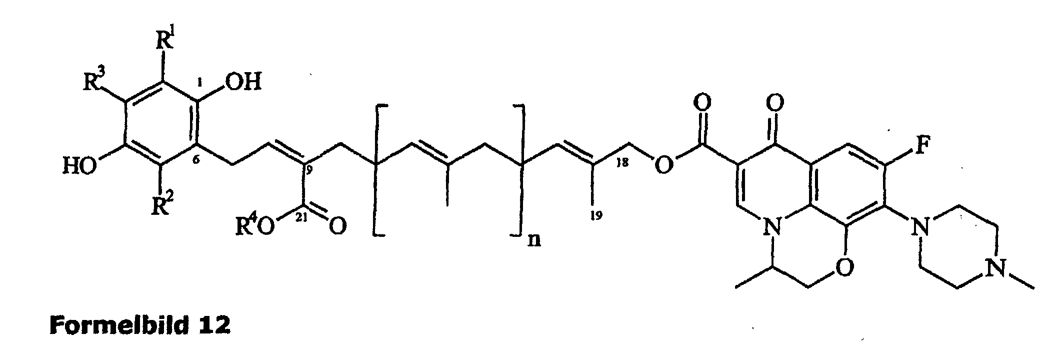

Die Formelbilder 6 bis 12 betreffen bevorzugte Ausführungsformen der erfindungsgemäßen Wirkstoffe konjugiert mit Antibiotika.

Durch Verwendung von substituierten (2,5-Dihydroxyphenyl)-acetaldehyden lässt sich die gefundene antimikrobielle wirksame Grundstruktur an den Stellen R1 bis R3 weiter abwandeln. Eine besondere Wirkungsverstärkung wird durch die Einführung von Halogenen, insbesondere von Fluor, sowie von mit Methoxygruppen am Ringsystem substituierten (2,5-Dihydroxyphenyl)-acetaldehyden erreicht.

Ein Verfahren zur Herstellung von neuen substituierten Hydrochinonen und deren Derivate ist dadurch gekennzeichnet, dass die Kopplung des mit den Resten R1-R3 substituierten des Hydrochinons an die terpenoide Seitenkette ausgehend von halogensubstituierten Hydrochinonen über metallorganische Zwischenstufen durch die Reaktion mit einer endständigen Epoxidfunktion der terpenoiden Seitenkette erfolgt.

Es ist ebenfalls möglich, die Herstellung von neuen substituierten Hydrochinonen und deren Derivaten so vorzunehmen, dass die Kopplung des mit den Resten R1-R3 substituierten des Hydrochinons an die terpenoide Seitenkette ausgehend von halomethyl-substituierten Hydrochinonen über metall-organischen Zwischenstufen durch die Reaktion mit einer Carbonylfunktion der terpenoiden Seitenkette erfolgt.

Eine weitere Möglichkeit zur Herstellung von neuen substituierten Hydrochinonen und deren Derivaten ist dadurch gekennzeichnet, dass die Kopplung des mit den Resten R1-R3 substituierten Hydrochinons an die terpenoide Seitenkette ausgehend von substituierten (2,5-Dihydroxyphenyl)-acetaldehyden durch Umsetzung mit intermediär aus geeigneten terpenoiden erzeugten Caranionen erfolgt.

Die Herstellung von neuen substituierten Hydrochinonen und deren Derivaten kann auch in der Weise erfolgen, dass die Kopplung des mit den Resten R1-R3 substituierten Hydrochinons an die terpenoide Seitenkette ausgehend von substituierten 4-(2,5-Dihydroxy-phenyl)-2-methyliden-carbonsäureestern, welche in 3-Position eine elektronenziehende Abgangsgruppe tragen, durch Umsetzung mit intermediär aus geeigneten Terpenoiden erzeugten Carbanionen erfolgt.

Das Verfahren zur Hersteliung von neuen substituierten Hydrochinonen und deren Derivaten ist dadurch gekennzeichnet, dass die stufenweise Abspaltung von Schutzgruppen aus den synthetisierten Verbindungen mit dem Ziel den freien Wirkstoff zu erhalten oder weitere Funktionalisierungen vorzunehmen, erfolgt.

Durch unterschiedliche α- sulfonierte Carbonylverbindungen können unterschiedliche Seitenketten verwirklicht werden.

Durch Alkylierung bzw. Arylierung der Wirkstoffe gemäß Formel 1 werden Mono (Formelbild 2) - und Di-Hydrochinonether (Formelbild 3) erhältlich, wobei R 6, R 7 = Aryl, Alkyl sein kann. Diese sind ebenfalls biologisch aktiv.

Durch Einführung von Säureanhydriden als Substituenten am C-Atom 18 sind biologisch aktive Verbindungen mit größerer Hydrophilie erhältlich. Bei der Umsetzung mit Phthalsäureabhydrid entsteht der Wirkstoff nach Formelbild 4. Durch Oxidation der Hydrochinone gemäß Formel 1 sind entsprechende biologisch aktive Chinone (Formelbild 5) erhältlich.

Wirkstoffe der allgemeinen Formel 1 können durch Veresterung mit Säuregruppen tragenden Antibiotika verbunden werden. Das kann einerseits auf chemischen Weg unter Nutzung bekannter Umsetzungen erfolgen. Das Wesen der Erfindung liegt an dieser Stelle in der Kombination neuer und bekannter Elemente, wodurch die Auswahl an antimikrobiell wirksamen Verbindungen, die für eine Behandlung von Infektionen mit multiresistenten Keimen geeignet sind, ganz wesentlich erweitert wird. Durch die vielfältigen Umsetzungsmöglichkeiten der Wirkstoffe nach Formel 1 werden ganz neue Wege für die antibiotische Therapie eröffnet. Erfindungsgemäß erfolgt die Veresterung besonders vorteilhaft mittels Biotransformation. Neue antimikrobielle Wirkstoffe nach Formel 5 bis 12 und Verfahren zu ihrer Herstellung, sind dadurch gekennzeichnet, dass Wirkstoffe der allgemeinen Formel 1 durch Kopplung am C 11 Atom mit von Wirkstoffen der allgemeinen Formel 1 mit R 4 = CH₃, die durch Kopplung am C 11 Atom mit Aminogruppen tragenden Antibiotika verbunden werden, da dadurch die Ausbeuten um 20% heraufgesetzt werden können.

Durch Veresterung der aus G. pfeifferi DSM 13239 erhältlichen oder synthetisch hergestellten Hydrochinone der Formel 1 (R5 = CH₂OH) mit cis-1,2-Epoxypropylphosphonsäure (Fosfomycin) lässt sich der Wirkstoff nach Formelbild 6 erhalten, der durch eine Bindung an die UDP-N-Acetyl-D-Glucosaminyl-3-enolpyrovyltransferase besonders fest an der Oberflächen von Staphylokokken gebunden wird. Damit wird einerseits die bakterielle Zellwandsynthese gestört, andererseits wird durch die besonderen Eigenschaften des Hydrochinons das Redoxpotential in der Umgebung der Zelle beeinflusst und damit die Aktivität extrazellulärer Enzyme herabgesetzt.

Durch Kopplung der aus G. pfeifferi DSM 13239 erhältlichen oder synthetisch hergestellten Hydrochinone der Formel 1 (R5 = CH₂OH) mit dem synthetisch herstellbaren Inhibitor der Zellwandsynthese Fosfonochlorin wird ein Wirkstoff nach Formelbild 7 erhalten, der eine besonders intensive Störung der bakteriellen Zellwand bewirkt.

Durch Bindung der aus G. pfeifferi DSM 13239 erhältlichen oder synthetisch hergestellten Hydrochinone der Formel 1 (R5 = CH₂OH) an Liposidomycin wird eine Wirkstoff nach Formelbild 8 erhalten, mit dem selektiv in die bakterielle Peptidoglucansynthese eingegriffen werden kann.

Eine Veresterung der aus G. pfeifferi DSM 13239 erhältlichen oder synthetisch hergestellten Hydrochinone der Formel 1 (R5 = CH₂OH) mit Cephalosporin C führt zu einem Wirkstoff nach Formelbild 9. Die durch Umsetzung mit β-Lactamantibiotika erhaltenen Wirkstoffe nach Formelbild 10 inaktivieren die bakteriellen Penicillinasen und heben bestehende Resistenzen teilweise auf. Ist die Resistenz dadurch eingetreten, dass von der Bakterienzelle Rezeptoren mit besonders hoher Affinität zum Penicillin gebildet wurden, wird eine Bildung der Hydrochinone an der Bakterienzellwand erreicht. Die Hydrochinone können deshalb in unmittelbarer Nähe der Bakterienzelle ihre enzyminhibierenden Eigenschaften entfalten. Da grampositive Bakterien auf die Aktivität extrazellulärer Enzyme angewiesen sind, wird auf diese Weise eine antibakterielle Wirkung erreicht.

Durch Veresterung der aus G. pfeifferi DSM 13 239 erhältlichen oder synthetisch hergestellten Hydrochinone der Formel 1 (RS = CH₂OH) mit Fusidinsäure ist der Wirkstoff 11 erhältlich. Diese Umsetzung bewirkt eine Erhöhung der Oberflächenaktivität und eine Verstärkung der lipophilen Eigenschaften.

Eine Veresterung der Wirkstoffe nach Formel 1, R5 = CH₂OH mit Ofloxacin führt zum Wirkstoff 12, mit dem eine Erweiterung des Wirkungsspektrums erreicht werden kann.

Weiter neue antimikrobieller Wirkstoffe nach Formel 1 werden erhalten, wenn sie am C18 über ein Heteroatom mit Kohlenhydratderivaten verbunden werden. Durch Umsetzung von Methyl-2,3,4-tri-O-benzyl-6-desoxy-6-iod-β-Dglucopyranosid wird 11-(Methyl-2,3,4-tri-O-benzyl-6-desoxy-b-D-glucopyranos -6-yl)-oxy- (2(1')Z,5E,9E)-2-[2'-(2,5-Bis-hydroxy-phenyl)-ethyliden]-6,10-dimethyl-5,9-undecadiensäure erhältlich, die auf Grund des Kohlenhydratanteils Wechselwirkungen mit der Bakterienzellmembran eingeht.

Es ist deshalb möglich, die Eigenschaften der Ganomycine der allgemeinen Formel 1 durch Biotransformation und/oder chemische Derivatisierung an die vorgesehene Verwendung anzupassen.

Die Verwendung einer oder mehrere der Verbindungen der Formel und/oder Extrakte gemäß Beispiel 1 als Radikalfänger ist sowohl in der Konservierungstechnologle als auch bei verschiedenen Erkrankungen möglich. Ebenso ist die Verwendung einer oder mehrere der Verbindungen der Formel 1 und/oder Extrakte gemäß dem Anspruche 1 zur Hemmung der Aktivität von neutralen Endopeptidasen und/oder des Anglotensin-Konverting-Enzyms bei verschiedenen Erkrankungen möglich, da die Proteasen an zahlreichen posttranslationalen Prozessen der Funktionsregelung des Säugertierorganismus betefligt sind. Durch eine Hemmung dieser Enzyme wurden antiinflammatorische, immunstimulierende, analgetische, antihypertensive und antivirale Aktivitäten erreicht.

Die Verwendung einer oder mehrere der Verbindungen der Formel und/oder Extrakte gemäß Beispiel 1 zur Hemmung der serumvermittelten Bindung von Lipopolysachariden bewirkt eine Fiebersenkung unter eine Normalisierung der Weitstellung peripherer Blutgefäße bei bestimmten Krankheitsbildern.

Die Verwendung biologisch aktiver Extrakte und/oder Verbindungen aus Pilzen der Gattung Ganoderma als Substanzen und als Extrakte mit antimikrobieller Wirksamkeit insbesondere als Konservierungsmittel für technische Zwecke nutzt vorwiegend die antimikrobiellen Eigenschaften.

Die Verwendung biologisch aktiver Extrakte und/oder Verbindungen aus Pilzen der Gattung Ganoderma als Substanzen und als Extrakte als Zusatzstoff für pharmazeutische und kosmetische Zubereitungen, insbesondere als Konservierungsmittel, nutzt die günstigen Kombination von antimikrobieller Wirksamkeit, Radikalfängereigenschaft, Hemmung der Proteasen und der daraus resultierenden antiinflammatorischen Wirksamkeit.

Die Verwendung biologisch aktiver Extrakte und/oder Verbindungen aus Pilzen der Gattung Ganoderma als vitalisierender und keimmindemder Zusatzstoffe für Lebensmittel, als Nahrungsergänzungsmittel und als Gesundheitspflegemittel nutzt ebenfalls die Kombination günstiger Eigenschaften und bewirkt darüber hinaus eine Linderung chronischer Schmerzen.

Die Verwendung biologisch aktiver Extrakte und/oder Verbindungen aus Pilzen der Gattung Ganoderma zur Anwendung in der Human- und Veterinärmedizin insbesondere bei der Bekämpfung von Infektionen als alleiniger Wirkstoff sowie in Form von Kombinationspräparaten löst Probleme die in jüngster Zeit bei der Bekämpfung multiresistenter Bakterien aufgetreten sind. In der Humanmedizin gestaltet sich die Bekämpfung schwerer grampositiver Infektionen, die bis zum Tod infolge einer Sepsis führen können, zunehmend schwieriger. Es ist deshalb von besonderem Vorteil, aus einer breiten Palette antimikrobiell aktiver Verbindungen auswählen zu können. Die Erweiterung der Palette antimikrobiell wirksamer Substanzen durch Derivatisierung der Wirkstoffe nach Formel 1 ist daher sehr wichtig, um für die Human- und Veterinärmedizin Mittel mit antimikrobieller Wirkung und unterschiedlichen Anwendungseigenschaften zur Verfügung zu stellen. Das gilt insbesondere für eine Verwendung bei multiresistenten grampositiven Keimen. In der Veterinärmedizin spielen Staphylokokkeninfektionen vor allem in der Rinderhaltung eine große Rolle, da die Keime beim Melken leicht übertragen werden und die Milch kontaminieren können. Die Anwendung der Wirkstoffe nach Formel 1 ist sowohl systemisch als auch lokal möglich.

Die Verwendung als Mittel gegen fischpathogene Bakterien und zum Einsatz in der Fischzucht ergibt sich aus der nachgewiesenen Wirksamkeit gegen fischpathogene Keime.

Aus dem bisher dargelegte ergibt sich als besonderer Vorteil die Verwendung als Mittel mit antimikrobieller Wirkung und als Radikalfänger. Die Verwendung ist dadurch gekennzeichnet, dass die Extrakte und die daraus isolierten Verbindungen allein sowie in Kombination untereinander eingesetzt werden. Auf den nachgewiesen biologischen Wirkungen basiert die erfindungsgemäße Anwendung als Arzneimittel, die eine oder mehrere der Verbindungen und/oder Extrakte enthalten. Zubereitungen, enthaltend eine oder mehrere der Verbindungen und/oder Extrakte, können insbesondere zur Herstellung eines Arzneimittels zur Behandlung von grampositiven Infektionen und der durch die Infektion bedingten Sepsis eingesetzt werden. Aus der spezifischen inhibierenden Wirkung der Extrakte aus G. pfeifferi DSM 13239 gegenüber der neutralen Endopeptidase kann ein Einsatz bei der Behandlung von Schmerzen abgeleitet werden, indem sie mit dem Abbau von opinoiden Peptiden und Endorphinen, die an den Opiat-Rezeptoren angreifen, interferieren. Andererseits bewirkt eine Hemmung der neutralen Endopeptidase eine Hemmung des Abbaus der eine Unterdrückung der Aldosteronsynthese und eine Steigerung der renalen Natriumausscheidung. Damit können Extrakte von G. pfeifferi DSM 13239 bei der Behandlung des Bluthochdrucks eingesetzt werden. Die Verwendung einer oder mehrere der Verbindungen und/oder Extrakte erhältlich aus G. pfeifferi DSM 13239 zur Hemmung der Aktivität von neutralen Endopeptidasen führt zu einer bedeutenden Erweiterung der Einsatzmöglichkeiten. Deshalb ist die Verwendung einer oder mehrere der erfindungsgemäßen Verbindungen und/oder Extrakte zur Herstellung eines Arzneimittels zur Behandlung von Bluthochdruck, Herz- Kreislauferkrankungen und Stoffwechselstörungen möglich.

Die Erfindung soll nachstehend an einigen Beispielen erläutert werden, ohne sich auf diese Beispiele zu beschränken.

### Ausführungsbeispiele

### Beispiel 1

### Vergleichende Untersuchung von Extrakten, erhältlich aus Ganoderma-Arten durch Behandlung mit Lösungsmitteln

Methodik: Fruchtkörper von G. pfeifferi wurden auf Fagus in Ludwigsburg (Mecklenburg-Vorpommern), G. lucidum in Hamburg-Flottbeck auf Querus, G. applanatum in Greifswald (Mecklenburg-Vorpommem) auf Fagus und G. canosum in *Görlitz (Sachsen)* auf Tsuga gesammelt.

Die frisch geernteten Fruchtkörper wurden gesäubert, in kleine Stücke zerschnitten und an der Luft bei Raumtemperatur getrocknet, in einer Schlagmühle pulverisiert und in einer Schlagmühle bis zur Verwendung aufbewahrt. Es wurden Extrakte der Fruchtkörper hergestellt, die vergleichend auf biologische Aktivität geprüft wurden.

Dabei wurde in der ersten Prüfstufe der Agardiffusionstestes eingesetzt. Die zu testenden Extrakte bzw. Substanzen wurden jeweils mit entsprechenden Lösungsmitteln (Dichlormethan, Methanol und Wasser) aufgelöst und auf Filterpapierplättchen in verschiedenen Konzentration aufgetragen. Zur Kontrolle dienten Vergleichsplättchen, die mit 25 µl des entsprechenden Lösungsmittels durchtränkt wurden. Als Referenzsubstanz wurde Ampicillin (10 µg/Plättchen) eingesetzt. Die getrockneten Filterpapierplättchen wurden abschließend auf Agarplatten aufgelegt. Für das Grundscreening wurde Nähragar II (VEB Immunpräparate, Berlin, G), für die Prüfung an aus Patientenmaterial isolierten Stämmen Mueller-Hinton-II-Agar (Becton Dickinson Microbiology Systems, Cockeysville, USA) eingesetzt. Mit einer Impföse wurde eine stecknadelkopfgroße Menge an Testkeimkultur in 3 ml einer sterilen 0,9%igen NaCl-Lösung suspendiert. Anschließend wurden 200 µl der Keimsuspension zu 20 ml sterilen, verflüssigten und wenig warmen Nähragar zugesetzt und in Petrischalen mit einem Durchmesser von 10 cm ausgegossen. Nach Auflegung der Plättchen auf dem festen Agar wurden die Platten für ca. 2 bis 3 Stunden bei 8°C vorinkubiert. Danach erfolgte eine Inkubation der Platten im Inkubationsschrank bei 37°C für 24 Stunden. *Micrococcus flavus* bildete eine Ausnahme. Diese Kultur wurde nur bei Raumtemperatur gelagert, da der Keim sein Wachstumsoptimum bei 20°C erreicht. Die Auswertung der Platten erfolgte durch Ausmessen der Durchmesser der Hemmhöfe. Sämtliche Arbeiten erfolgten unter aseptischen Bedingungen.

Ergebnisse: Extrakte mit Lösungsmittel unterschiedlicher Polarität erhältlich aus G. pfeifferi zeigen bei der vergleichenden Untersuchung eine deutlich höhere Aktivität als die Extrakte anderer Ganoderma-Arten (Tab. 1-4).

**Tab. 1**

| Vergleich der antibakteriellen Wirksamkeit von Dichlormethan-Extrakten | | | | |
|---|---|---|---|---|
| Prüfkeim | Hemmhofdurchmesser in mm | | | |
| | G. pfeifferi | G. lucidum | G. applanatum | G. carnosum |
| S. aureus | 26 | 11 | 14 | 13 |
| B. subtilis | 21 | 11 | 13 | 12 |
| M. flavus | 30 | 13 | 14 | 13 |
| E. coli | 10 | -¹ | - | - |
| P. mirabilis | 18 | - | - | - |
| S. marcescens | 22 | - | - | - |

| | | | | |
|---|---|---|---|---|
| ¹Keine antibakteriellle Aktivität | | | | |

**Tab. 2**

| Vergleich der antibakteriellen Wirksamkeit von ethanolischen Extrakten | | | | |
|---|---|---|---|---|
| Prüfkeim | Hemmhofdurchmesser in mm | | | |
| | G. pfeifferi | G. lucidum | G. applanatum | G. carnosum |
| S. aureus | 24 | 12 | -¹ | 16 |
| B. subtilis | 15 | 11 | - | 14 |
| M. flavus | 18 | 14 | - | 17 |
| E. coli | 8 | - | - | - |
| P. mirabilis | 17 | - | - | - |
| S. marcescens | 22 | - | - | - |

| | | | | |
|---|---|---|---|---|
| ¹Keine antibakteriellle Aktivität | | | | |

**Tab. 3**

| Vergleich der antibakteriellen Wirksamkeit von kaltwässrigen Extrakten | | | | |
|---|---|---|---|---|
| Prüfkeim | Hemmhofdurchmesser in mm | | | |
| | G. pfeifferi | G. lucidum | G. applanatum | G. carnosum |
| S. aureus | 12 | - | - | - |
| B. subtilis | - | - | - | - |
| M. flavus | 8 | - | - | - |
| E. coli | - | - | - | - |
| P. mirabilis | 9 | - | - | - |
| S. marcescens | - | - | - | - |

**Tab. 4**

| Vergleich der antibakteriellen Wirksamkeit von heißwässrigen Extrakten | | | | |
|---|---|---|---|---|
| Prüfkeim | Hemmhofdurchmesser in mm | | | |
| | G. pfeifferi | G. lucidum | G. applanatum | G. camosum |
| S. aureus | 32 | 16 | 17 | 10 |
| B. subtilis | 16 | 14 | 15 | 13 |
| M. flavus | 18 | 17 | 17 | 12 |
| E. coli | 12 | - | - | - |
| P. mirabilis | 25 | - | 11 | - |
| S. marcescens | 25 | - | - | - |

### Beispiel 2

### Extrakte, erhältlich aus dem Fruchtkörper der Spezies Ganoderma pfeifferi DSM 13239.

Methodik: Pulverisierte Fruchtkörper wurden in Extraktionshülsen gefüllt und mit Dichlormethan (E. Merck, Darmstadt) in einer Soxhlet-Apparatur bis zur völligen Entfärbung extrahiert. Das war in der Regel nach 24 h der Fall. Der Pilzrückstand wurde nach Verdunsten von Dichlormethan-Resten 5mal je 2 h mit 80%igem Ethanol (E. Merck, Darmstadt) unter ständigem Schütteln bei Raumtemperatur extrahiert. Der Pilzrückstand wurde mit einem Büchner-Trichter abgetrennt und an der Luft getrocknet. Anschließend wurde mit destilliertem Wasser 5mal je 2 h bei Raumtemperatur geschüttelt, die Abtrennung des Pilzrückstandes erfolgte wiederum mittels Büchner-Trichter. Der Rückstand des kaltwässrigen Extraktes wurde 3mal mit destilliertem Wasser bei 70°C extrahiert und anschließend filtriert.

Alle Extrakte wurden im Vakuumrotationsverdampfer (Büchi Labortechnik AG, Flawil, Schweiz) bei 40°C eingeengt und anschließend lyophilisiert (Gefriertrocknungsanlagen GmbH, Osterode, G). Sie standen in dieser Form für umfangreiche Testungen auf biologische Aktivität, die in den nachfolgenden Beispielen beschrieben werden, zur Verfügung.

Ergebnis: Durch Extraktion mit Lösungsmittel unterschiedlicher Polarität können aus dem Fruchtkörper von G. pfeifferi lagerfähige biologisch aktive Extrakte gewonnen werden.

### Beispiel 3

### Extrakte, erhältlich aus dem Fruchtkörper der Spezies Ganoderma pfeifferi DSM 13239, wobei die Pilze in Pilzfarmen auf Holz-Substraten kultiviert werden

Methodik: Aus frisch geemteten jungen Fruchtkörpern der Spezies G. pfeifferi wurden aus dem Bereich des Übergangs vom Hut zum Stiel mit einer sterilen Pinzette Gewebestücke entnommen und bei Zimmertemperatur auf Hagem-Agar kultiviert. Nachdem der Pilz sichtbares Wachstum zeigte, wurden einige Mycelstücke ausgestanzt und für 2-3 Wochen in ein Hagem-Flüssigmedium überführt. Die Herstellung der Medien erfolgte nach den Vorschriften von Kreisel und Schauer (Methoden des mykologischen Laboratoriums, Fischer-Verlag Jena 1987). Mit dem auf diese Weise gewonnenen Mycel wurden sterilisierte Buchenholzspäne als Substrat infiziert. Nach einer Kultivierungszeit von 8 Monaten bei 20 bis 23°C in geeigneten Behältnissen konnten erste Fruchtkörper gewonnen werden. Die Fruchtkörper wurden entsprechend dem Beispiel 2 aufgearbeitet und auf biologische Aktivität untersucht.

Ergebnis: Die Kultivierung der Fruchtkörper von G. pfeifferi auf geeignetem Holzsubstrat ist möglich. Aus den auf diese Weise erhältlichen Fruchtkörpern können biologisch aktive Extrakte gewonnen werden.

### Beispiel 4

### Extrakte, erhältlich aus dem Fruchtkörper der Spezies Ganoderma pfeifferi DSM 13239, wobei die Pilzkultivierung nach Vorbehandlung des Holzes mit zelluloseabbauenden Enzymen erfolgte

Methodik: Buchen-Späne wurde für 24 h mit einer Mischung Zellulose abbauender Enzyme (Affina GmbH Berlin, Germany) inkubiert. Danach erfolgte die Beimpfung mit G. pfeifferi analog dem Beispiel 3.

Ergebnis: Der schwierige Schritt des Anwachsens in dem Substrat wird durch die Vorbehandlung mit Zellulose abbauenden Enzymen verbessert. Damit wird eine technische Lösung geschaffen, die Kultivierung schwer anzüchtbarer Pilze erleichtert. Die biologische Aktivität der Extrakte wird durch die Vorbehandlung nicht beeinflusst.

### Beispiel 5

### Extrakte, erhältlich aus dem Mycel der Spezies Ganoderma pfeifferi DSM 13239 nach Anzucht in Fermentern

Methodik: Frisch geerntete junge Fruchtkörpern der Spezies G. pfeifferi wurden gereinigt und unter sterilen Bedingungen aufgebrochen. Aus dem Bereich des Übergangs vom Hut zum Stiel wurden mit einer sterilen Pinzette Gewebestücke entnommen und bei Zimmertemperatur auf Hagem-Agar kultiviert. Nachdem der Pilz sichtbares Wachstum zeigte, wurden einige Mycelstücke ausgestanzt und mit einem Hagem-Flüssigmedium im Bühler-Homogenisator (Edmund Bühler, Tübingen, G) zerkleinert. Anschließend erfolge die weitere Kultur in 250 ml Erlenmeyer-Kolben mit Hagem-Flüssigmedium nach H. Kreisel und F. Schauer (Methoden des mykologischen Laboratoriums, Fischer-Verlag Jena 1987). Jeweils 100 ml Kulturmedium wurden mit 10 ml der wie oben beschreiben hergestellten Impfsuspension versetzt. Die Kultivierung erfolgte bei Raumtemperatur und konstanten Lichtverhältnissen für 30 Tage, wobei eine kontinuierliche Durchmischung mittels einer Schüttelmaschine (Innova 2100, New Brunswick Scientific Co, INC. EDISON, New Jersey, USA) mit einer Geschwindigkeit von 125 U/min erreicht wurde. Die weitere Maßstabsvergrößerung erfolgte durch Überführung in einen Bioreaktor *von* 10 I Volumen. Der Fermenter wurde mit 6 I autoklaviertem HAGEM-Medium (flüssig) versetzt. Dieses wurde mit 60 ml Impfsuspension versetzt. Die Kuttivierung im Fermenter erfolgte bei Raumtemperatur unter ständiger Begasung mit steriler Luft, kontinuierlichem Rühren und im Tag-Nacht-Rhythmus.

Zur Bestimmung des Myceltrockengewichtes wurde das Mycel durch Filtration vom Medium getrennt, in tarierte Kristallisierschalen überführt, analog Beispiel 2 lyophilisiert und das Trockengewicht auf einer Feinwaage (Sartorius, Göttingen, G) ermittelt. Die getrockneten Mycele wurden in Extraktionshülsen gefüllt und mit Dichlormethan (E. Merck, Darmstadt) in einer Soxhlet-Apparatur 24 h extrahiert. Der Mycelrückstand wurde nach Verdunsten von Dichlormethan-Resten 3mal je 2 h mit 80%igem Ethanol (E. Merck, Darmstadt) unter ständigem Schütteln bei Raumtemperatur extrahiert. Der Mycelrückstand wurde mit einem Büchnertrichter abgetrennt und an der Luft getrocknet. Anschließend wurde mit destilliertem Wasser 3mal je 2 h bei Raumtemperatur geschüttelt, die Abtrennung des Pilzrückstandes erfolgte wiederum mittels Büchner-Trichter. Der Rückstand des kaltwässrigen Extraktes wurde 3mal mit destilliertem Wasser bei 70°C extrahiert und anschließend filtriert.

Alle Extrakte wurden im Vakuumrotationsverdampfer (Büchi Labortechnik AG, Flawil, Schweiz) bei 40°C eingeengt und anschließend lyophilisiert (Gefriertrocknungsanlagen GmbH, Osterode, G). Sie standen in dieser Form für umfangreiche Testungen auf biologische Aktivität, die in den nachfolgenden Beispielen beschrieben werden, zur Verfügung.

Ergebnis: Die Kultivierung von G. pfeifferi ist mit Flüssig-Medium möglich. Eine Kultivierung im 10 l-Fermenter erlaubt die Gewinnung von Mycel in größeren Mengen.

Die Ausbeute beträgt durchschnittlich 5 g Mycel/l. Der kultivierte Pilz wurde unter der Nummer DSM 13239 bei der Deutschen Sammlung von Mikroorganismen und Zellkulturen GmbH, Maschenroder Weg I b, D-38124 Braunschweig hinterlegt.

### Beispiel 6

### Verbesserung der Kultivierungsbedingungen durch Zusatz von Ammoniumsuccinat vorzugsweise in Konzentrationen von 0,1 bis 1%

Methodik: Durchführung der Versuche nach Beispiel 5, aber unter Zusatz von 0,1-1% Ammoniumsuccinat.

Ergebnis: Steigerung der Ausbeute an Mycel von 0,34 + 0,0568 g /100 mlauf 0,691 + 0,048 g/100 ml durch Zusatz von 0,5% Ammoniumsuccinat.

### Beispiel 7

### Extrakte, erhältlich aus dem Mycel der Spezies Ganoderma pfeifferi DSM 13239, wobei die Pilze in Flüssigmedien mit Kohlenhydratquellen unter Einhaltung eines Beleuchtungs- und Schüttelregimes kultiviert werden.

Methodik: Es wurde Hagem-Medium, Malzmedium und ein synthetisches Medium auf ihre Eignung mit speziellen Zusätze von Spurenelementen und Vitaminen bei Einhaltung unterschiedlicher Beleuchtungs- und Schüttelregime unter Verwendung der im Beispiel 5 aufgeführten Methoden untersucht.

Ergebnis: Verschiedene Flüssigmedien sind zur Kultivierung von G. pfeifferi geeignet. Vorzugsweise sollte Malzmedium mit einem Gehalt an Malzextrakt von 20-40 g/1000 I und einem Ausgangs-pH von 4,5-7,5 eingesetzt werden. Unter diesen Bedingungen wird während eine 30tägigen Kultur eine Steigerung des Mycelgewichtes von 0,061+0,0006 g/100 ml auf 0,691 +0,0482 g /100 ml erreicht. Bei Verwendung von Malzmedium pH 4,5 wurden Ausbeuten des Dichlormethan-Extraktes von 15% erzielt, die signifikant über den über den unter anderen Kulturbedingungen erreichbaren Werten von 1 bis 7% lagen.

### Beispiel 8

### Verbesserung der Ausbeute an Mycel und Extrakten durch Zusatz von Holzextrakt, insbesondere Dekokt aus Buche, allein oder in Kombination mit zelluloseabbauenden Enzymen zum Flüssigmedium

Methodik: Holz-Dekokt wurde durch 1stündiges Kochen von 30 g zerkleinertem Holz einer Buche mit 300 ml destilliertem Wasser hergestellt. Der Dekokt wurde den Kulturansätzen gemäß Beispiel 6 in einer Konzentration von 10% zugefügt. Bei einem Teil der Versuche erfolgte gleichzeitig die Zugabe einer Mischung zelluloseabbauender Enzyme (Affina GmbH, Berlin, G).

Ergebnis: Die Optimierung der Kultuvierungsbedingungen bewirkt eine Zunahme des Mycelwachstums (Kontrolle nach Beispiel 6: 0,34 +0,0568 g; optimiertes Medium mit Holzextraktzusatz: 1,079 + 0,1097 *g*/*100 ml; optimiertes Medium mit Holzdekokt + Zellulasen; 1,481 + 0,12 g*/*100 ml*), und eine verstärkte Bildung von Sekundärmetaboliten. Die Extraktausbeute nach Beispiel 6 betrug nach einer Kultivierungsdauer von 30 bei Extraktion mit Dichlormethan 30 mg und wurde durch die Optimierung auf 119 mg gesteigert, die Ausbeute nach Extraktion mit Ethanol stieg von 191 mg auf 491 mg.

### Beispiel 9

### Extrakte, erhalten durch eine Extraktion des Fruchtkörpers oder des Mycels mit einem liphophilen Lösungsmittel

Methodik: Pulverisierte Fruchtkörper oder lyophilisiertes Mycel wurde in Extraktionshülsen gefüllt und mit verschiedenen lipophilen Lösungsmittel in einer Soxhlet-Apparatus für 24 h extrahiert. Alle Extrakte wurden im Vakuumrotationsverdampfer (Büchi Labortechnik AG, Flawil, Schweiz) bei 40°C eingeengt, lyophilisiert (Gefriertrocknungsanlagen GmbH, Osterode, G) und anschließend das Trockengewicht ermittelt.

Ergebnis: Dichlormethan ist am besten geeignet, um eine hohe Ausbeute biologisch aktiver Extrakte zu erhalten.

### Beispiel 10

### Extrakte, erhalten durch eine Extraktion des Kulturmediums mit Ethylacetat

Methodik: Das Medium der Pilzkultur wurde im Vakuumrotationsverdampfer (Büchi Labortechnik AG, Flawil, Schweiz) auf ca. 50 ml eingeengt und anschließend durch mehrfaches Schütteln mit Ethylacetat extrahiert. Der Vorgang wurde wiederholt, bis die Ethylacetatphase keine Färbung aufwies.

Ergebnis: Es wurden Ausbeuten zwischen 0,1 und 0,5% erhalten. Die Extrakte zeigten im Agardiffusionstest gemäß Beispiel 1 eine Hemmwirkung gegen S. aureus mit Hemmhöfen bis 17 mm.

### Beispiel 11

### Extrakte, erhältlich durch eine Extraktion von Kulturmycel mit lipophilen Lösungsmitteln, vorzugsweise mit Dichlormethan

Methodik: Im Gegensatz zu Beispiel 9 wurde die Kultur bereits nach 5 Tagen geerntet. Die Extraktion erfolgte nach Beispiel 9, die Bestimmung der antibakteriellen Aktivität nach Beispiel 1 gegen S. aureus und M. flavus.

Ergebnis: Bereits nach 5 Kulturtagen lässt sich eine antibakterielle Aktivität durch Hemmhöfe von 18 bzw. 20 mm nachweisen (Tab. 5).

**Tab. 5**

| Antibakterielle Aktivität von Dichlormethanextrakten erhältlich aus Kulturen von G. pfeifferi nach 5 Kulturtagen | |
|---|---|
| Testkeim | Hemmhof (mm) |
| S. aureus | 18 |
| M. flavus | 20 |

### Beispiel 12

### Extrakte, erhältlich durch eine Extraktion des Rückstandes, der bei der Extraktion von Fruchtkörpern von G. pfeifferi mit lipophilen Lösungsmitteln erhalten wird, mit Ethanol

Methodik: Der Rückstand, der bei Extraktion von G. pfeifferi mit lipophilen Lösungsmitteln nach Beispiel 9 erhalten wird, wurde 5mal je 2 h mit 80%igem Ethanol (E. Merck, Darmstadt) unter ständigem Schütteln bei Raumtemperatur extrahiert. Der Extrakt wurde durch Filtration mit einem Büchnertrichter abgetrennt und an der Luft getrocknet. Die Prüfung auf antimikrobielle Wirksamkeit erfolgte nach Beispiel 1.

Ergebnis: Die ethanolischen Extrakte sind antibakteriell aktiv (Tab. 6).

**Tab. 6**

| Antibakterielle Aktivität von ethanolischen Extrakten aus Rückständen der Extraktion nach Beispiel 9 | |
|---|---|
| Keim | Hemmhofdurchmesser (mm) |
| S. aureus | 24 |
| B. subtilis | 15 |
| M. flavus | 18 |
| E. coli | 8 |
| P. mirabilis | 17 |
| S. marcescens | 22 |

### Beispiel 13

### Extrakte, erhältlich durch eine Wasserextraktion des Rückstandes der Dichlormethanextraktion

Methodik: Der Rückstand, der bei Extraktion von G. pfeifferi mit lipophilen Lösungsmitteln nach Beispiel 9 erhalten wird, wurde 5mal je 2 h mit 70°C heißem Wasser unter ständigem Schütteln bei Raumtemperatur extrahiert. Der Extrakt wurde durch Filtration mit einem Büchnertrichter abgetrennt und an der Luft getrocknet. Die Prüfung auf antimikrobielle Wirksamkeit erfolgte nach Beispiel 1.

Ergebnis: Die wässrigen Extrakte sind antibakteriell aktiv (Tab. 7).

**Tab. 7**

| Antibakterielle Aktivität von wässrigen Extrakten aus G. pfeifferi | |
|---|---|
| Keim | Hemmhofdurchmesser (mm) |
| S. aureus | 32 |
| B.subtilis | 19 |
| M. flavus | 20 |
| E.coli | 12 |
| P. mirabilis | 26 |
| S. marcescens | 25 |

### Beispiel 14

### Extrakte, erhältlich durch eine Wasserextraktion des Rückstandes der Ethanolextraktion nach Beispiel 12

Methodik: Der Rückstand, der durch aufeinanderfolgende Extraktion von G. pfeifferi mit lipophilen Lösungsmitteln und Alkoholen erhalten wird, wurde 5mal je 2 h mit 70°C heißem Wasser unter ständigem Schütteln bei Raumtemperatur extrahiert. Der Extrakt wurde durch Filtration mit einem Büchnertrichter abgetrennt und an der Luft getrocknet. Die Prüfung auf antimikrobielle Wirksamkeit erfolgte nach Beispiel 1.

Ergebnis: Die wässrigen Extrakte des Rückstandes der Ethanolextraktion sind antibakteriell aktiv (Tab. 8).

**Tab. 8**

| Antibakterielle Aktivität von wässrigen Extrakten aus Rückständen der Extraktion nach Beispiel 12 | |
|---|---|
| Keim | Hemmhofdurchmesser (mm) |
| S. aureus | 32 |
| B.subtilis | 16 |
| M. flavus | 18 |
| E.coli | 12 |
| P. mirabilis | 25 |
| S. marcescens | 25 |

### Beispiel 15

### Extrakte aus Kulturmycel G. pfeifferi erhalten durch eine Extraktion mit einwertigen Alkoholen

Kulturen von G. Pfeifferi gemäß Beispiel 7, teilweise mit Zusatz von Holzdekokt gemäß Beispiel 8 wurden am 5. Versuchstag geerntet. Das Pilzmycel wurde 5mal je 2 h mit 80%igem Ethanol (E. Merck, Darmstadt) unter ständigem Schütteln bei Raumtemperatur extrahiert. Der Mycelrückstand wurde mit einem Büchner-Trichter abgetrennt. Die Extrakte wurden im Vakuumrotationsverdampfer (Büchi Labortechnik AG, Flawil, Schweiz) bei 40°C eingeengt und anschließend lyophilisiert (Gefriertrocknungsanlagen GmbH, Osterode, G).

Ergebnis: Durch Extraktion mit Alkoholen können Extrakteausbeuten erhalten werden, die etwa ein Drittel der Gesamtmycelmasse ausmachen (Tab. 9).

**Tab. 9**

| Extraktausbeuten und Anteil an der Gesamtmasse des Mycels nach einer Kultivierung von 5 Tagen | | |
|---|---|---|
| Kultivierungsbedingungen | Ausbeute des Ethanolischen Extrakts (mg) | Anteil an der Gesamtmycelmasse (%) |
| Malzmedium pH 5 | **549** | **32,5** |
| Malzmedium pH 7,5 | **596** | **30,3** |
| Malzmedium pH 5,5 mit Zusatz von Buchendekokt | **1308** | **35,9** |

### Beispiel 16

### Extraktion von Fruchtkörper und Kulturmycel mit Wasser

Methodik: Fruchtkörper nach Beispiel 1 und Kulturmycel nach Beispiel 5 wurden mit Wasser von 70°C , in einem weiteren Versuch zunächst mit Wasser von 20°C und anschließend mit Wasser von 70°C extrahiert.

Ergebnisse: Es werden Extraktrakte erhalten, die etwa 6% der Mycelgesamtmasse ausmachen. Eine Auftrennung der Extrakte wird erreicht, wenn die Extraktion stufenweise durchgeführt wird (Tab. 10).

**Tab. 10**

| Ausbeuten wässriger Extrakte und Anteil an der Gesamtmasse des Fruchtkörpers bzw. des Mycels | | |
|---|---|---|
| Ausgangsstoff/Bedingungen | Ausbeute des Extrakts (mg) | Anteil an der Gesamtmycelmasse (%) |
| Fruchtkörper, 70°C | **45** | **7,5** |
| Mycel, 70°C | **42** | **7,0** |
| Fruchtkörper, 20°C | **27** | **4,5** |
| Mycel, 20°C | **28** | **4,7** |
| Fruchtkörper, erst 20°C, dann 70°C | **19** | **3,2** |
| Mycel, erst 20 ° C, dann 70°C | **14** | **2,4** |

### Beispiel 17

### Extrakte aus Rückständen der Extraktion nach Beispiel 9 durch eine Extraktion mit einwertigen Alkoholen

Rückstände einer Extraktion nach Beispiel 9 ausgehend von Pilzkulturen in synthetischem Medium und in Malzmedium, die am 15. Versuchstag geerntet wurden, wurden 5mal je 2 h mit 80%igem Ethanol (E. Merck, Darmstadt) unter ständigem Schütteln bei Raumtemperatur extrahiert. Der Mycelrückstand wurde danach mit einem Büchner-Trichter abgetrennt. Die Extrakte wurden im Vakuumrotationsverdampfer (Büchi Labortechnik AG, Flawil, Schweiz) bei 40°C eingeengt und anschließend lyophilisiert (Gefriertrocknungsanlagen GmbH, Osterode, G).

Ergebnis: Es werden hohe Ausbeute der ethanolischen Extrakte erhalten (Tab. 11).

**Tab. 11**

| Extraktausbeuten und Anteil an der Gesamtmasse des Mycels nach einer Kultivierung von 15 Tagen | | |
|---|---|---|
| Kultivierungsbedingungen | Ausbeute des Ethanolischen Extrakts (mg) | Anteil an der Gesamtmycelmasse (%) |
| Synthetisches Medium pH5,5 | **274** | **38,5** |
| Malzmedium pH5,5 | **756** | **20,9** |

### Beispiel 18:

### Extrakte, erhältlich aus Rückständen der Extraktion nach Beispiel 9

Methodik: Der nach der Extraktion der Fruchtkörper mit Dichlormethan nach Beispiel 9 verbleibende Rückstand wurde mit Ethanol nach Beispiel 17 extrahiert. Der ethanolische Extrakt wurde anschließend zwischen Wasser und Ethylacetat verteilt.

Ergebnis: Die Ethylacetat-Phase ist antimikrobiell aktiv (Tab. 12).

**Tab. 12**

| Antibakterielle Aktivität des Ethylacetatextraktes | |
|---|---|
| Keim | Hemmhofdurchmesser |
| S.aureus | 16 mm |
| B. subtilis | 14 mm |
| M.flavus | 15 mm |

### Beispiel 19

### Auftrennung der Extrakte nach Beispiel 18 in biologisch aktive Fraktionen mittels Kieselgel

Methodik: Die Ethylacetat-Phase nach Beispiel 18 wurde über Kieselgel fraktioniert. Als Laufmittel wurde ein Gradient aus Dichlormethan-Ethylacetat, anschließend erfolgte die weitere Fraktionierung mit dem Gradienten Ehylacetat-Methanol.

Ergebnis: Es wurde ein Vielzahl Fraktionen erhalten, die alle antibakteriell aktiv sind. Besonders aktiv ist der Extrakt, der mit dem Laufmittel Dichlormethan:Ethylacetat 4:1 erhalten wird (Tab. 13).

| Fraktion | Wirksamkeit gegen S. aureus (Hemmhof in mm) |
|---|---|
| Dichlormethan : Ethylacetat = 1 : 0 | 10 |
| Dichlormethan : Ethylacetat = 4 :1 | 25 |
| Dichlormethan : Ethylacetat = 2 :1 | 10 |
| Dichlormethan : Ethylacetat = 1 : 1 | 15 |
| Dichlormethan : Ethylacetat = 0,5 : 1 | 10 |
| Dichlormethan : Ethylacetat = 0,25 :1 | 10 |
| Ethylacetat : Methanol = 4 : 1 | 10 |
| Ethylacetat : Methanol = 2 : 1 | 10 |
| Ethylacetat : Methanol = 1 : 1 | 10 |
| Ethylacetat : Methanol = 0 : 1 | 10 |

### Beispiel 20

### Auftrennung der Extrakte nach Beispiel 18 in biologisch aktive Fraktionen mittels Sephadex

Methodik: Die Ethylacetat-Phase nach Beispiel 18 wurde mit Methanol als Laufmittel über Sephadex LH-20 fraktioniert.

Ergebnis: Es wurde 6 Fraktionen erhalten, die im unterschiedlichen Ausmaß antibakteriell aktiv sind (Tab.14) .

**Tab. 14**

| Extrakte, erhältlich aus der Ethylacetatphase durch Auftrennung an Sephadex | |
|---|---|
| Fraktion | Wirksamkeit gegen S. aureus (Hemmhof in mm) |
| A | 10 |
| B | 10 |
| C | 15 |
| D | 20 |
| E | 25 |
| F | 10 |

### Beispiel 21

### Isolierung von 3,26-Dihydroxy-lanosta-8,24-dien-7-on (Ganoderon B)

Methodik: 400 mg des Dichlormethanextraktes des Fruchtkörpers wurden in der mobilen Phase gelöst und an Sephadex LH-20 (LKB Pharmacia, Upsala, S) mit der mobilen Phase n-Hexan:Dichlormethan 2:7 bei einer Flussrate von 24 ml/h aufgetrennt. Nach 24 h wurde mit Methanol gespült. Nach Dünnschichtchromatografie-Monitoring wurden Fraktionen gleicher Zusammensetzung vereinigt. Die Fraktion mit dem R_{f}-Wert 0,38 wurde an Sephadex LH-20 (LKB Pharmacia , Upsala, S) mit der mobilen Phase Methanol:Wasser = 4:1 weiter gereinigt. Die gewonnene Substanz wurde aus Methanol umkristallisiert.

Ergebnis: Es wurde eine bisher unbekannte Substanz mit dem Schmelzpunkt 165°C isoliert, die anhand der Spektren als 3,26-Dihydroxy-lanosta-8,24-dien-7-on identifiziert wurde und die wir als Ganoderon B bezeichnet haben.

### Beispiel 22

### Isolierung von 2-[2-(2,5-Dihydroxyphenyl)-ethyliden]-11-hydroxy-6,10-dimethylundeca-5,9-diensäure (Ganomycin A)

Methodik: 400 mg des Dichlormethanextraktes des Fruchtkörpers wurden in der mobilen Phase gelöst und an Sephadex LH-20 (LKB Pharmacia , Upsala, S) mit der mobilen Phase n-Hexan:Dichlormethan 2:7 bei einer Flussrate von 24 ml/h aufgetrennt. Nach 24 h wurde mit Methanol gespült. Nach Dünnschichtchromatografie-Monitoring wurden Fraktionen gleicher Zusammensetzung vereinigt. Die Fraktion mit dem R_{f}-Wert 0,44 wurde an Sephadex LH-20 mit Dichlormethan und Methanol = 4:1 als Laufmittel rechromatochrafiert. Die weitere Reinigung erfolgte mit Methanol als Laufmittel bei einer Fließgeschwindigkeit von 12 ml/h.

Ergebnis: Es wurde eine bisher unbekannte Substanz in Form eines gelben Öls isoliert, die anhand der Spektren als 2-[2-(2,5-Dihydroxyphenyl)-ethyliden]-11-hydroxy-6,10-dimethyl-undeca-5,9-diensäure identifiziert wurde und die wir als Ganomycin A bezeichnet haben.

### Beispiel 23

### Gewinnung von 2-[2-(2,5-Dihydroxyphenyl)-ethyliden]--6,10-dimethylundeca-5,9-diensäure (Ganomycin B) als weiterem Wirkstoff mit der Formel 1 aus G. pfeifferi

Methodik: 400 mg des Dichlormethanextraktes des Fruchtkörpers wurden in der mobilen Phase gelöst und an Sephadex LH-20 (LKB Pharmacia, Upsala, S) mit der mobilen Phase n-Hexan: Dichlormethan 2:7 bei einer Flussrate von 24 ml/h aufgetrennt. Die weitere Reinigung erfolgte mit dem Laufmittel Methanol:Wasser 2:1 Die Fraktion mit dem R_{f}-Wert 0,58 wurde durch Säulenchromatographie an Sephadex LH-20 mit dem Laufmittel Aceton:Dichlormethan = 2:1 gereinigt.

Ergebnis: Es wurde eine bisher unbekannte Substanz in Form eines gelben Öls isoliert, die anhand der Spektren als 2-[2-(2,5-Dihydroxyphenyl)-ethyliden]-6,10-dimethyl-undeca-5,9-diensäure identifiziert wurde und die wir als Ganomycin B bezeichnet haben.

### Beispiel 24

### Synthese von Ganomycin B

Zu 42 ml einer 0.67 molaren Lösung von Lithiumdiisopropylamid (dargestellt aus n-Butyllithium und Diisopropylamin) in THF (entspricht 28.14 mmol LDA) werden bei -78°C unter Schutzgasatmosphäre 5.13 g (28.14 mmol) 2-(Phenylthio)-essigsäure-methylester gelöst in 35 ml THF gegeben. Nach 40 Minuten Rühren bei dieser Temperatur erfolgt die Zugabe von 3.55 g (11.00 mmol) Homogeranyl-tosylat gelöst in 20 ml DMSO. Die Lösung wird auf Raumtemperatur erwärmt und noch 18 Stunden gerührt. Zur Aufarbeitung werden 25 ml ges. wässr. NH₄Cl-Lsg zugegeben. Anschließend wird 3x mit je 30 ml Dichlormethan extrahiert, die vereinigten organischen Phasen werden nacheinander mit je 20 ml ges. wässr. CuSO₄-Lsg., ges. wässr. NaCl-Lsg. und dest. Wasser gewaschen und über MgSO₄ getrocknet. Der nach der Entfernung des Lösungsmittels verbleibende Rückstand wird säulenchromatographisch gereinigt (Laufsystem Heptan:Essigester = 7:1). Ausbeute 2.34 g (7.04 mmol, 64%) (*5E*)-6,10-Dimethyl-2-(phenylthio)-5,9-undecadiensäure-methylester. Diese Verbindung wird gelöst in 8.5 ml THF und zu 10.5 ml einer 0.67 molaren Lösung von Lithiumdiisopropylamid (dargestellt aus n-Butyllithium und Diisopropylamin) in THF (entspricht 7.04 mmol LDA) werden bei -78°C unter Schutzgasatmosphäre gegeben. Nach 40 Minuten Rühren bei dieser Temperatur wird die Lösung mittels einer Spritze in einen auf 0° C temperierten Kolben überführt, in dem 1.34 g (9.86 mmol) frisch umgeschmolzenes Zinkchlorid vorgelegt wurden. Die Lösung wird nun solange bei 0°C gerührt, bis sich nahezu das gesamte Zinkchlorid gelöst hat (ca. 10 Minuten), danach erfolgt die Zugabe von 4.80 g (12.61 mmol) [2,5-Bis-[(*tert*.butyl-dimethyl-silyl)-oxy]-phenyl]-acetaldehyd gelöst in 5 ml THF. Nach weiteren 10 Minuten Rühren werden 25 ml ges. wässr. NH₄Cl-Lsg zugegeben. Anschließend wird 3x mit je 30 ml Dichlormethan extrahiert, die vereinigten organischen Phasen werden nacheinander mit je 20 ml ges. wässr. CuSO₄-Lsg., ges. wässr. NaCl-Lsg. und dest. Wasser gewaschen und über MgSO₄ getrocknet. Der nach der Entfernung des Lösungsmittels verbleibende Rückstand wird mit 15 ml Aceton aufgenommen. Zu dieser Lösung werden nacheinander 1.28 g (6.00 mmol) N-Ethyl-2-fluor-pyridinium-tetrafluoroborat und 0.84 ml (6.00 mmol) frisch destilliertes Triethylamin gegeben. Nach 5-10 Minuten Rühren bei Raumtemperatur erfolgt die Zugabe von 0.87 g (6.50 mmol) Lithiumiodid. Die Reaktionsmischung wird auf 60°C erwärmt und für 1 Stunde bei dieser Temperatur gerührt. Nach dem Abkühlen auf Raumtemperatur werden 50 ml Dichlormethan zugegeben. Die Lösung wird 2x mit je 15 ml ges. wässr. CuSO₄-Lösung, anschließend mit 15 ml dest. Wasser gewaschen und über MgSO₄ getrocknet. Die säulenchromatographische Reinigung (Laufsystem Heptan:Essigester = 10:1) des nach dem Abdestillieren des Lösungsmittels verbleibenden Rückstands liefert die beiden isomeren (*2(1')Z,5E*)- und (*2(1')E,5E*)-2-[2'-(2,5-Bis-[(*tert*.butyl-dimethylsilyl)-oxy]-phenyl)-ethyliden]-6,10-dimethyl-5,9-undecadiensäure-methylester in einem Verhältnis von 45:55 zugunsten des (*2(1')E,5E*)-Isomers und einer Gesamtausbeute von 48%.

Zur Spaltung der Esterfunktion werden 0.20 g (2*(1')Z,5E,9E*)-2-[2'-(2,5-Bis-[(*tert*.butyl-dimethyl-silyl)-oxy]-phenyl)-ethyliden]-6,10-dimethyl-5,9-undecadiensäure-methylester in einer Lösung von 1.00 g Kaliumhydroxid in 10 ml 96%igem Ethanol für 4 Stunden unter Rückfluss erhitzt. Die auf Raumtemperatur abgekühlte Reaktionsmischung wird auf 50 ml Eiswasser, welches mit 3 ml konz. Schwefelsäure versetzt ist, gegeben. Das Produkt wird 3x mit je 15 ml Ether extrahiert. Die vereinigten organischen Phasen werden mit 15 ml ges. wässr. NaCl-Lösung gewaschen, über MgSO₄ getrocknet und unter vermindertem Druck eingeengt. Die Reinigung des Produktes erfolgt säulenchromatographisch (Laufsystem Heptan:Essigester = 6:1). Ausbeute 87% (*2(1')Z,5E,9E*)-2-[2'-(2,5-Bis-[(*tert*.butyl-dimethyl-silyl)-oxy]-phenyl)-ethyliden]-6,10-dimethyl-5,9-undecadiensäure.

Zur Abspaltung der *tert*.butyl-dimethyl-Silylgruppen werden 0.10 g [2'-(2,5-Bis-[(*tert*.butyl-dimethyl-silyl)-oxy]-phenyl)-ethyliden]-6,10-dimethyl-5,9-undecadiensäure zusammen mit 2 Äquivalenten wasserfreien Kaliumfluorid und 0.2 Äquivalenten 48% wässr. HBr in 10 ml DMF gelöst und bei Raumtemperatur für 24 Stunden unter Argonatmosphäre gerührt. Anschließend werden 10 ml 2.0 molare wässrige HCl-Lösung zugegeben und die Reaktionsmischung 3x mit je 15 ml Ether extrahiert. Die vereinigten organischen Phasen werden mit 10 ml ges. wässr. NaCl-Lösung gewaschen, über MgSO₄ getrocknet und unter vermindertem Druck eingeengt. Die Reinigung des Rohprodukts erfolgt säulenchromatographisch (Laufsystem Heptan:Essigester = 1:1). Ausbeute 87% Ganomycin B.

### Beispiel 25

### Gewinnung von Ganomycin B über metallorganische Zwischenstufen

Methodik: Zu einer auf 0°C temperierten Lösung von 1.10 g (10.00 mmol) 1,4-Hydrochinon und 3.05 ml (22.00 mmol) Triethylamin in 20 ml Chloroform werden unter Argonatmosphäre mittels einer Spritze langsam 5.82 g (22.00 mmol) *tert*.Butyl-dimethyl-silyl-triflat gegeben. Nach der Erwärmung der Reaktionslösung auf Raumtemperatur wird diese für 18 Stunden gerührt und anschließend auf 50 ml Eiswasser gegeben. Nach Abtrennung der org. Phase wird die wässrige mit 100 ml Chloroform extrahiert. Die vereinigten organischen Phasen werden nacheinander mit je 100 ml 1 molarer wässr. HCl, 1 molarer wässr. NaOH und ges. wässr. Bicarbonatlösung gewaschen und über MgSO₄ getrocknet. Nach Entfernung des Lösungsmittels unter vermindertem Druck wird das Produkt durch Destillation gereinigt. Ausbeute 3.05 g (9.00 mmol, 90%) 1,4-Bis-[(*tert*.butyl-dimethyl-silyl)-oxy]-benzol.

Dieses Zwischenprodukt wird in einem zweiten Reaktionsschritt in 40 ml Tetrachlorkohlenstoff gelöst und auf 0°C gekühlt. Unter kräftigem Rühren werden 3.70 ml (7.20 mmol) Brom in 10 ml Tetra-chlorkohlenstoff langsam zugetropft, so dass die Temperatur der Reaktionslösung 5°C nicht übersteigt. Nach Beendigung der Zugabe wird noch 2 Stunden bei dieser Temperatur gerührt, danach die Lösung zur Aufarbeitung nacheinander mit je 15 ml dest. Wasser, 3%iger wässr. Thiosulfatlösung, 10%iger wässriger NaOH-Lsg. und wieder mit dest. Wasser gewaschen. Das nach der Entfernung des Lösungsmittels unter vermindertem Druck erhaltene Rohprodukt wird aus Chloroform/Acetonitril umkristallisiert. Ausbeute 2.25 g (5.40 mmol, 60%) 1,4-Bis-[(*tert.*butyldimethyl-silyl)-oxy]-2-brom-benzol.

Als nächster Reaktionsschritt erfolgt die Herstellung einer Grignardverbindung, Dazu werden in einem Dreihalskolben mit Rückflusskühler 0.13 g (5.40 mmol) Magnesiumspäne und 0.25 g (0.60 mmol) 1,4-Bis-[(*tert*.butyl-dimethyl-silyl)-oxy]-2-brom-benzol in 10 ml abs. Ether vorgelegt. Das Anspringen der Reaktion ist an einer Trübung und Erwärmung der Lösung erkennbar. Unter starkem Rühren werden dann langsam 2.00 g (4.80 mmol) 1,4-Bis-[(*tert*.butyldimethyl-silyl)-oxy]-2-brom-benzol gelöst in 100 ml abs. Ether so zugetropft, dass der Ether am gelinden Sieden gehalten wird. Nach Beendigung der Zugabe wird für ca. 30 Minuten unter Rückfluss erhitzt, bis nahezu das gesamte Magnesium gelöst ist. Die etherische Lösung des *in situ* erzeugten [2,5-Bis-[(*tert*.butyl-dimethyl-silyl)-oxy]-phenyl]-magnesium-bromids (5.40 mmol) wird mit 30 ml THF verdünnt, auf -30°C abgekühlt und tropfenweise mit 2.50 ml einer 0.10 molaren Lösung von Dilithium-tetrachlorocuprat versetzt. Nach 30 Minuten Rühren bei dieser Temperatur wird die gesamte Lösung tropfenweise zu einer auf -30°C gekühlten Lösung von 1.44 g (5.40 mmol) des (*E*)-6,10-Dimethyl-2-oxiranyl-undeca-5,9-dien-carbonsäure-methylesters, dessen Synthese im Anschluss beschrieben ist, in 30 ml THF gegeben. Nach ca. 2 Stunden Rühren bei der angegebenen Temperatur ist die Reaktion beendet, zur Aufarbeitung werden 10 ml ges. wässr. NH₄Cl-Lösung und 15 ml dest. Wasser zugegeben. Nach Trennung der Phasen wird die wässrige Phase noch einmal mit 20 ml Ether extrahiert, die vereinigten organischen Phasen werden 2x mit je 15 ml ges. wässr. NaCl-Lösung gewaschen und über MgSO₄ getrocknet. Der nach dem Abdestillieren des Lösungsmittels erhaltene Rückstand wird säulenchromatographisch gereinigt (Laufsystem Heptan:Essigester = 9:1). Ausbeute 1.83 g (3.02 mmol, 56%) (*5E*)-2-[2'-(2,5-Bis-[(*tert.*butyl-dimethylsilyl)-oxy]-phenyl)-1'-hydroxy-ethyl]-6,10-dimethyl-5,9-undecadiensäuremethyl-ester.

Zu einer auf 0°C gekühlten Lösung von 1.64 g (2.72 mmol) (*5E*)-2-[2'-(2,5-Bis-[(*tert*.butyl-dimethyl-silyl)-oxy]-phenyl)-1'-hydroxy-ethyl]-6,10-dimethyl-5,9-undecadiensäure-methylester und 1.52 ml (10.90 mmol) Triethylamin in 8 ml Dichlormethan werden 0.42 ml (5.50 mmol) Methansulfonsäure-chlorid gegeben. Die Reaktionsmischung wird für 3 Stunden bei Raumtemperatur gerührt, anschließend mit 20 ml Dichlormethan verdünnt und nacheinander mit je 10 ml ges. wässr. NaHCO₃-Lsg., 0.10 molarer wässriger HCl-Lsg. und ges. wässr. NaCl-Lsg. gewaschen. Nach der Trocknung der organischen Phase über MgSO₄ wird das Lösungsmittel unter vermindertem Druck abdestilliert, der verbleibende Rückstand in 8 ml Toluol aufgenommen und mit 1.2 ml (8.20 mmol) 1,8-Diazabicyclo[5.4.0]undecen (DBU) versetzt. Die Lösung wird für 12 Stunden bei einer Temperatur von 80°C gerührt, anschließend mit 15 ml Ether verdünnt und nacheinander mit je 10 ml 0.10 molarer wässriger HCl-Lsg., ges. wässr. NaHCO₃-Lsg. und ges. wässr. NaCl-Lsg. gewaschen. Der nach der Trocknung der organischen Phase über MgSO₄ und der Entfernung des Lösungsmittels erhaltene Rückstand wird säulenschromatographisch gereinigt (Laufsystem Heptan:Essigester = 10:1). Die beiden isomeren (*2(1')Z,5E*)- und (*2(1')E,5E*)-2-[2'-(2,5-Bis-[(*tert.*butyl-dimethyl-silyl)-oxy]-phenyl)-ethyliden]-6,10-dimethyl-5,9-un-decadiensäure-methylester werden in einem Verhältnis von 20:80 zugunsten des (*2(1')E,5E*)-Isomers und in einer Gesamtausbeute von 83% erhalten.

Zur Synthese des *(E)*-6,10-Dimethyl-2-oxiranyl-undeca-5,9-dien-carbonsäuremethylesters werden 27.00 ml (54.00 mmol) einer 2 molaren Lösung von Allylmagnesiumchlorid in Ether innerhalb von 30 Minuten zu einer Lösung von 1.10 g (6.40 mmol) Geranylchlorid in 50 ml THF/HMPT (v/v = 1/1) getropft. Die resultierende Mischung wird für 15 Stunden bei Raumtemperatur gerührt und anschließend mit 20 ml ges. wässr. NH₄Cl-Lsg. versetzt. Die organische Phase wird abgetrennt, die wässrige 4x mit je 25 ml Dichlormethan extrahiert. Die vereinigten organischen Phasen werden unter vermindertem Druck eingeengt. Der verbleibende Rückstand wird mit 50 ml Ether aufgenommen, nacheinander mit 2 molarer wässr. Salzsäure, ges. wässr. NaCl-Lösung und dest.

Wasser gewaschen, über MgSO₄ getrocknet und unter vermindertem Druck eingeengt. Die Vakuumdestillation des so erhaltenen Rückstands liefert 0.89 g (4.99 mmol, 78%) des *(E)*-6,10-Dimethyl-undeca-1,5,9-triens.

24.70 ml (12.40 mmol) einer 0.5 molaren Lösung von Bis-(3-methyl-butan-2-yl)-boran in Ether werden innerhalb von 10 Minuten bei 0-5°C zu einer gut gerührten Lösung von 2.00 g (11.20 mmol) des (*E*)-6,10-Dimethylundeca-1,5,9-triens in 25 ml THF getropft. Die Reaktionsmischung wird für 15 Stunden gerührt und anschließend mit 3.50 ml 3 molarer wässr. NaOH-Lösung, 3.50 ml 30%iger wässriger Wasserstoffperoxidlösung versetzt. Nach einer Stunde Rühren wird mit 10.00 ml wässr. ges. NH₄Cl-Lösung neutralisiert und die wässrige Phase 3x mit je 20.00 ml Ether extrahiert. Die vereinigten organischen Phasen werden über MgSO₄ getrocknet und unter vermindertem Druck eingeengt. Das so erhaltene Rohprodukt wird säulenchromatographisch gereinigt (Laufsystem Heptan:Ether = 7:3). Ausbeute 0.85 g (4.26 mmol, 38%) (*E*)-6,10-Dimethyl-undeca-5,9-dien-1-ol als farbloses Öl.

3.50 g (8.90 mmol) Pyridiniumdichromat werden bei Raumtemperatur zu einer Lösung von 0.50 g (2.60 mmol) *(E)*-6,10-Dimethylundeca-5,9-dien-1-ol in 15 ml DMF gegeben. Nach 20 Stunden Rühren wird die gesamte Lösung auf 80 ml Eiswasser gegossen und 3x mit je 60 ml Ether extrahiert. Die vereinigten organischen Extrakte werden über MgSO₄ getrocknet und säulenchromatographisch gereinigt (Laufsystem Heptan:Ether = 7:3). Ausbeute 0.17 g (0.81 mmol, 31%) (*E*)-6,10-Dimethyl-undeca-5,9-dien-carbonsäure als farbloses Öl.

1.70 g (8.08 mmol) (*E*)-6,10-Dimethylundeca-5,9-dien-carbonsäure werden in 10 ml Methanol/Wasser (v/v = 10/1) gelöst unter Rühren wird solange 1.0 molare etherische Diazomethanlösung zugetropft bis keine Stickstoffentwicklung mehr zu beobachten ist. Der nach der Entfernung des Lösungsmittelgemisches verbleibende Rückstand wird säulenchromatographisch gereinigt (Laufsystem Heptan:Ether = 7:1). Ausbeute 1.68 g (7.49 mmol, 93%) (*E*)-6,10-Dimethyl-undeca-5,9-dies-carbonsäure-methylester als farbloses Öl.

Zu 14 ml einer 0.67 molaren Lösung von Lithiumdiisopropylamid (dargestellt aus n-Butyllithium und Diisopropylamin) In THF (entspricht 8.92 mmol LDA) werden bei -78°C unter Schutzgasatmosphäre 1.50 g (6.69 mmol) *(E)*-6,10-Dimethylundeca-5,9-dien-carbonsäure-methylester, gelöst in 3 ml THF, tropfenwelse zugegeben. Innerhalb einer Stunde wird die Lösung auf -50°C erwärmt, anschließend wird der Ameisensäureethylester 0.83 g (11.15 mmol) zugegeben und für einer weitere Stunde bei der angegebenen Temperatur gerührt. Zur Aufarbeitung wird die gesamte Reaktionslösung auf 50 ml Eiswasser gegossen und 3x mit jeweils 15 ml Ether extrahiert. Die vereinigten organischen Phasen werden mit 20 ml dest. Wasser gewaschen und über MgSO₄ getrocknet. Der nach der Entfernung des Lösungsmittels verbleibende Rückstand kann säulenchromtograpisch (Laufsystem Heptan:Ether = 7:1) oder durch Vakuumdestillation gereinigt werden. Ausbeute 1.12 g (4.42 mmol, 66%) (*E*)-6,10-Dimethyl-2-formyl-undeca-5,9-dien-carbonsäure-methylester als farbloses Öl.

0.20 g (4.10 mmol) einer 50%igen Natriumhydrid/Öl-Dispersion werden in einer Fritte unter Schutzgas-Atmosphäre 2x mit je 10 ml trockenem Hexan gewaschen. Das vom Öl befreite NaH wird unter Schutzgas in einen Dreihalskolben überführt, mit 20 ml trockenem DMSO versetzt und für eine Stunde unter Rühren auf 60°C erhitzt. Nach der Zugabe von 25 ml THF wird die Reaktionsmischung auf 0°C gekühlt, mittels einer Spritze werden zunächst 0.90 g (4.10 mmol) Trimethylsuifonium-iodid, gelöst in 20 ml trockenem DMSO und anschließend 1.06 g (4.20 mmol) *(E)*-6,10-Dimethyl-2-formyl-undeca-5,9-dien-carbonsäure-methylester, gelöst in 15 ml THF, zugegeben. Nach 48 Stunden Rühren bei Raumtemperatur ist die Reaktion beendet. Die Reaktionsmischung wird auf 300 ml Eiswasser gegeben und 3x mit je 30 ml Ether extrahiert. Die vereinigten organischen Phasen werden 2x mit je 20 ml dest. Wasser gewaschen und über MgSO₄ getrocknet. Der nach dam Abdestillieren des Lösungsmlttels unter vermindertem Druck erhaltene Rückstand wird über eine kurze Frittensäule gereinigt (Laufsystem Heptan:Ether = 7:1). Ausbeute 0.80 g (2.99 mmol, 73%) (*E*)-6,10-Dimethyl-2-oxiranyl-undeca-5,9-diencarbonsäure-methylester als farbloses Öl.

Ergebnis: Die Abspaltung der Schutzgruppen erfolgt wie in Beispiel 24 beschrieben und liefert Ganomycin B.

### Beispiel 26

### Synthese von Ganomycin B ausgehend von 1,4-Bis-[(tert.butyl-dimethyl-silyl)-oxy]-2-methyl-benzol

Methodik: Zu einer auf 0°C temperierten Lösung von 1.24 g (10.00 mmol) 1,4-Hydroxy2-methyl-benzol und 3.05 ml (22.00 mmol) Triethylamin in 20 ml Chloroform werden unter Argonatmosphäre mittels einer Spritze langsam 5.82 g (22.00 mmol) *tert*.Butyl-dimethyl-silyl-triflat gegeben. Nach der Erwärmung der Reaktionslösung auf Raumtemperatur wird diese für 18 Stunden gerührt und anschließend auf 50 ml Eiswasser gegeben. Nach Abtrennung der org. Phase wird die wässrige mit 100 ml Chloroform extrahiert. Die vereinigten organischen Phasen werden nacheinander mit je 100 ml 1 molarer wässr. HCl, 1 molarer wässr. NaOH und ges. wässr. Bicarbonatlösung gewaschen und über MgSO₄ getrocknet. Nach Entfernung des Lösungsmittels unter vermindertem Druck wird das Produkt durch Umkristallisation aus Chloroform/Acetonitril gereinigt. Ausbeute 3.35 g (9.50 mmol, 95%) 1,4-Bis-[(*tert*.butyl-dimethylsilyl)-oxy]-2-methyl-benzol.

Dieses Zwischenprodukt wird mit 1.77 g (9.97 mmol) N-Brom-succinimid und 20 mg Benzoylperoxid in 20 ml Tetrachlorkohlenstoff für 2 Stunden unter Rückfluss erhitzt. Der nach dem Abkühlen der Lösung ausfallende Feststoff wird abfiltriert und 2x mit je 10 ml Tetrachlorkohlenstoff gewaschen. Die Filtrate werden vereinigt, der nach der Entfernung des Lösungsmittels unter vermindertem Druck zurückbleibende Feststoff wird aus Petrolether umkristallisiert. Ausbeute 3.03 g (7.03 mmol, 74%) 1,4-Bis-[(*tert*.butyl-dimethyl-silyl)-oxy]-2-brommethyl-benzol. Daraus wird wiederum eine Grignard-Verbindung hergestellt. In einem Dreihalskolben mit Rückflusskühler werden 0.13 g (5.40 mmol) Magnesiumspäne und 0.26 g (0.60 mmol) 1,4-Bis-[(*tert*.butyldimethyl-silyl)-oxy]-2-brommethylbenzol in 10 ml abs. Ether vorgelegt. Das Anspringen der Reaktion ist an einer Trübung und Erwärmung der Lösung erkennbar. Unter starkem Rühren werden dann langsam 2.00 g (4.80 mmol) 1,4-Bis-[(*tert*.butyl-dimethyl-silyl)-oxy]-2-brommethyl-benzol gelöst in 100 ml absolutem Ether so zugetropft, dass der Ether am gelinden Sieden gehalten wird. Nach Beendigung der Zugabe wird für ca. 30 Minuten unter Rückfluss erhitzt, bis nahezu das gesamte Magnesium gelöst ist.

Die etherische Lösung des *in situ* erzeugten [2,5-Bis-[(*tert*.butyl-dimethylsilyl)-oxy]-phenyl]-methyl-magnesium-bromids (5.40 mmol) wird mit 30 ml THF verdünnt, auf -30°C abgekühlt und tropfenweise mit 2.50 ml einer 0.10 molaren Lösung von Dilithium-tetrachlorocuprat versetzt. Nach 30 Minuten Rühren bei dieser Temperatur wird die gesamte Lösung tropfenweise zu einer auf -30°C gekühlten Lösung von 1.37 g (5.40 mmol) des *(E)*-6,10-Dimethyl-2-formyl-undeca-5,9-dien-carbonsäure-methylesters, dessen Synthese bereits in Beispiel 25 beschrieben wurde, in 30 ml THF gegeben. Nach ca. 2 Stunden Rühren bei der angegebenen Temperatur ist die Reaktion beendet, zur Aufarbeitung werden 10 ml ges. wässr. NH₄Cl-Lösung und 15 ml dest. Wasser zugegeben. Nach Trennung der Phasen wird die wässrige Phase noch einmal mit 20 ml Ether extrahiert, die vereinigten organischen Phasen werden 2x mit je 15 ml ges. wässr. NaCl-Lösung gewaschen und über MgSO₄ getrocknet. Der nach dem Abdestillieren des Lösungsmittels erhaltene Rückstand wird säulenchromatographisch gereinigt (Laufsystem Heptan:Essigester = 9:1). Ausbeute 1.83 g (3.02 mmol, 56%) (*5E*)-2-[2'-(2,5-Bis-[(*tert*.butyl-dimethylsilyl)-oxy]-phenyl)-1'-hydroxy-ethyl]-6,10-dimethyl-5,9-undecadiensäuremethylester.

Die Wasserabspaltung erfolgt wie in Beispiel 25 beschrieben und liefert die beiden isomeren (*2(1')Z,5E*)- und (*2(1')E,5E*)-2-[2'-(2,5-Bis-[(*tert*.butyldimethyl-silyl)-oxy]-phenyl)-ethyliden]-6,10-dimethyl-5,9-un-decadiensäuremethylester in einem Verhältnis von 20:80 zugunsten des (*2(1')E,5E*)-Isomers und in einer Ausbeute von 83%.

Ergebnis: Die Abspaltung der Schutzgruppen erfolgt wie in Beispiel 24 beschrieben und liefert Ganomycin B.

### Beispiel 27

### Synthese von Ganomycin B ausgehend von terpenoiden Phenylsulfonen

Methodik: Eine Mischung von 4.76 g (12.50 mmol) [2,5-Bis-[(*tert*.butyldimethyl-silyl)-oxy]-phenyl]-acetaldehyd, 2.15 ml (25.00 mmol) Acrylsäuremethylester und 0.28 g (2.50 mmol) 1,4-Diaza-bicyclo[2.2.2]-octan wird für 40 Tage bei Raumtemperatur gerührt. Der nach der Entfernung des überschüssigen Acrylsäure-methylesters verbleibende Rückstand wird säulenchromatographisch (Laufsystem Heptan:Essigester = 10:1) gereinigt. Ausbeute 3.21 g (6.88 mmol, 55%) 4-[2,5-Bis-[(*tert*.butyl-dimethyl-silyl)-oxy]-phenyl]-3-hydroxy-2-methyliden-butansäure-methyl-ester.

2.80 ml (34.86 mmol) Pyridin, 2.00 ml (21.34 mmol) Acetanhydrid und 30 mg (0.24 mmol) 4-(Dimethyl-amino)-pyridin werden bei Raumtemperatur zu einer Lösung von 3.21 g (6.88 mmol) 4-[2,5-Bis-[(*tert*.butyl-dimethyl-silyl)-oxy]-phenyl]-3-hydroxy-2-methyliden-butan-säure-methylester in 80 ml Dichlormethan gegeben. Die Lösung wird für zwei Stunden gerührt und anschließend auf 20 ml ges. wässr. NH₄Cl-Lsg. gegeben, die organische Phase wird zunächst 2x mit je 10 ml ges. wässr. CuSO₄-Lösung, anschließend 2x mit je 10 ml ges. wässr. NaCl-Lösung gewaschen und über MgSO₄ getrocknet. Nach dem Abdestillieren des Lösungsmittels erfolgt die säulenchromatographische Reinigung des Rohproduktes (Laufsystem Heptan:Essigester = 10:1). Ausbeute 3.26 g (6.40 mmol, 93%) 4-[2,5-Bis-[(*tert*.butyl-dimethyl-silyl)-oxy]-phenyl]-3-acetoxy-2-methyliden-butansäure-methyl-ester.

Dieses Zwischenprodukt wird mit einem Sulfon umgesetzt, das auf folgende Weise gewonnen wird. Zu einer Aufschlämmung von 3.78 g (23.03 mmol) Natriumbenzolsulfinat in 20 ml DMF gibt man unter Rühren eine Lösung von 5.00 g (23.03 mmol) Geranylbromid in 5 ml DMF. Man erwärmt für 2 Stunden auf 120°C, kühlt ab und versetzt mit 100 g Eiswasser. Die Lösung wird anschließend 3x mit je 20 ml Dichlormethan extrahiert, die vereinigten organischen Phasen zunächst 2x mit je 15 ml ges. wässr. CuSO₄-Lsg., anschließend mit 15 ml dest. Wasser gewaschen und über MgSO₄ getrocknet. Der nach der Entfernung des Lösungsmittels unter vermindertem Druck verbleibende Rückstand wird säulenchromatographisch gereinigt (Laufsystem Heptan:Essigester = 10:1). Ausbeute 4.74 g (17.04 mmol, 74%) [(*2E*,*6E*)-3,7-Dimethyl-2,6-octadienyl]-phenyl-sulfon.

Zu einer Lösung von 2.20 g (7.90 mmol) [(*2E,6E*)-3,7-Dimethyl-2,6-octadienyl]-phenylsulfon in THF/1,3-Dimethyl-3,4,5,6-tetrahydro-2(*1H*)-pyrimidinon. (49 ml/12 ml) werden unter Argonatmosphäre bei -78 °C tropfenweise 15.2 ml einer 1.30 molaren Lösung von n-Butyllithium in Hexan (19.8 mmol) gegeben. Die Reaktionsmischung wird für 20 Minuten bei der angegebenen Temperatur gerührt, dann erfolgt innerhalb einer Stunde die tropfenweise Zugabe von 4.02 g (7.90 mmol) 4-[2,5-Bis-[(*tert*.butyl-dimethyl-silyl)-oxy]-phenyl]-3-acetoxy-2-methyliden-butansäure-methyl-ester, gelöst in 20 ml THF. Unter Rühren wird die Lösung innerhalb von 2 Stunden auf -30°C erwärmt, mit 60 ml ges, wässr. NH₄Cl-Lsg. versetzt und 4x mit je 20 ml Ether extrahiert. Die vereinigten organischen Phasen werden mit 15 ml ges. wässr. NaCl-Lsg. gewaschen und über MgSO₄ getrocknet. Die säulenchromatographische Reinigung (Laufsystem Heptan : Essigester = 10 : 1) des nach dem Abdestillieren des Lösungsmittels verbleibenden Rückstands liefert die beiden isomeren (*2(1')Z,5E*)- und (*2(1')E,5E*)-2-[2'-(2,5-Bis-[(*tert*.butyl-dimethylsilyl)-oxy]-phenyl)-ethyliden]-6,10-dimethyl-4-phenyl-sulfonyl-5,9-undecadiensäure-methylester in einem Verhältnis von 27:73 zugunsten des (*2(1')E,5E*)-Isomers und in einer Gesamtausbeute von 68%.

Zu einer Lösung von 0.25 g (0.34 mmol) (*2(1')Z,5E*)-2-[2'-(2,5-Bis-[(*tert*.butyl-dimethyl-silyl)-oxy]-phenyl)-ethyliden]-6,10-dimethyl-4-phenylsulfonyl-5,9-undecadiensäure-methylester in 9 ml THF wird eine Lösung von PdCl₂/(dppp) (61 mg, 0.10 mmol) in 2.5 ml THF gegeben. Die Reaktionsmischung wird auf 0°C temperiert, innerhalb von 3.5 Stunden erfolgt die Zugabe von 3.4 ml einer 1.0 molaren Lösung von Lithium-triethylborhydrid in THF. Nachdem für weitere 2.5 Stunden bei der angegebenen Temperatur gerührt wurde, wird die Reaktionsmischung mit 35 ml Ether verdünnt und mit jeweils 10 ml 1.0 molarer wässr. NaCN-Lsg. und mit ges. wässr. NaCl-Lsg. gewaschen. Die organische Phase wird über Magnesiumsulfat getrocknet und unter vermindertem Druck eingeengt. Die Reinigung des verbleibenden Rückstands erfolgt säulenchromatographisch (Laufsystem Heptan:Essigester = 10:1). Ausbeute 0.10 g (0.17 mmol, 50%) (2*(1')Z,5E*)-2-[2'-(2,5-Bis-[(*tert*.butyldimethyl-silyl)-oxy]-phenyl)-ethyliden]-6,10-dimethyl-5,9-undecadiensäuremethylester.

Ergebnis: Die Abspaltung der Schutzgruppen erfolgt wie in 24 beschrieben und liefert Ganomycin B.

### Beispiel 28

### Synthese von Ganomycin A

Zur Synthese des als Zwischenprodukt benötigten [(*4E,8E*)-5,9-Dimethyl-10-[(*tert.*butyl-dimethyl-silyl)-oxy]-4,8-decadienyl]-triphenylphosphonium-iodids werden zu einer Lösung von 5.00 g (25.47 mmol) Geranylacetat in 20 ml 95%igem Ethanol 2.83 g (25.47 mmol) Selendioxid gegeben. Die Lösung wird für eine Stunde unter Rückfluss erhitzt. Die erkaltete Lösung wird filtriert und unter vermindertem Druck eingeengt. Der verbleibende Rückstand wird in 20 ml trockenem Dichlormethan aufgenommen, mit 1.16 g (30.56 mmol) Natriumborhydrid versetzt und für 10 Minuten bei Raumtemperatur gerührt. Zur Aufarbeitung werden vorsichtig 20 ml dest. Wasser zugegeben, anschließend wird die org. Phase zuerst mit 10 ml 3%iger wässr. NaHSO₄-Lsg. und dann mit 10 ml dest. Wasser gewaschen. Das, nach der Trocknung über MgSO₄ und Entfernung des Lösungsmittels unter vermindertem Druck, erhaltene 8-Hydroxy-geranylacetat (3.30 g, 15.54 mmol, 61%) wird in 10 ml trockenem DMF aufgenommen, mit 2.81 g (18.65 mmol) *tert*.Butyl-dimethyl-silylchlorid und 2.64 g (38.85 mmol) Imidazol versetzt und für 15 Stunden bei 35°C gerührt. Die gesamte Lösung wird auf 15 ml Eiswasser gegeben und 2x mit je 10 ml Dichlormethan extrahiert. Die vereinigten organischen Phasen werden zuerst mit 10 ml 3%iger wässr. NaHSO₄-Lsg., dann mit 10 ml dest. Wasser gewaschen, über MgSO₄ getrocknet und unter vermindertem Druck eingeengt. Der so erhaltene Rückstand wird in 10 ml 1%iger methanotischer Natriummethanolatlsg. aufgenommen und für 10 Stunden bei Raumtemperatur gerührt. Das nach der Neutralisation der Lösung mit sauren Ionenaustauschern und der Entfernung des Lösungsmittels unter vermindertem Druck in einer Gesamtausbeute von 3.99 g (14.01 mmol) erhaltene 8-[(*Tert*.butyl-dimethylsilyl)oxy]-geraniol wird zu 2 ml frisch destilliertem 2,6-Lutidin gegeben. Die erhaltene Lösung wird bei 0°C langsam zu einer kräftig gerührten Suspension von 0.62 g (14.70 mmol) trockenem LiCl in 7.5 ml DMF getropft. Nach ca. 30 Minuten bildet sich ein weißer, dicklicher Niederschlags aus, der mit 1.13 ml (14.7 mmol) Methansulfonylchlorid versetzt wird. Nach 6 Stunden Rühren bei Raumtemperatur wird die gesamte Lösung auf 50 ml Eiswasser gegeben und 2x mit je 75 ml Ether extrahiert. Die vereinigten organischen Phasen werden zunächst mit 30 ml dest. Wasser, dann mit 30 ml ges. wässr. CuSO₄-Lsg., zuletzt mit 30 ml ges. wässr. NaCl-Lsg gewaschen und mit MgSO₄ getrocknet. Der [(*2E*,*6E*)-2,6-Dimethyl-8-chlor-2,6-octadienyl]-(*tert*.butyl-dimethyl-silyl)-ether wir nach der Entfernung des Lösungsmittels als schwach gelbes Öl in einer Gesamtausbeute von 52% (4.01 g, 13.24 mmol) erhalten.

Zu einer auf -78°C gekühlten Suspension von 1.52 g (8.00 mmol) Kupfer(I)-iodid in 40 ml THF werden unter Rühren langsam 43.2 ml einer 0.7 molaren Lösung von Vinylmagnesiumbromid in THF gegeben. Die Suspension wird 20 Minuten gerührt anschließend auf -25°C erwärmt und bei dieser Temperatur für weitere 35 Minuten gerührt. Zu der so erhaltenen olivgrünen Lösung werden 4.01 g (13.24 mmol) des [(*2E,6E*)-2,6-Dimethyl-8-chlor-2,6-octadienyl]-*tert*.butyl-dimethyl-silyl-ethers gelöst in 2 ml THF tropfenweise zugegeben. Nach 6 Stunden Rühren bei Raumtemperatur werden 150 ml Ether zugegeben und die org. Phase nacheinander mit jeweils 50 ml dest. Wasser, 3%iger wässr. NH₄Cl-Lsg, ges. wässr. CuSO₄-Lsg und ges. wässr. NaCl-Lsg. gewaschen und über MgSO₄ getrocknet. Der nach dem Abdestillieren des Lösungsmittels unter vermindertem Druck erhaltene Rückstand wird säulenchromatographisch gereinigt (Laufsystem Heptan). Ausbeute 3.04 g (10.19 mmol, 77%) [(2E,6E)-2,6-Dimethyl-2,6,9-decatrienyl-]-*tert*.butyl-dimethyl-silyl-ether.

Zu 18 ml einer auf -10°C gekühlten, frisch hergestellten 1.2 molaren Lösung von Disiamylboran in THF werden tropfenweise 3.04 g (10.19 mmol) [(2E,6E)-2,6-Dimethyl-2,6,9-decatrienyl-]-*tert*.butyl-dimethyl-silyl-ether gelöst in 3 ml THF gegeben. Die erhaltene gelbe Lösung wird für 5.5 Stunden kräftig gerührt. Anschließend werden nacheinander 7 ml dest. Wasser, 7 ml 3 molare wässr. NaOH-Lsg. und 7 ml 30%iges wässriges Wasserstoffperoxid zugetropft. Nach 18 Stunden Rühren bei Raumtemperatur werden 190 ml Ether zugegeben, die organische Phase 3x mit je 30 ml dest. Wasser gewaschen und über MgSO₄ getrocknet. Der nach der Entfernung des Lösungsmittels unter vermindertem Druck erhaltene Rückstand wird säulenchromatographisch gereinigt (Laufsystem Heptan:Ether = 20:1). Ausbeute 2.63 g (8.46 mmol, 83%) an (4E,8E)-5,9-Dimethyl-10-[(*tert*.butyl-dimethyl-silyl)-oxy]-4,8-decadien-1-ol.

Zu einer auf 0°C gekühlten Lösung von 2.63 g (8.46 mmol) (4E,8E)-5,9-Dimethyl-10-[(*tert*.butyl-dimethyl-silyl)oxy]-4,8-decadien-1-ol, 2.89 g (11.00 mmol) Triphenylphosphin und 0.80 g (11.55 mmol) Imidazol in 15 ml Ether und 10 ml Acetonitril werden langsam 3.08 g (12.10 mmol) Iod gegeben. Nach 45 Minuten Rühren erfolgt die Zugabe von 150 ml Ether. Die organische Phase wird nacheinander mit je 30 ml ges. wässr. Natriumthiosulfatlösung, ges. wässr: CuSO₄-Lsg und dest. Wasser gewaschen und über MgSO₄ getrocknet. Der nach dem Abdestillieren des Lösungsmittels erhaltene Rückstand wird in 10 ml Benzol aufgenommen, mit 2.47 g (9.36 mmol) Triphenylphosphin versetzt und für 24 Stunden unter Rückfluss erhitzt. Der nach dem Abkühlen zurückbleibende Feststoff wird mit 100 ml Dichlormethan aufgenommen und das Lösungsmittel anschließend unter vermindertem Druck wieder abdestilliert. Das zurückbleibende Öl wird durch Zugabe von trockenem Ether zur Kristallisation gebracht. Ausbeute 4.64 g (6.77 mmol, 80%) des Phosphoniumsalzes.

Zu einer auf -78°C gekühlten Lösung von 2.06 g (3.00 mmol) [(*4E,8E*)-5,9-Dimethyl-10-[(*tert*.butyl-dimethyl-silyl)-oxy]-4,8-decadienyl]-triphenylphosphonium-iodid in 10 ml THF werden langsam 9.2 ml einer 0.65 molaren Lösung von Kalium-bis(trimethylsilyl)amid (6.00 mmol) in Toluol gegeben. Nach 1.5 Stunden Rühren bei der angegebenen Temperatur werden 240 µl (3.20 mmol) Chlorameisensäure-methylester zugegeben. Die Lösung wird für weitere 30 Minuten bei -78°C gerührt, anschließend langsam auf Raumtemperatur erwärmt und nochmals für 1.5 Stunden gerührt. Nach der Zugabe von 0.92 g (2.42 mmol) [2,5-Bis-[(*tert*.butyl-dimethyl-silyl)-oxy]-phenyl]-acetaldehyd, gelöst in 3 ml THF, wird 32 Stunden bei Raumtemperatur gerührt, die Lösung anschließend mit 25 ml Ether verdünnt und nacheinander mit je 10 ml ges. wässr. NH₄Cl-Lsg. und mit dest. Wasser gewaschen. Der nach der Trocknung der organischen Phase über MgSO₄ und dem Abdestillieren des Lösungsmittels verbleibende Rückstand wird säulenchromatographisch gereinigt (Laufsystem Heptan:Essigester = 10:1). Die beiden isomeren (*2(1')Z,5E,9E*)- und (*2(1')E,5E,9E*)-2-[2'-(2,5-Bis-[(*tert.*butyl-dimethyl-silyl)-oxy]-phenyl)-ethyliden]-6,10-dimethyl-11-[(*tert*.butyl-dimethyl-silyl)-oxy]-5,9-undecadien-säuremethyl-ester werden in einem Verhältnis von 14:86 zugunsten des (*2(1')E,5E,9E*)-Isomers und in einer Gesamtausbeute von 54% erhalten.

Die Entschützung der endständigen OH-Funktion der terpenoiden Seitenkette erfolgt indem 2 Äquivalente 50%iger wässriger HF Lösung zu einer auf 0°C gekühlten Lösung von 0.50 g (*2(1')Z,5E,9E*)-2-[2'-(2,5-Bis-[(*tert.*butyldimethyl-silyl)-oxy]-phenyl)-ethyliden]-6,10-dimethyl-11-[(*tert*.butyldimethyl-silyl)-oxy]-5,9-undecadiensäure-methylester in 15 ml Acetonitril gegeben werden. Nach 1 Stunde erfolgt die Zugabe von 10 ml ges. wässr. NaHCO₃-Lsg., das Reaktionsgemisch wird anschließend 3x mit je 10 ml Ether extrahiert, die vereinigten organischen Phasen mit ges. wässr. NaCl-Lsg. gewaschen und über MgSO₄ getrocknet. Die Reinigung des nach dem Abdestillieren des Lösungmittels verbleibenden Rückstands erfolgt säulenchromatographisch (Laufsystem Heptan:Essigester = 6:1). Ausbeute 92% (*5E,9E*)-2-[2'-(2,5-Bis-[(*tert*.butyl-dimethyl-silyl)-oxy]-phenyl)-ethyliden]-6,10-dimethyl-11-hydroxy-5,9-undecadiensäure-methylester.

Ergebnis: Die Abspaltung der anderen Schutzgruppen erfolgt wie in 24 beschrieben und liefert Ganomycin A.

### Beispiel 29

### Synthese von Ganomycin B durch Kopplung von [2,5-Bis-[(tert.butyldimethyl-silyl)-oxy]-phenyl]-acetaldehyd mit (E)-6,10-Dimethyl-undeca-5,9-dien-carbonsäure-methylester

Zu einer Lösung von 0.46 ml (3.30 mmol) Diisopropylamin in 3 ml THF werden bei 0°C tropfenweise 1.39 ml einer 2.33 molaren etherischen Lösung von n-Butyllithium (3.23 mmol) gegeben. Nach 20 Minuten Rühren bei 0°C wird die Lösung auf -78°C abgekühlt. Mittels einer Spritze werden 0.66 g (2.95 mmol) *(E)*-6,10-Dimethyl-undeca-5,9-dien-carbonsäure-methylester,dessen Synthese bereits in Beispiel 25 beschrieben wurde, gelöst in 3 ml THF, zugegeben. Nach 1 Stunde Rühren bei der angegebenen Temperatur erfolgt die Zugabe von 0.92 g (2.42 - mmol) [2,5-Bis-[(*tert*.butyl-dimethyl-silyl)-oxy]-phenyl]-acetaldehyd, gelöst in 3 ml THF. Nach einer weiteren Stunde Rühren bei -78°C ist die Reaktion beendet. Zur Aufarbeitung werden 5 ml ges. wässr. NH₄Cl-Lsg. zugegeben, anschließend wird auf Raumtemperatur erwärmt. Die Reaktionsmischung wird 3x mit je 5 ml Ether extrahiert, die vereinigten organischen Phasen werden 2x mit je 5 ml ges. wässr. NaCl-Lösung. gewaschen und über MgSO₄ getrocknet. Der nach dem Abdestillieren des Lösungsmittels verbleibende Rückstand wird säulenchromatographisch gereinigt (Laufsystem Heptan:Essigester = 10:1). Ausbeute 1.14 g (1.89 mmol, 78%) (*5E*)-2-[2'-(2,5-Bis-[(*tert*.butyl-dimethyl-silyl)-oxy]-phenyl)-1'-hydroxy-ethyl]-6,10-dimethyl-5,9-undecadiensäure-methylester.

Die Wasserabspaltung erfolgt wie in Beispiel 24 beschrieben und liefert die beiden isomeren

(*2(1')Z,5E*)- und (*2(1')E,5E*)-2-[2'-(2,5-Bis-[(*tert*.butyl-dimethyl-silyl)-oxy]-phenyl)-ethyliden]-6,10-dimethyl-5,9-un-decadiensäure-methylester in einem Verhältnis von 20:80 zugunsten des (*2(1')E,5E*)-Isomers und in einer Ausbeute von 83%

### Ergebnis: Die Abspaltung der Schutzgruppen erfolgt wie in 24 beschrieben und liefert Ganomycin B.

### Beispiel 30

### Synthese weiterer Derivate nach Formel 1 durch Variation der Sulfon-Komponente

Methodik: Der gemäß Beispiel 29 dargestellte 4-[2,5-Bis-[(*tert*.butyl-dimethylsilyl)-oxy]-phenyl]-3-acetoxy-2-methyliden-butansäure-methyl-ester kann mit weiteren Sulfonen, z.B. [(*2E,6E*)-3,7-Dimethyl-8-[(*tert*.butyl-dimethyl-silyl)-oxy]-2,6-octadienyl]-phenyl-sulfon, umgesetzt werden.

[(*2E,6E*)-3,7-Dimethyl-8-[(*tert.*butyl-dimethyl-silyl)-oxy]-2,6-octadienyl]-phenyl-sulfon kann wie folgt erhalten werden: Zu einer Aufschlämmung von 2.17 g (13.24 mmol) Natriumbenzolsulfinat in 15 ml DMF gibt man unter Rühren eine Lösung von 4.01 g (13.24 mmol) [(*2E,6E*)-2,6-Dimethyl-8-chlor-2,6-octadienyl]-(*tert*.butyl-dimethyl-silyl)-ether, dessen Synthese bereits in Beispiel 28 beschrieben wurde, in 4 ml DMF. Man erwärmt für 2 Stunden auf 120°C, kühlt ab und versetzt mit 70 g Eiswasser. Die Lösung wird anschließend 3x mit je 15 ml Dichlormethan extrahiert, die vereinigten organischen Phasen zunächst 2x mit je 10 ml ges. wässr. CuSO₄-Lsg., anschließend mit 10 ml dest. Wasser gewaschen und über MgSO₄ getrocknet. Der nach der Entfernung des Lösungsmittels unter vermindertem Druck verbleibende Rückstand wird säulenchromatographisch gereinigt (Laufsystem Heptan:Essigester = 10:1). Ausbeute 3.73 g (9.14 mmol, 69%) [(*2E,6E*)-3,7-Dimethyl-8-[(*tert*.butyl-dimethyl-silyl)-oxy]-2,6-octadienyl]-phenyl-sulfon.

Ergebnis: Die Umsetzung mit [(*2E,6E*)-3,7-Dimethyl-8-[(*tert*.butyl-dimethylsityl)-oxy]-2,6-octadienyl]-phenyl-sulfon, gefolgt von der in den Beispielen 24 und 28 beschrieben Abspaltung der Schutzgruppen, liefert Ganomycin A.

### Beispiel 31

### Aufbau langkettiger Ganomycin-Homologer

Zu einer Lösung von 1.00g (1.66 mmol) (*2(1')Z,5E,9E*)-2-[2'-(2,5-Bis-[(*tert*.butyl-dimethyl-silyl)-oxy]-phenyl)-ethyliden]-6,10-dimethyl-11-hydroxy-5,9-undecadiensäure-methylester (Darstellung siehe Beispiel 28), 0.20 g (4.77 mmol) Lithiumchlorid und 1.12 g (8.20 mmol) Collidin in 12 ml DMF werden bei -3°C tropfenweise 0.4 ml (5.00 mmol) Mesylchlorid gegeben. Die Mischung wird innerhalb einer Stunde auf 3°C erwärmt und dann auf 10 ml ges. wässr. NaHCO₃-Lsg. gegeben. Die so erhaltene Lösung wird 3x mit je 10 ml Hepatan/Essigester (v/v = 1/1) extrahiert. Die vereinigten organischen Phasen werden über MgSO₄ getrocknet und unter vermindertem Druck eingeengt. Der so erhaltene (*2(1')Z,5E,9E*)-2-[2'-(2,5-Bis-[(*tert*.butyl-dimethyl-silyl)-oxy]-phenyl)-ethyliden]-6,10-dimethyl-11-chlor-5,9-undecadiensäure-methylester wird ohne weitere Reinigung umgesetzt.

Zu einer Lösung von 0,61 g (1.50 mmol) [(*2E,6E*)-3,7-Dimethyl-8-[(*tert*.butyldimethyl-silyl)-oxy]-2,6-octadienyl]-phenyl-sulfon (Darstellung siehe Beispiel 30) in THF/1,3-Dimethyl-3,4,5,6-tetrahydro-2(*1H*)-pyrimidinon. (10 ml/3 ml) werden unter Argonatmosphäre bei -78 °C tropfenweise 2.9 ml einer 1.30 molaren Lösung von n-Butyllithium in Hexan (3.76 mmol) gegeben. Die Reaktionsmischung wird für 20 Minuten bei der angegebenen Temperatur gerührt, dann erfolgt innerhalb einer Stunde die tropfenweise Zugabe des *(2(1')Z,5E,9E)*-2-[2'-(2,5-Bis-[(*tert.*butyl-dimethyl-silyl)-oxy]-phenyl)-ethyliden]-6,10-dimethyl-11-chlor-5,9-undecadiensäure-methylesters, gelöst in 4 ml THF. Unter Rühren wird die Lösung innerhalb von 2 Stunden auf -30°C erwärmt, mit 10 ml ges, wässr. NH₄Cl-Lsg. versetzt und 4x mit je 5 ml Ether extrahiert. Die vereinigten organischen Phasen werden mit 5 ml ges. wässr.

NaCl-Lsg. gewaschen und über MgSO₄ getrocknet. Die säulenchromatographische Reinigung (Laufsystem Heptan:Essigester = 10:1) des nach dem Abdestillieren des Lösungsmittels verbleibenden Rückstands liefert

(*2(1')Z,5E,9E,13E,17E*)-2-[2'-(2,5-Bis-[(*tert*.butyl-dimethyl-silyl)-oxy]-phenyl)-ethyliden]-6,10,14,18-tetramethyl-11-phenylsulfonyl-19-[(*tert*.butyldimethyl-silyl)-oxy]-5,9,13,17-nonadeca-tetraensäure-methylester in einer Ausbeute von 66% (1.09 g, 1.09 mmol).

Ergebnis: Die Reduktion der Phenylsulfonylfunktion erfolgt wie in Beispiel 30 beschrieben und liefert den (*2(1')Z,5E,9E,13E,17E*)-2-[2'-(2,5-Bis-[(*tert*.butyldimethyl-silyl)-oxy]-phenyl)-ethyliden]-6,10,14,18-tetramethyl-19-[(*tert*.butyl-dimethyl-silyl)-oxy]-5,9,13,17-nonadeca-tetraensäure-methylester in 48%iger Ausbeute.

Ausgehend von (*2(1')Z,5E,9E,13E,17E*)-2-[2'-(2,5-Bis-[(*tert*.butyl-dimethylsilyl)-oxy]-phenyl)-ethyliden]-6,10,14,18-tetramethyl-19-[(*tert*.butyl-dimethyl-silyl)-oxy]-5,9,13,17-nonadeca-tetraensäure-methylester kann nun die komplette Abspaltung der Schutzgruppen, wie in Beispiel 24 beschrieben, erfolgen.

Ergebnis: Man erhält die (*2(1')Z,5E,9E,13E,17E*)-2-[2'-(2,5-dihydroxyphenyl)-ethyliden]-6,10,14,18-tetramethyl-19-hydroxy-5,9,13,17-nonadecatetraensäure

Eine weitere Möglichkeit besteht in der alleinigen Abspaltung der endständigen *tert*.Butyl-dimethyl-Silylgruppe, entsprechend Beispiel 28.

Ergebnis: Man erhält den (*2(1')Z,5E,9E,13E,17E*)-2-[2'-(2,5-Bis-[(*tert*.butyldimethyl-silyl)-oxy]-phenyl)-ethyliden]-6,10,14,18-tetramethyl-19-hydroxy-5,9,13,17-nonadeca-tetraensäure-methylester.

Dieser kann erneut der in diesem Beispiel beschriebenen Reaktionsfolge: Chlorierung der endständigen OH-Funktion, Umsetzung mit dem Suflonbaustein, Reduktion der Phenyisulfonylfunktion, Abspaltung der Schutzgruppen unterzogen werden.

Ergebnis: Bei Verwendung des [(*2E,6E*)-3,7-Dimethyl-8-[(*tert*.butyl-dimethylsilyl)-oxy]-2,6-octadienyl]-phenyl-sulfons erhält man die (*2(1')Z,5E,9E,13E,17E,21E,25E*)-2-[2'-(2,5-dihydroxy-phenyl)-ethyliden]-6,10,14,18,22,26-hexamethyl-27-hydroxy-5,9,13,17,21,25-heptaicosahexaensäure.

Ergebnis: Bei Verwendung des [(*2E,6E*)-3,7-Dimethyl-2,6-octadienyl]-phenylsulfons erhält man die (*2(1')Z,5E,9E,13E,17E,21E,25E*)-2-[2'-(2,5-dihydroxyphenyl)-ethyliden]-6,10,14,18,22,26-hexamethyl-5,9,13,17,21,25-heptaicosahexaensäure.

Zur weiteren Kettenverlängerung wird der beschriebene Reaktionszyklus erneut durchlaufen.

### Beispiel 32

### Funktionalisierung der endständigen OH-Gruppe von Ganomycin A - Einführung einer Azidfunktion

Methodik: Zu einer Lösung von 0.31 g (0.50 mmol) (*2(1')Z,5E,9E*)-11-Chlor-2-[2'-(2,5-bis-[(*tert*.butyl-dimethyl-silyl)-oxy]-phenyl)-ethyliden]-6,10-dimethyl-5,9-undecadiensäure-methylester in 1.00 ml trockenem DMF werden 36 mg (0.55 mmol) Natriumazid gegeben. Die Lösung wird auf 120°C erhitzt und für 6 Stunden bei dieser Temperatur gerührt. Die erkaltete Lösung wird auf 5 g Eis gegossen und 3x mit je 3 ml Ether extrahiert. Die vereinigten Etherfraktionen werden über Magnesiumsulfat getrocknet, das Lösungsmittel anschließend unter vermindertem Druck abdestilliert. Der verbleibende Rückstand wird säulenchromatographisch gereinigt (Laufsystem Heptan:Essigester = 10:1). Ausbeute 245 mg (0.39 mmol, 78%) (*2(1')Z,5E,9E*)-11-Azido-2-[2'-(2,5-bis-[(*tert*.butyl-dimethyl-silyl)-oxy]-phenyl)-ethyliden]-6,10-dimethyl-5,9-undecadiensäure-methylester.

Ergebnis: Die Abspaltung der Schutzgruppen, wie in 24 beschrieben, liefert die (*2(1')Z,5E,9E*)-11-Azido-2-[2'-(2,5-bis-hydroxy-phenyl)-ethyliden]-6,10-dimethyl-5,9-undecadiensäure.

### Beispiel 33

### Funktionalisierung der endständigen OH-Gruppe von Ganomycin A - Einführung eines Fluoratoms

Methodik: Zu einer Lösung von 0.11 g (0.87 mmol) Dimethylaminotrimethylsilan in 2.5 ml Trichlorfluormethan werden bei -78°C 0.14 ml (1.05 mmol) Diethylaminoschwefeltrifluorid (DAST) gegeben. Nach 10 Minuten Rühren bei dieser Temperatur wird die Lösung auf Raumtemperatur erwärmt und anschließend wieder auf -78°C abgekühlt. Die Zugabe von 301 mg (0.50 mmol) (*2(1')Z,5E,9E*)-2-[2'-(2,5-Bis-[(*tert.* butyl-dimethyl-silyl)-oxy]-phenyl)-ethyliden]-6,10-dimethyl-11-hydroxy-5,9-undecadiensäure-methylester (Darstellung siehe Beispiel 28), gelöst in 1.25 ml Trichlorfluormethan erfolgt mittels einer Spritze. Nach 45 Minuten Rühren wird auf Raumtemperatur erwärmt, die Lösung auf 5 ml Eiswasser gegeben und 3x mit je 5 ml Ether extrahiert. Die vereinigten organischen Phasen werden mit je 10 ml ges. wässr. NaHCO₃-Lsg. und mit ges. wässr. NaCl-Lsg. gewaschen, über MgSO₄ getrocknet und unter vermindertem Druck eingeengt.

Der verbleibende Rückstand wird säulenchromatographisch gereinigt (Laufsystem Heptan:Essigester = 10:1). Ausbeute 127 mg (0.21 mmol, 42%) (*2(1')Z,5E,9E*)-2-[2'-(2,5-Bis-[(*tert*.butyl-dimethyl-silyl)-oxy]-phenyl)-ethyliden]-6,10-dimethyl-11-fluor-5,9-undecadiensäure-methylester.

Ergebnis: Die Abspaltung der Schutzgruppen, wie in Beispiel 23 beschrieben, liefert die (*2(1')Z,5E,9E*)-2-[2'-(2,5-Bis-hydroxy-phenyl)-ethyliden]-6,10-dimethyl-11-fluor-5,9-undecadiensäure.

### Beispiel 34

### Funktionalisierung der endständigen OH-Gruppe von Ganomycin A - Oxidation zur Aldehydfunktion

Methodik: Zu einer unter Argon gerührten Suspension von 0.92 g (10.56 mmol) aktiviertem Mangandioxid in 15 ml Petrolether werden 301 mg (0.50 mmol) (*2(1')Z,5E,9E*)-2-[2'-(2,5-Bis-[(*tert*.butyl-dimethyl-silyl)-oxy]-phenyl)-ethyliden]-6,10-dimethyl-11-hydroxy-5,9-undecadiensäure-methylester (Darstellung siehe Beispiel 28), gelöst in 1.0 ml Petrolether getropft. Nach 8 Stunden intensivem Rührens wird die Lösung durch Kieselgur filtriert, der verbleibende Rückstand mit Ether gewaschen, die vereinigten organischen Phasen über Magnesiumsulfat getrocknet und unter vermindertem Druck eingeengt. Eine weitere Reinigung ist nicht erforderlich. Ausbeute 270 mg (0.45 mmol, 90%) (*2(1')Z,5E,9E*)-2-[2'-(2,5-Bis-[(*tert*.butyl-dimethyl-silyl)-oxy]-phenyl)-ethyliden]-10-formyl-6-methyl-5,9-undecadiensäure-methylester.

Ergebnis: Die Abspaltung der Schutzgruppen, wie in Beispiel 24 beschrieben, liefert die (2*(1')Z,5E,9E*)-2-[2'-(2,5-Bis-hydroxy-phenyl)-ethyliden]-10-formyl-6-methyl-5,9-undecadiensäure.

### Beispiel 35

### Funktionalisierung der endständigen OH-Gruppe von Ganomycin A - Einführung zweier Fluoratome

Methodik: Zu einer Lösung von 0.11 g (0.87 mmol) Dimethylaminotrimethylsilan in 2.5 ml Trichlorfluormethan werden bei -78°C 0,14 ml (1.05 mmol) Diethylaminoschwefeltrifluorid (DAST) gegeben. Nach 10 Minuten Rühren bei dieser Temperatur wird die Lösung auf Raumtemperatur erwärmt und anschließend wieder auf -78°C abgekühlt. Die Zugabe von 300mg (0.50 mmol) (*2(1')Z,5E,9E*)-2-[2'-(2,5-Bis-[(*tert*.butyl-dimethyl-silyl)-oxy]-phenyl)-ethyliden]-10-formyl-6-methyl-5,9-undecadiensäure-methylester (Darstellung siehe Beispiel 32), gelöst in 1.25 ml Trichiorfluormethan erfolgt mittels einer Spritze. Nach 45 Minuten Rühren wird auf Raumtemperatur erwärmt, die Lösung auf 5 ml Eiswasser gegeben und 3x mit je 5 ml Ether extrahiert. Die vereinigten organischen Phasen werden mit je 10 ml ges. wässr. NaHCO₃-Lsg. und mit ges. wässr. NaCl-Lsg. gewaschen, über MgSO₄ getrocknet und unter vermindertem Druck eingegengt.

Der verbleibende Rückstand wird säulenchromatographisch gereinigt (Laufsystem Heptan:Essigester = 10:1). Ausbeute 140 mg (0.23 mmol, 45%) (*2(1')Z,5E,9E*)-2-[2'-(2,5-Bis-[(*tert*.butyl-dimethyl-silyl)-oxy]-phenyl)-ethyliden]-11,11-difluor-6,10-dimethyl-5,9-undecadiensäure-methylester.

Ergebnis: Die Abspaltung der Schutzgruppen, wie in Beispiel 24 beschrieben, liefert die (2*(1')Z,5E,9E*)-2-[2'-(2,5-Bis-hydroxy-phenyl)-ethyliden]- 11,11-difluor-6,10-dimethyl-5,9-undecadiensäure.

### Beispiel 36

### Funktionalisierung der endständigen OH-Gruppe von Ganomycin A - Veretherung der OH-Funktion

Methodik: Eine Lösung von 301 mg (0.50 mmol) (*2(1')Z,5E,9E*)-2-[2'-(2,5-Bis-[(*tert*.butyl-dimethyl-silyl)-oxy]-phenyl)-ethyliden]-6,10-dimethyl-11-hydroxy-5,9-undecadiensäure-methylester (Darstellung siehe Beispiel 28) in 1.0 ml DMF wird unter Argonatmosphäre bei 0°C langsam zu einer Suspension von 20 mg (0.80 mmol) NaH in 0.5 ml DMF gegeben. Die Mischung wird für 30 Minuten bei der angegebenen Temperatur gerührt und dann auf Raumtemperatur erwärmt. Nun werden 0.82 mmol des in der Tabelle 15 angegebenen Halogenids zugegeben. Die Reaktionsmischung wird für 36 Stunden bei Raumtemperatur gerührt, auf 10 ml Eiswasser gegeben und 3x mit je 5 ml Ether extrahiert. Die vereinigten organischen Phasen werden. mit 10 ml ges. wässr. NaCl-Lsg gewaschen, über MgSO₄ getrocknet und unter vermindertem Druck eingeengt. Der verbleibende Rückstand wird säulenchromatographisch gereinigt (Laufsystem Heptan:Essigester = 10:1).

**Tab. 15**

| Reaktionsprodukte und Ausbeuten beim Einsatz verschiedener Halogenide | | |
|---|---|---|
| Halogenid | Ausbeute | Produkt |
| Benzyl-bromid | 70% | (*2(1')Z,5E,9E*)-2-[2'-(2,5-Bis-[(*tert*.butyl-dimethyl-silyl)-oxy]-phenyl)-ethyliden]-6,10-dimethyl-11-phenylmethoxy-5,9-undecadiensäure-methylester |
| Ethyl-bromid | 74% | (2*(1')Z,5E,9E*)-2-[2'-(2,5-Bis-[(*tert*.butyl-dimethyl-silyl)-oxy]-phenyl)-ethyliden]-6,10-dimethyl-11-ethoxy-5,9-undecadiensäure-methylester |
| Methyl-iodid | 80% | (*2(1')Z,5E,9E*)-2-[2'-(2,5-Bis-[(*tert*.butyl-dimethyl-silyl)-oxy]-phenyl)-ethyliden]-6,10-dimethyl-11-methoxy-5,9-undecadiensäure-methylester |

Ergebnis: Die Abspaltung der Schutzgruppen, wie in Beispiel 24 beschrieben, liefert die 11-Phenylmethoxy-, die 11-Ethoxy, die 11-Methoxy- und die 11-(Methyl-2,3,4-tri-O-benzyl-6-desoxy-b-D-glucopyranos-6-yl)-oxy-(*2(1')Z,5E,9E*)-2-[2'-(2,5-Bis-hydroxy-phenyl)-ethyliden]-6,10-dimethyl-5,9-undecadiensäure.

### Beispiel 37

### Funktionalisierung der endständigen OH-Gruppe von Ganomycin A - Einführung einer Aminofunktion

Methodik: Zu einer Lösung von 157 mg (0.25 mmol)(*2(1')Z,5E,9E*)-11-Azido-2-[2'-(2,5-bis-[(*tert.*butyl-dimethyl-silyl)-oxy]-phenyl)-ethyliden]-6,10-dimethyl-5,9-undecadiensäure-methylester 2.5 ml trockenem Pyridin werden 0.13 g (0.50 mmol) Triphenyfphosphin gegeben. Nach etwa 2 Stunden Rühren bei Raumtemperatur wird die Reaktionslösung mit 2.5 ml Acetanhydrid versetzt und für weitere 12 Stunden bei Raumtemperatur gerührt. Der nach dem Abdestillieren des Lösungsmittels unter vermindertem Druck verbleibende Rückstand wird in 5 ml Ether aufgenommen, 2x mit je 2.5 ml 3%iger wässriger Natriumhydrogensulfatlösung und anschließend 2x mit je 2.5 ml dest. Wasser gewaschen. Nach der Trocknung mit Magnesiumsulfat wird das Lösungsmittel unter vermindertem Druck abdestilliert und der verbleibende Rückstand säulenchromatographisch gereinigt (Laufsystem Heptan:Essigester = 10:1). Ausbeute 212 mg (0.33 mmol, 66%) (*2(1')Z,5E,9E*)-11-(N-Acetylamino)-2-[2'-(2,5-bis-[(*tert*.butyl-dimethyl-silyl)-oxy]-phenyl)-ethyliden]-6,10-dimethyl-5,9-undecadiensäure-methylester.

Ergebnis: Die Abspaltung der Schutzgruppen, wie in Beispiel 24 beschrieben, liefert die (*2(1')Z,5E,9E*)-11-(N-Acetyl-amino)-2-[2'-(2,5-Bis-hydroxy-phenyl)-ethyliden]-6,10-dimethyl-5,9-undecadiensäure.

### Beispiel 38

### Umsetzung mit Phthalsäureanhydrid

Methodik: 500 mg (3.38 mmol) Pthalsäureanhydrid werden zusammen mit 301 mg (0.50 mmol) (*2(1')Z,5E,9E)*-2-[2'-(2,5-Bis-[(*tert*.butyl-dimethyl-silyl)-oxy]-phenyl)-ethyliden]-6,10-dimethyl-11-hydroxy-5,9-undecadiensäuremethylester (Darstellung siehe Beispiel 28) in 5 ml Toluol für zwei Stunden unter Rückfluss erhitzt. Die abgekühlte Lösung wird mit je 3 ml ges. wässr. NaHCO3-Lösung und mit ges. wässr. NaCl-Lösung gewaschen, über MgSO₄ getrocknet und unter vermindertem Druck eingeengt. Der verbleibende Rückstand wird säulenchromatographisch gereinigt (Laufsystem Heptan:Essigester = 10:1). Ausbeute 75 mg (0.10 mmol, 20%) des Phtalsäure-monoesters neben 40 mg (0.03 mmol, 12%) des Phtalsäurediesters.

Ergebnis: Die Hydrophilie der Verbindungen nach Formel 1 wird beträchtlich verstärkt.

### Beispiel 39

### Oxidation der Hydrochinone

Methodik: 0,01 mMol Ganomycin B ind 10 ml Methylenchlorid werden mit 0,02 mMol Cer-IV-Ammonium-nitrat (Ammoniumhexanitratocerat (IV) =(NH₄)₂ Ce (NO₃)₆ 3 h unter Rühren bei Raumtemperatur umgesetzt, der Ansatz in 20 ml Wasser gegeben, im Scheidetrichter geschüttelt, die organische Phase abgetrennt und das Lösungsmittel entfernt.

Ergebnis: Die Oxidation der Hydrochinone erweitert die Anwendungsmöglichkeiten der Wirkstoffe nach Formel 1.

### Beispiel 40

### Veresterung mit dem Gyrasehemmer Ofloxacin

Zu einer Lösung von 55 mg (0.15 mmol) Ofloxacin in 0.5 ml trockenem Pyridin werden bei 0°C unter Schutzgasatmosphäre mittels einer Spritze langsam 108 mg (0.38 mmol) Trifluormethansulfon-säureanhydrid zugegeben. Die Lösung wird auf Raumtemperatur erwärmt und für zwei Stunden gerührt. Anschließend erfolgt die Zugabe von 905 mg (1.50 mmol) (*2(1')Z,5E,9E*)-2-[2'-(2,5-Bis-[(*tert*. butyl-dimethyl-silyl)-oxy]-phenyl)-ethyliden]-6,10-dimethyl-11-hydroxy-5,9-undecadiensäure-methylester, dessen Synthese in Beispiel 28 beschrieben wurde, gelöst in 0.5 ml trockenem Pyridin. Die Reaktionslösung wird für weitere 2 Stunden bei Raumtemperatur gerührt und anschließend auf 5 ml Eiswasser gegeben und 3x mit je 5 ml Ether extrahiert. Die vereinigten organischen Phasen werden mit je 5 ml 3%iger wässr. NaHSO₄-Lsg. und mit dest. Wasser gewaschen, über MgSO₄ getrocknet und unter vermindertem Druck eingeengt. Die Auftrennung des erhaltenen Rückstands mittels HPLC (Laufsystem Heptan:Essigester = 5:1) liefert 47 mg (0.05 mmol, 33%) des Ofloxacinesters nach Formelbild 12 und 403 mg (0.67 mmol, 44% bezogen auf die eingesetzte Masse) des nicht umgesetzten (*2(1')Z,5E,9E*)-2-[2'-(2,5-Bis-[(*tert*. butyl-dimethyl-silyl)-oxy]-phenyl)-ethyliden]-6,10-dimethyl-11-hydroxy-5,9-undecadiensäure-methylesters.

### Beispiel 41

### Veresterung mit Penicillin G

Zu einer auf -20°C gekühlten Lösung von 100 mg (0.30 mmol) Penicillin G und 235 mg (0.39 mmol) (*2(1')Z,5E,9E*)-2-[2'-(2,5-Bis-[(*tert*.butyl-dimethyl-silyl)-oxy]-phenyl)-ethyliden]-6,10-dimethyl-11-hydroxy-5,9-undecadien-säuremethylester, dessen Synthese in Beispiel 28 beschrieben wurde, in 1.50 ml trockenem Dichlormethan werden unter Schutzgasatmosphäre 0.13 ml (1.64 mmol) Pyridin and 72 mg (0.39 mmol) Cyanursäurechlorid gegeben. Nach zwei Stunden Rühren bei der angegebenen Temperatur werden 5 ml Dichlormethan und 5 ml 1.0 molare wässr. Schwefelsäure zugegeben, die organische Phase wird abgetrennt, die wässrige noch 2x mit je 5 ml Dichlormethan extrahiert. Die vereinigten organischen Phasen werden mit je 5 ml ges. wässr. NaHCO3-Lsg. und ges. wässr. NaCl-Lösung gewaschen, über MgSO₄ getrocknet und unter vermindertem Druck eingeengt. Die Reinigung des erhattenen

Rückstands mittels HPLC (Laufsystem Heptan:Essigester = 5:1) liefert 47 mg (0.13 mmol, 43%) des "Penicillin G"-esters nach Formelbild 10.

### Beispiel 42

### Wirkstoffe der allgemeinen Formel 1 mit einem Kohlenhydratrest als Substituent R 5

Methodik: Eine Lösung von 301 mg (0.50 mmol) *(2(1')Z,5E,9E*)*-*2-[2'-(2,5-Bis-[(*tert*.butyl-dimethyl-silyl)-oxy]-phenyl)-ethyliden]-6,10-dimethyl-11-hydroxy-5,9-undecadiensäure-methylester (Darstellung nach Beispiel 28) in 1.0 ml DMF wird unter Argonatmosphäre bei 0°C langsam zu einer Suspension von 20 mg (0.80 mmol) NaH in 0.5 ml DMF gegeben. Die Mischung wird für 30 Minuten bei der angegebenen Temperatur gerührt und dann auf Raumtemperatur erwärmt. Nun werden 0.82 mmol Methyl-2,3,4-tri-O-benzyl-6-desoxy-6-iod-b-D-gluco-pyranosid zugegeben. Die Reaktionsmischung wird für 36 Stunden bei Raumtemperatur gerührt, auf 10 ml Eiswasser gegeben und 3x mit je 5 ml Ether extrahiert. Die vereinigten organischen Phasen werden mit 10 ml ges. wässr. NaCl-Lsg gewaschen, über MgSO₄ getrocknet und unter vermindertem Druck eingeengt. Der verbleibende Rückstand wird säulenchromatographisch gereinigt (Laufsystem Heptan:Essigester = 10:1).

Ergebnis: (2*(1')Z,5E,9E*)-2-[2'-(2,5-Bis-[(*tert*.butyl-dimethyl-silyl)-oxy]-phenyl)-ethyliden]-6,10-dimethyl-11-[(methyl-2,3,4-tri-O-benzyl-6-desoxy-b-D-glucopyranos-6-yl)-oxy]-5,9-undecadiensäure in einer Ausbeute von 51%. Die Abspaltung der Schutzgruppen erfolgt wie im Beispiel 24 beschrieben und liefert die 11-(Methyl-2,3,4-tri-O-benzyl-6-desoxy-b-D-glucopyranos-6-yl)-oxy-(*2(1')Z,5E,9E*)-2-[2'-(2,5-Bis-hydroxy-phenyl)-ethyliden]-6,10-dimethyl-5,9-undecadiensäure.

### Beispiel 43

### Veresterung mit Fusidinäure

Methodik: Die Verbindungen der Formel mit R 5 = CH₂OH wird im 1,35 äquimolaren Verhältnis mit Fusidinsäure (20 mM) in einem organischen Lösungsmittel unter dem Einfluss einer Lipase (2,5%; gewonnen aus z.B. *Candida* *rugosa* oder *Mucor meihei,* Sigma Aldrich Chemie GmbH) 6 h bei 35°C umgesetzt. Zur Unterstützung der Umsatzgeschwindigkeit werden die Reaktionslösungen bei 100 U/min geschüttelt. Die Ausbeute an Kondensationsprodukten beträgt 40%.

Ergebnis: Eine Veresterung mit Fusidinsäure bewirkt eine Erhöhung der Oberflächenaktivität und eine Verstärkung der lipophilen Eigenschaften.

### Beispiel 45

### Herstellung neuer antimikrobieller Wirkstoffe durch Kondensation eines Wirkstoffes der allgemeinen Formel 1 mit R1-R3 = H, R 4 = COOH und R5 = CH₃ mit 6-Aminopenicillansäure mittels Biotransformation

Methodik: Die Verbindung der Formel 1 mit R 1-R 3 = H, R 4 = COOH und R5 = CH3 wird im äquimolaren Verhältnis mit 6-Aminopenicillansäure (20 mM) in 0,1 M Natriumphosphatpuffer (pH 6,0) unter dem Einfluss einer Penicillin-Acylase (0,5%; gewonnen aus z.B. *E. coli* Sigma Aldrich Chemie GmbH) 4 h bei 35°C umgesetzt. Zur Unterstützung der Umsatzgeschwindigkeit werden die Reaktionslösungen bei 100 U/min geschüttelt.

Ergebnis: Die Ausbeute an Kondensationsprodukten beträgt 53%.

### Beispiel 46

### Herstellung neuer antimikrobieller Wirkstoffe durch Kondensation der Wirkstoffe der allgemeinen Formel 1 mit R1-R3 = H, R 4 = COOH und R5 = CH₃ mit 7-Amino-cephalosporinsäure mittels Biotransformation

Methodik: Die Verbindung der Formel 1 mit R 1-R 3 = H, R 4 = COOH und R5 = CH3 wird im äquimolaren Verhältnis mit 7-Amino-cephalosporinsäure (20 mM) in 0,1 M Natriumphosphatpuffer (pH 7,0) unter dem Einfluss einer Acylase (0,5%; gewonnen aus *Bacillus megaterium* nach Toyo Jozo Company, 1974 Britisch Pat. 1,347, 665) 0 2 h bei 37°C umgesetzt. Zur Unterstützung der Umsatzgeschwindigkeit werden die Reaktionslösungen bei 100 U/min geschüttelt.

Ergebnis: Die Ausbeute an Kondensationsprodukten beträgt 46%.

### Beispiel 46

### Herstellung neuer antimikrobieller Wirkstoffe durch Kondensation der Wirkstoffe der allgemeinen Formel 1 mit R1-R3 = H, R 4 = COOH und R5 = CH₃ mit 7-Amino-desacetoxycephalosporinsäure mittels Biotransformation

Die Verbindung der Formel 1 mit R 1-R 3 = H, R 4 = COOH und R5 = CH₃ wird im äquimolaren Verhältnis mit 7-Amino-desacetoxycephalosporinsäure (40 mM) in 0,1 M Natriumphosphatpuffer (pH 6,0) unter dem Einfluss einer Acylase (0,5%; gewonnen aus z.B. *E. coli*) 100 min bei 37°C umgesetzt. Zur Unterstützung der Umsatzgeschwindigkeit werden die Reaktionslösungen bei 100 U/min geschüttelt.

Ergebnis: Die Ausbeute an Kondensationsprodukten beträgt 55 und 60%.

### Beispiel 47

### Herstellung neuer antimikrobieller Wirkstoffe durch Kondensation der Wirkstoffe der allgemeinen Formel 1 mit R1-R3 = H, R 4 = COOCH₃ und R5 = CH₃ mit 7-Amino-cephalosporinsäure mittels Biotransformation

Die Herstellung erfolgt nach Beispiel 46. Die Ausbeute beträgt etwa 63%

### Beispiel 48

### Nachweis der Radikalfängereigenschaft der Extrakte mittels Chemilumineszenz

Der Nachweis wurde wie folgt geführt:
A: 30 ml humanes Blut wurden mit 170 ml PBS + Luminol (0,33 mM final) 20 min bei 37°C vorinkubiert.
B: 30 µl humanes Blut wurden mit 170 ml PBS + Luminol (0,33 mM final) und 20 ml Substanzverdünnung 20 min bei 37°C vorinkubiert.

Zu A wurden 20 µl eine 1:100 Verdünnung des Extraktes nach Beispiel 11 und 20 µl Zymosan (10 mg/ml) pipettiert.

Zu B wurden 20 µl Zymosan (10 mg/ml) pipettiert.

Als Kontrolle verwendeten wir PBS statt Zymosan als Stimulator mit und ohne Substanz in den Ansätzen A und B. Nach intensivem Mischen wurde die Kinetik der Lumineszenz über 60 min gemessen. Die Untersuchungen erfolgten an zwei unterschiedlichen Tagen mit Blut von zwei verschiedenen Blutspendern.

Ergebnis: Die Extrakte hemmen eindeutig die durch Luminol induzierte und die spontane Sauerstoffradikalfreisetzung oder fangen die freigesetzten Radikale ab.

### Beispiel 49

### Hemmung der neutralen Endopeptidase

Methodik: Der Nachweis der spezifischen Hemmung der neutralen Endopeptidase erfolgte nach Melzig et. al., Pharmazie 51 (1996) 501-503.

Ergebnis: Durch den Extrakt des Fruchtkörpers mit 80%igem Alkohol wird bei einer Konzentration von 100 µg/ml eine Hemmung um 68%, bei 200 µg/ml um 84% erreicht.

Bei dem entsprechenden ethanolischen Extrakt des Kulturmycels wurde die

Aktivität des Enzyms schon bei einer Konzentration von 50 µg/ml um 70% gehemmt.

Die wässrigen Extrakte aus Fruchtkörper und Mycel sind ebenfalls wirksam. Nach Inkubation mit 50 µg/ml des kaltwässrigen Extraktes wurde eine Hemmung der Enzymaktivität um 65% gefunden.

### Beispiel 50

### Verwendung der Extrakte zur Hemmung der Aktivität von Proteasen

Proteasen sind an zahlreichen posttranslationalen Prozessen der Funktionsregulation des Makroorganismus beteiligt. Bei einer Hemmung der Proteasen sind vielfältige pharmakologische Wirkungen zu erwarten. Der Nachweis einer Enzyminhibition durch wässrige und ethanoloische Extrakte gemäß Beispiel 15 und 16 erfolgte am Beispiel des Angiotensin-Konverting-Enzyms nach Melzig et al., Pharmazie 51 (1996), 501-503.

Ergebnis: Die wässrigen und alkoholischen Extrakte weisen in Konzentrationen von 100-200 µg/ml eine Hemmung des Angiotensin-Konverting Enzyms auf.

### Beispiel 51

### Einfluss auf die serumvermittelte Lipopolysacharid-Bindung

Methodik: Der Test erfolgte nach Grunwald (CD-14 als Rezeptor für Endotoxin: in vitro-Untersuchungen zu Bindungsmodalitäten von LPS und zur Beeinflussung der Expression. Dissertation Ernst-Moritz-Amdt-Universität Greifswald 1993). Für die Testung wurden Zellinien aus Ovarien chinesischer Hamster verwendet, die nach Transfektion des CD 14-Gens den CD 14-Rezeptor exprimieren. Das Lipopolysacharid wurde mit FITC markiert, so dass die Bindung an den Rezeptor durch Messung der Fluoreszenzintensität ermittelt werden konnte.

Ergebnis: Durch ethanolische Extrakte aus Fruchtkörper nach Beispiel 2 und Mycel nach Beispiel 15 in Konzentrationen zwischen 0,1 und 0,2% wurde eine Hemmung der serumvermittelten Lipopylysacharid-Bindung um 40-60% erreicht, ebenso bewirkt der Wirkstoff nach Beispiel 22 und 23 eine Hemmung der Lipopolysacharid-Bindung.

Lipopolysacharide werden bei einer Infektion mit gramnegativen Bakterien aus deren Zellwand freigesetzt. Nach Bindung an das Lipopolysacharid-bindende Protein können sie Makrophagen sowie mononukleäre Zellen zur Freisetzung endogene Mediatoren anregen und so zu Fieber, Veränderungen im weißen Blutbild sowie zur abnormen Weitstellung der Gefäße in der Körperperipherie führen. Im schlimmsten Fall kann es auf Grund der Lipopolysacharid-bindung zum septischen Schock kommen.

Extrakte und daraus isolierte Wirkstoffe, die einerseits auf Grund ihrer antimikrobiellen Eigenschaften die Vermehrung der eingedrungenen Bakterien hemmen und andererseits die Bindung der freigesetzten Lipopolysacharide vermindern, haben daher bei einer Anwendung als Arzneimittel viele Vorteile.

### Beispiel 52

### Verwendung biologisch aktiver Extrakte aus Pilzen der Gattung Ganoderma als Konservierungsmittel für technische Zwecke

Methodik: Ein heißwässriger Extrakt aus Fruchtkörpern von G. pfeifferi wurden in einer Konzentration von 2% zu einer Tensidlösung folgender Zusammensetzung gegeben: Alkylbenzensulfonat 8,0; Alkansulfonat 8,1; Alkylpolyglycolether 3,1; Natriumpolyphosphat 1,0; Parfümöl 0,005; Wasser ad 100. Die Probe und eine entsprechende Kontrolle werden mit S. aureus kontaminiert. Nach Ablauf von 24 h wurde 1 ml entnommen und in 10 ml eines Neutralisierungsgemisches aus Caseinpeptid-Soja-Nährbouillon mit 3% Tween 80. 0,3% Lecithin und 0,1% Cystein gegeben. Anschließend erfolgte die Keimzahlbestimmung auf Caseinpepton-Soja-Agar (Oxoid, Unipath Ltd., Basingstoke, Hampshire, GB).

Ergebnis: Während die Keimzahl bei den nicht konservierten Kontrollen 3,1 x 10⁸ betrug, blieben die konservierten Proben keimfrei.

### Beispiel 53

### Anwendung als vor freien Radikalen schützender vitalisierender und keimmindernder Zusatzstoff für pharmazeutische und kosmetische Zubereitungen

Methodik: Die antimikrobielle Wirksamkeit wurde im Agardiffusionstest nach Beispiel 1, die konservierende Eigenschaft im Konservierungsbelastungstest mit einer Wasser in Öl-Hautpflege-Creme nachgewiesen, die mit S.aureus mit einem Titer 1:10⁶ beimpft wurde.

Der Nachweis der Radikalfängerfunktion wurden entsprechend Beispiel 48 durchgeführt.

Ergebnis: Die alkoholischen Extrakte waren antibakteriell aktiv, bei den mit alkoholischen Extrakten nach Beispiel 17 geschützten Proben wurde kein Keimwachstum nachgewiesen.

Der Zusatz der Hydrochinone verhindert oxidative Zersetzungen und bewirkt aufgrund seiner Radikalfängereigenschaften einen Schutz vor hautschädigenden Radikalbildnern.

Darüber hinaus wirken die Ganoderma-Extrakte entzündungshemmend. Die Kombination dieser Eigenschaften bietet gute Voraussetzungen für eine Anwendung in der kosmetischen Industrie.

### Beispiel 54

### Verwendung von alkoholischen und wässrigen Extrakten aus dem Mycel und aus Fruchtkörpern von G. pfeifferi als vitalisierende, schmerzlindernde und keimmindernde Zusatzstoffe für Lebensmittel

Methodik: Der Schutz der Zellen vor toxischen Radikalen, nachgewiesen nach Beispiel 48, kann auch als ernährungsphysiologisch bedeutsam bei der Supplementierung von Nahrungsmitteln mit Extrakten aus G. pfeifferi angesehen werden. Eine alimentäre Supplementierung wird auch für andere Kofaktoren antioxidativer Systeme wie Vitamin C und E und Spurenelemente zur Entgiftung von Sauerstoffradikalen durchgeführt. Dieser für Extrakte aus G. pfeifferi spezifische Effekt wird sehr vorteilhaft durch die bereits bekannte, auch bei anderen Pilzen der Gattung Ganoderma nachgewiesene Immunstimulation ergänzt. Andererseits kann die spezifisch inhibierende Wirkung der Extrakte aus G. pfeifferi auf die neutrale Endopeptidase zur Schmerzlinderung genutzt werden, da nach sie im Sinne einer Hemmung der Enkephalinase mit dem Abbau von Neuropeptiden und Endorphinen, die an den Opiat-Rezeptoren angreifen, interferieren.

Aufgrund der starken Hemmung der Neutralen Endopeptidase ist zusätzlich eine Hemmung der Adsorption von Viren auf der Zelloberfläche und damit ein antiviraler Effekt zu erwarten.

Ergebnis: Die Kombination von Radikalfängereigenschaften, immunstimulierender Wirkung, schmerzlindernder Wirkung und antimikrobiellen Eigenschaften schafft sehr günstige Voraussetzungen für eine Anwendung als Nahrungsergänzungsstoff.

### Beispiel 55

### Verwendung als Gesundheitspflegemittel und Nahrungsergänzungsstoff

Methodik: Wässrige und alkoholische Extrakte aus Pitzen der Gattung *Ganoderma* hemmen die Cholesterolakkumulation in kultivierten humanen Aortenintimazellen und die Proliferation der Zellen uns schützen zu vor Arteriosklerose. Durch Hemmung der neutralen Endopeptidase und des Angiotensin-Konverting-Enzyms wird in kaskadenartig organisierte Funktionen des Säureorganismus eingegriffen. Durch die im Beispiel 49 nachgewiesene Hemmung der neutralen Endopeptidase Hemmung wird nach Melzig et al., Pharmazie 51 (1996), 501-503 eine Steigerung der renalen Natriumausscheidung und damit eine Blutdrucksenkung bewirkt. Die im Beispiel 50 nachgewiesene Hemmung des Angiotensin-Konverting-Enzyms führt nach Gräfe (Biochemie der Antibiotika, Spektrum Akademischer Verlag, Heidelberg, Berlin New York, 1992) ebenfalls zu einer Blutdrucksenkung.

Ergebnis: Da bei vielen Patienten mit metabolischem Syndrom ein erhöhter Cholesterinspiegel mit einem erhöhten Blutdruck kombiniert ist, werden die bekannten Wirkungen von Pilzen der Gattung Ganoderma bei G. pfeifferi sehr vorteilhaft durch die blutdrucksenkende Wirkung ergänzt.

Durch ethanolische Extrakte aus Fruchtkörper nach Beispiel 2 und Mycel nach Beispiel 7 in Konzentrationen zwischen 0,1 und 0,2% wurde eine Hemmung der serumvermittelten Lipopylysacharid-Bindung um 40-60% erreicht, ebenso bewirkt der Wirkstoff nach Beispiel 22 eine Hemmung der Lipopolysacharid-Bindung.

Lipopolysacharide werden bei einer Infektion mit gramnegativen Bakterien aus deren Zellwand freigesetzt. Nach Bindung an das Lipopolysacharid-bindende Protein können sie Makrophagen sowie mononukleäre Zellen zur Freisetzung endogene Mediatoren anregen und so zu Fieber, Veränderungen im weißen Blutbild sowie zur abnormen Weitstellung der Gefäße in der Körperperipherie führen. Im schlimmsten Fall kann es auf Grund der Lipopolysacharid-bindung zum septischen Schock kommen.

Extrakte und daraus isolierte Wirkstoffe, die einerseits auf Grund ihrer antimikrobiellen Eigenschaften die Vermehrung der eingedrungenen Bakterien hemmen und andererseits die Bindung der freigesetzten Lipopolysacharide vermindern, haben daher bei einer Anwendung als Arzneimittel viele Vorteile.

### Beispiel 56

### Anwendung in der Human- und Veterinärmedizin insbesondere bei der Bekämpfung von Infektionen als alleiniger Wirkstoff sowie in Form von Kombinationspräparaten

Methodik: Auf standardisierte Testplättchen für die Resistenzbestimmung für die handelsüblichen Antibiotika Amikazin, Trimethroprim und Fusidinsäure (Sensi-Disk Antibiotika-Testblättchen, Becton Dickinson Microbiology Systems, Cockeysville, USA) wurden Extrakte nach Beispiel 9 in einer Konzentration von 2 mg/l aufgetropft. Die Bestimmung der antibakteriellen Wirksamkeit erfolgte nach Beispiel 1 an aus Patientenmaterial isolierten multiresistenten Koagulasenegativen Staphylokokken.

Ergebnis: Die erfindungsgemäßen Extrakte, daraus isolierte Wirkstoffe und Kombinationen mit konventionellen Antibitika sind gegen aus Patientenmaterial isolierten Staphylokokken wirksam (Tab. 16).

**Tab.16**

| Antibakterielle Wirkung von Extrakten, daraus isolierten Wirkstoffen und Kombinationen | | | |
|---|---|---|---|
| | Hemmhof (mm) | | |
| | S. epidermidis 473 | S. epidermidis 125 | S. epidermidis 847 |
| Extrakt nach Beispiel 9 | 10 | 10 | 12 |
| Ganomycin B | 20 | 16 | 16 |
| Extrakt nach Beispiel 9 + Amikazin | 18 | 14 | 18 |
| Extrakt nach Beispiel 9 + Trimethroprim | 40 | 30 | 34 |
| Extrakt nach Beispiel 9 + Fusidinsäure | 34 | 28 | 30 |

### Beispiel 57

### Verwendung der substituierten Hydrochinone der allgemeinen Formel 1 in der Human- und Veterinärmedizin als Mittel mit antimikrobieller Wirkung, insbesondere bei multiresistenten grampositiven Keimen

Methodik: Wirkstoffe der allgemeinen Formel 1 mit R₁-R₃ = H, R₄ = COOH, R₅ = CH₃. wurden in Agardiffusionstest nach Beispiel 1 auf Wirksamkeit gegen Erreger grampositiver Infektionen untersucht.

Ergebnis: Sowohl methicilinsensible Staph. aureus-Stämme als auch methicillin.resistente Stämme wie der Norddeutsche Epidemiestamm werden durch Ganonomycin B gehemmt. Ebenfalls gehemmt werden aus Patientenmaterial isolierte multiresistente S. epidermidis und S. haemolyticus Stämme. Auch gegen resiste Enterokokken wurde eine Wirksamkeit beobachtet (Tab. 17).

**Tab. 17**

| Antibakterielle Wirksamkeit gegen Erreger grampositiver Infektionen | |
|---|---|
| Erreger | Hemmhof (mm) |
| S. aureus ATCC 6538 | 30 |
| S. aureus ATCC 25923 | 25 |
| S. aureus ATCC 29213 | 22 |
| S. aureus Referenzstamm "Norddeutscher Stamm" (Smith Kline Beecham) | 16 |
| S. aureus (aus Patientenmaterial isolierte MRSA-Stämme) | 10-16 |
| S. epidermidis (aus Patientenmaterial isolierte multiresistente-Stämme) | 16-20 |
| S. haemolyticus (aus Patientenmaterial isolierte multiresistente-Stämme) | 12-16 |
| Enterokokken (aus Patientenmaterial isolierte multiresistente Stämme) | 10-14 |

Aufgrund der starken Hemmung der Neutralen Endopeptidase ist zusätzlich eine Hemmung der Adsorption von Viren auf der Zelloberfläche und damit ein antiviraler Effekt zu erwarten.

### Beispiel 58

### Verwendung als Mittel gegen fischpathogene Bakterien und zum Einsatz in der Fischzucht

Methodik: Fischbakteriosen stellen ein besonderes wirtschaftliches und ökologisch relevantes Problem dar. Die Untersuchungen erfolgten mit gramnegativen Bakterien, die Fischkrankheiten wie die Rotmaulseuche oder die Rote Pest auslösen. Die Anzucht der vom Staatlichen Fischseuchenbekämfungsdienst Niedersachsen und Fischgesundheitsdienst zur Verfügung gestellten Bakterien erfolgte auf Tryptlcase-Soy-Agar (Sigma Chemical Co., St. Lois, USA). Die Prüfung der Extrakte aus dem Fruchtkörper und Wirkstoffe erfolgte im Agardiffusionstes.

Ergebnis: Besonders gegen die Aeromonas-Arten zeigte sich eine antibakterielle Wirkung. (Tab. 18). Da es sich bei den erfindungsgemäßen Extrakten um Naturstoffe handelt ist mit einer guten ökologischen Verträglichkeit zu rechnen.

**Tab. 18**

| Anwendung in der Fischzucht | | | | |
|---|---|---|---|---|
| Extrakt bzw. Wirkstoff | Hemmhofdurchmesser (mm) | | | |
| | Aeromonas salmonicida subsp Salmonicida 67 A 1 | Aeromonas hydrophila DSM 30019 | Vibrio anguillarum ATCC 1188 | Yersinia ruckeri F 531/82 |
| Dichlormethan-Extrakt | 10 | 12 | - | - |
| Ethanolischer Extrakt | 10 | 10 | - | - |
| Kaltwässriger Extrakt | 9 | 8 | - | - |
| Heißwässriger Extrakt | 13 | 14 | 9 | 10 |
| Ganomycin A | 10 | 15 | 10 | - |
| Ganomycin B | 12 | 17 | 26 | - |

### Beispiel 59

### Verwendung als Radikalfänger

Methodik: Die Radikalfängerfunktion der Wirkstoffe nach Formel 1 Ganomycin A und Ganomycin B erweitert die Möglichkeiten der arzneilichen Verwendung. Der Nachweis ertolgte wie bei der Untersuchung der Extrakte nach Beispiel 48.

Ergebnis: Die Ganomycine A und B hemmen bei einer Konzentration von 10 µ g/ml die durch Luminol induzierte und die spontane Sauerstoffradikalfreisetzung.

Die Kombination von Radikalfängereigenschaften und antimikrobiellen Eigenschaften schafft sehr günstige Voraussetzungen für eine Anwendung.

## Patentansprüche

1. Biologisch aktive Extrakte, erhältlich aus Ganoderma pfeifferi DSM 13239, durch Behandlung mit Lösungsmitteln.

2. Extrakte nach Anspruch 1, erhältlich aus dem Fruchtkörper natürlich vorkommender Pilze der Spezies Ganoderma pfeifferi und/oder aus den Fruchtkörpern von in Pilzfarmen gezüchteter Pilze der Spezies Ganoderma pfeifferi DSM 13239.

3. Extrakte nach Anspruch 1, erhältlich aus dem Mycel der Spezies Ganoderma pfeifferi DSM 13239 nach Anzucht in Fermentern.

4. Triterpene der Zusammensetzung 3,26-Dihydroxy-lanosta-8,24-dien-7-on erhältlich aus den Extrakten gemäß mindestens einem der Ansprüche 1 bis 3.

5. Wirkstoffe mit der Formel 1 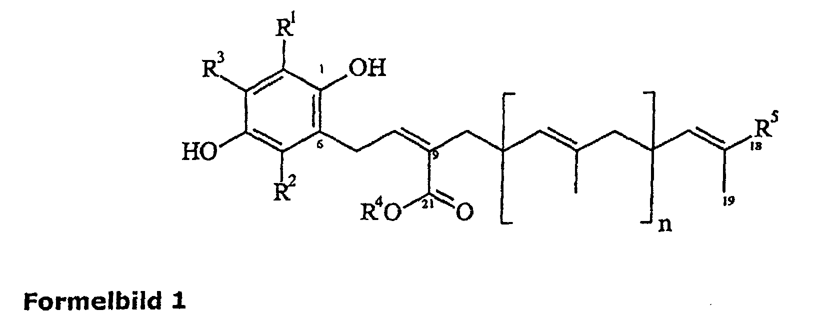 erhältlich aus den Extrakten gemäß mindestens einem der Ansprüche 1 bis 3 mit den Resten R¹-R⁵, wobei R¹, R² und R³ als Wasserstoff, vorliegen, R⁵ als CH₃ und CH₂OH und R⁴ als freie Säure vorliegt, sowie durch chemische Synthese, wobei Derivate erhalten werden, bei denen R¹, R² und R³ als Wasserstoff, Halogen, Alkyl- oder Alkoxygruppe vorliegen, R⁵ als -CH₂-Aryl-, -CH₂-Alkyl-,-CH₂-O-Aryl-, -CH₂-O-Alkyl-, -CH₂-N₃ -CH₂-Carbonsäure-, -CH₂-Aldehyd- oder -CH₂-Alkoholgruppe, -CH₂NR⁹₂, -CX₃, -CHX₂, -CH₂X bzw. -CH₃ oder über Heteroatom mit einem Kohlenhydratderivat verbunden, vorliegt, wobei X = F, Cl, Br und R⁹ ein Wasserstoffatom, ein Alkyl- oder Arylrest sein kann und R⁴ so gewählt wird, dass die freie Säure, ein Säurehalogenid, ein -amid, ein Salz oder ein Ester mit aliphatischen oder aromatischen Alkoholen der Verbindung vorliegt und n eine Zahl von 1 bis 10 annimmt.

6. Verfahren zur Herstellung von neuen substituierten Hydrochinonen und deren Derivaten nach Anspruch 5, **dadurch gekennzeichnet, dass** die Kopplung des mit den Resten R¹-R³ substituierten Hydrochinons an die terpenoide Seitenkette ausgehend von halogensubstituierten Hydrochinonen über metallorganischen Zwischenstufen durch die Reaktion mit einer endständigen Epoxidfunktion der mir R⁴ und R⁵ substituierten terpenoiden Seitenkette erfolgt.

7. Verfahren zur Herstellung von neuen substituierten Hydrochinonen und deren Derivaten nach Anspruch 5, **dadurch gekennzeichnet, dass** die Kopplung des mit den Resten R¹-R³ substituierten Hydrochinons an die terpenoide Seitenkette ausgehend von halomethyl-substituierten Hydrochinonen über metall-organischen Zwischenstufen durch die Reaktion mit einer Carbonylfunktion der mir R⁴ und R⁵ substituierten terpenoiden Seitenkette erfolgt.

8. Verfahren zur Herstellung von neuen substituierten Hydrochinonen und deren Derivaten nach Anspruch 5, **dadurch gekennzeichnet, dass** die Kopplung des substituierten Hydrochinons an die terpenoide Seitenkette ausgehend von mit den Resten R¹-R³ substituierten (2,5-Dihydroxyphenyl)-acetaldehyden durch Umsetzung mit intermediär aus geeigneten mit R⁴ und R⁵ substituierten Terpenoiden erzeugten Carbanionen erfolgt.

9. Verfahren zur Herstellung von neuen substituierten Hydrochinonen und deren Derivaten nach Anspruch 5, **dadurch gekennzeichnet, dass** die Kopplung des mit den Resten R¹-R³ substituierten Hydrochinons an die terpenoide Seitenkette ausgehend von substituierten 4-(2,5-Dihydroxyphenyl)-2-methyliden-carbonsäureestern, welche in 3-Positionen eine elektronenziehende Abgangsgruppe tragen, durch Umsetzung mit intermediär aus mit R5 substituierten Terpenoiden erzeugten Carbanionen erfolgt.

10. Wirkstoffe nach Anspruch 5, die in der Formel 1 mit folgenden Substituenten versehen sind: R¹, R² und R³ = H, F, Cl, Br, I oder n-Alkyl ; R⁴ = Me(I), Me(II), Ammonium, Alkylammonium, Aryl oder Alkyl R⁵ = H, Alkyl-, Aryl-, R'OH, CHO, R'CHO, COOH oder R'COOH (R' = Aryl oder Alkyl).

11. Wirkstoffe nach Anspruch 5 in Form von Mono- und Dihydrochinonether, gemäß Formel 2 und 3, wobei R⁶, R⁷ = Aryl, Alkyl.

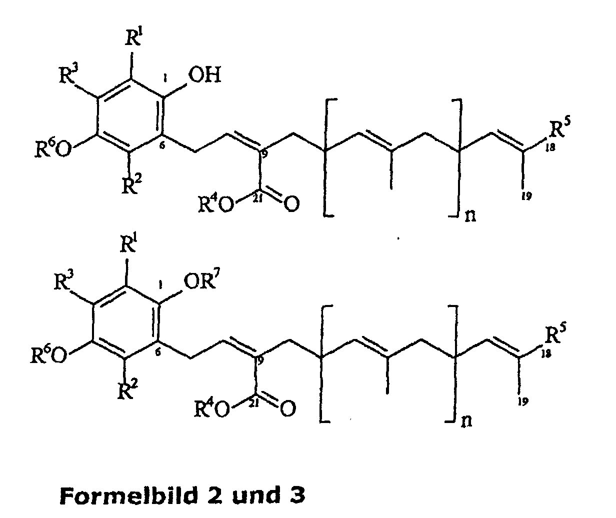

12. Wirkstoffe nach Anspruch 5, **dadurch gekennzeichnet, dass** als Substituenten am C-Atom 18 Säureanhydride, vorzugsweise Phthalsäureanhydrid eingeführt werden.

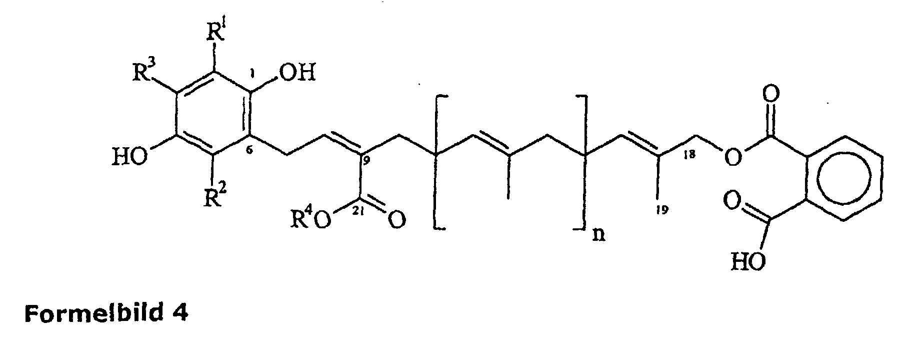

13. Wirkstoffe der allgemeinen Formel 1 bis 12, erhältlich durch Oxidation der Hydrochinone in die Chinone

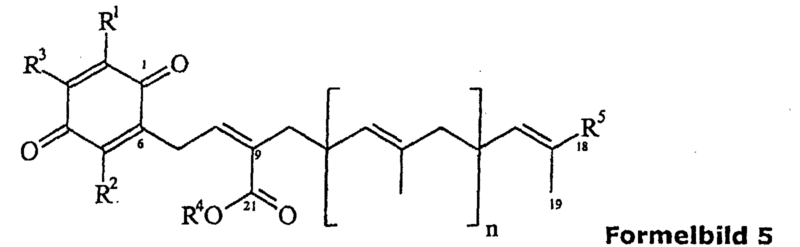

14. Verfahren zur Herstellung neuer substituierter Hydrochinonether nach Anspruch 5, **dadurch gekennzeichnet, dass** die entsprechenden Chinone bzw. Hydrochinone alkyliert oder aryliert werden.

15. Verfahren zur Herstellung neuer antimikrobieller Wirkstoffe nach Formel 5 bis 12, **dadurch gekennzeichnet, dass** Wirkstoffe der allgemeinen Formel 1 durch Veresterung mit Säuregruppen tragenden Antibiotika verbunden werden.

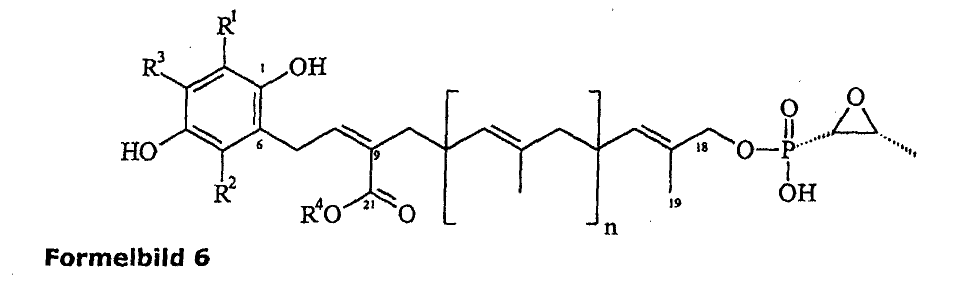

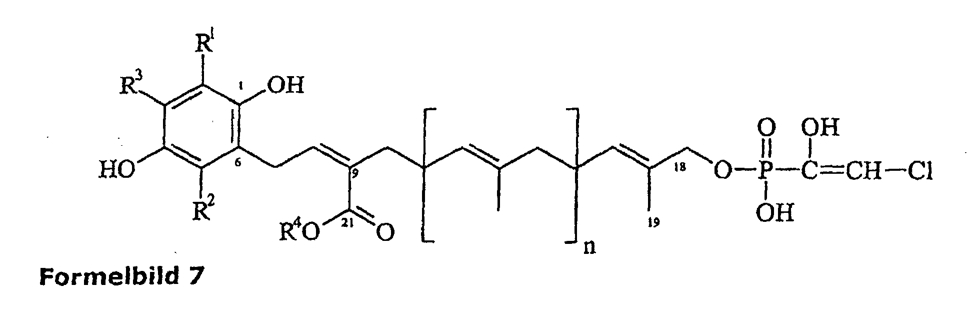

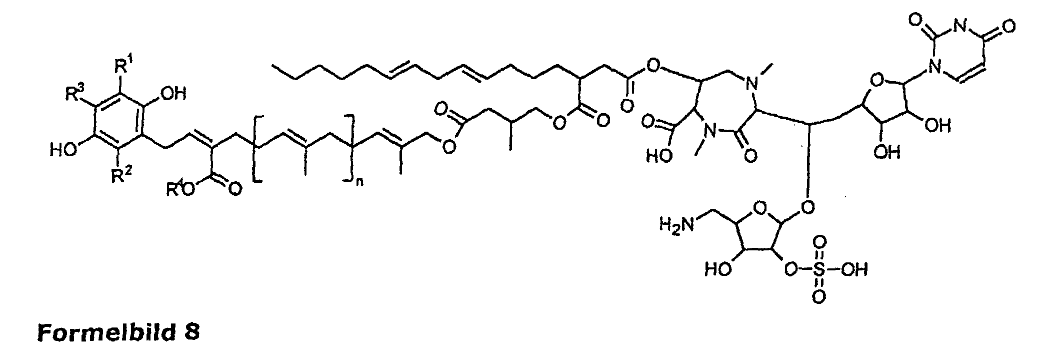

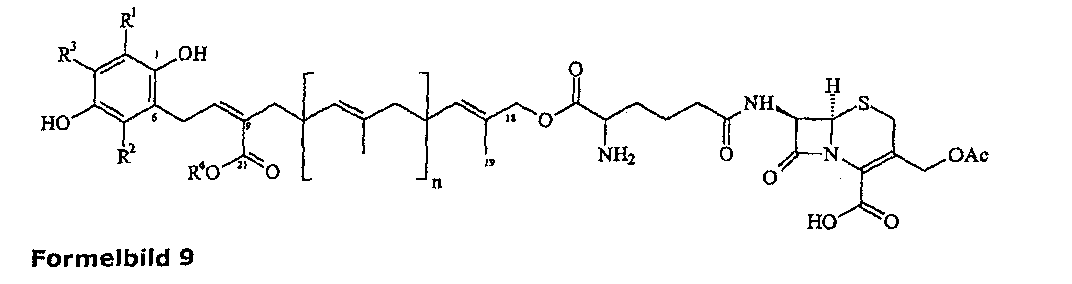

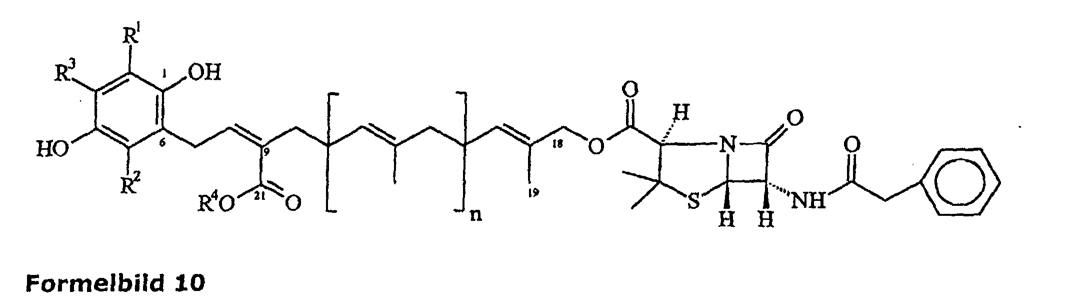

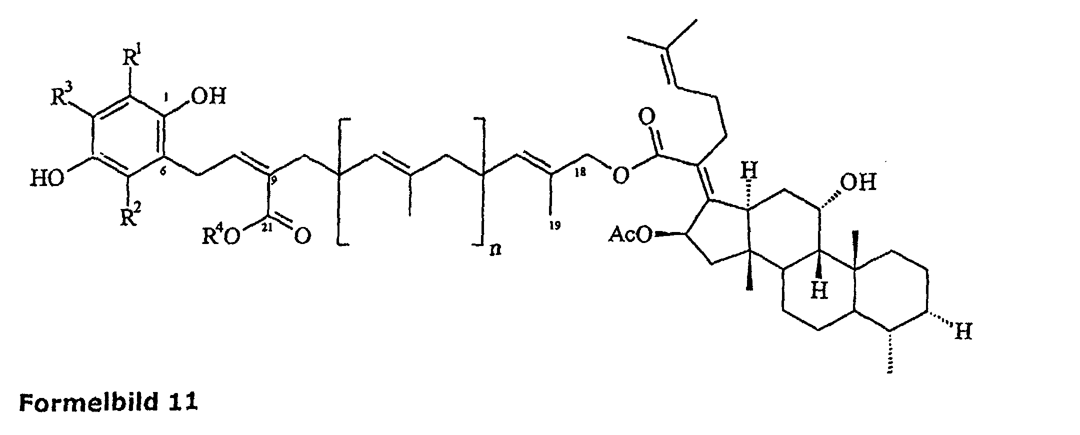

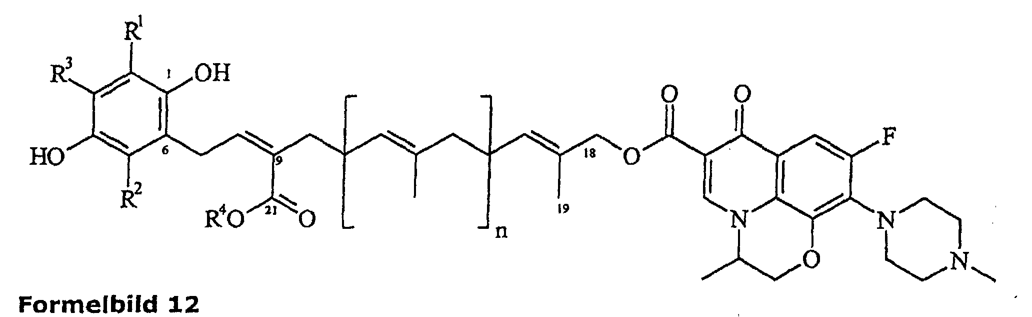

16. Verfahren zur Herstellung neuer antimikrobieller Wirkstoffe, **dadurch gekennzeichnet, dass** Wirkstoffe der allgemeinen Formel 1 durch Kopplung am C-Atom 11 mit Aminogruppen tragenden Antibiotika verbunden werden.

17. Verfahren zur Herstellung neuer antimikrobieller Wirkstoffe nach Formel 5 bis 12, **dadurch gekennzeichnet, dass** Wirkstoffe der allgemeinen Formel 1 mit R⁴ = CH₃ verwendet werden und am C-11 Atom mit Aminogruppen tragenden Antibiotika verbunden werden.

18. Verfahren zur Herstellung neuer antimikrobieller Wirkstoffe nach Formel 1, **dadurch gekennzeichnet, dass** Wirkstoffe der allgemeinen Formel 1 am C-18 über ein Heteroatom mit Kohlenhydratderivaten verbunden werden.

19. Nicht medizinische Verwendung einer oder mehrerer der Verbindungen gemäß Ansprüchen 4, 5, 10 bis 13 und/oder Extrakte gemäß einem der Ansprüche 1 bis 3 als Radikalfänger.

20. Verwendung einer oder mehrerer der Verbindungen gemäß Ansprüchen 4, 5, 10 bis 13 und/oder Extrakte gemäß Anspruch 1 bis 3 zur Herstellung eines Arzneimittels, das als Radikalfänger einsetzbar ist.

21. Nicht medizinische Verwendung einer oder mehrerer der Verbindungen gemäß den Ansprüchen 4, 5, 10 bis 13 und/oder Extrakte gemäß einem der Ansprüche 1 bis 3 zur Hemmung der Aktivität von neutralen Endopeptidasen.

22. Verwendung einer oder mehrerer der Verbindungen gemäß Ansprüchen 4, 5, 10 bis 13 und/oder Extrakte gemäß einem der Ansprüche 1 bis 3 zur Herstellung eines Arzneimittels zur Hemmung der Aktivität von neutralen Endopeptidasen, der Aktivität des Angiotensin-Konverting-Enzyms oder zur Hemmung der serumvermittelten Bindung von Lipopolysachariden.

23. Nicht medizinische Verwendung einer oder mehrerer der Verbindungen gemäß den Ansprüchen 4, 5, 10 bis 13 und/oder Extrakte gemäß einem der Ansprüche 1 bis 3 zur Hemmung der Aktivität des Angiotensin-Konverting-Enzyms.

24. Verwendung biologisch aktiver Extrakte gemäß Ansprüchen 1 bis 3 und/oder Verbindungen gemäß Ansprüchen 4, 5, 10 bis 13 aus Pilzen der Gattung Ganoderma als Substanzen und als Extrakte zur Herstellung eines Arzneimittels mit antimikrobieller Wirksamkeit.

25. Verwendung biologisch aktiver Extrakte gemäß Ansprüchen 1 bis 3 und/oder Verbindungen gemäß Ansprüchen 4, 5, 10 bis 13 aus Pilzen der Gattung Ganoderma als Zusatzstoff in kosmetischen Zubereitungen.

26. Verwendung biologisch aktiver Extrakte gemäß Ansprüchen 1 bis 3 und/oder Verbindungen gemäß Ansprüchen 4, 5, 10 bis 13 aus Pilzen der Gattung Ganoderma als Konservierungsmittel für technische Zwecke oder in pharmazeutischen und kosmetischen Zubereitungen.

27. Verwendung biologisch aktiver Extrakte gemäß Ansprüchen 1 bis 3 und/oder Verbindungen gemäß Ansprüchen 4, 5, 10 bis 13 aus Pilzen der Gattung Ganoderma als vitalisierender und keimmindernder Zusatzstoffe für Lebensmittel und als Nahrungsergänzungsmittel,

28. Verwendung biologisch aktiver Extrakte gemäß Ansprüchen 1 bis 3 und/oder Verbindungen gemäß Ansprüchen 4, 5, 10 bis 13 aus Pilzen der Gattung Ganoderma zur Herstellung eines Gesundheitspflegemittels.

29. Verwendung biologisch aktiver Extrakte gemäß Ansprüchen 1 bis 3 und/oder Verbindungen gemäß Ansprüchen 4, 5, 10 bis 13 aus Pilzen der Gattung Ganoderma zur Herstellung eines Arzneimittels zur Behandlung von Infektionen als alleiniger Wirkstoff sowie in Form von Kombinationspräparaten.

30. Verwendung der substituierten Hydrochinone oder deren Derivaten nach Ansprüchen 4, 5, 10 bis 13 zur Herstellung eines Arzneimittels mit antimikrobieller Wirkung.

31. Verwendung einer oder mehrerer Verbindungen gemäß den Ansprüchen 4, 5, 10 bis 13 und/oder biologisch aktiven Extrakten gemäß Ansprüchen 1 bis 3 zur Herstellung eines Mittels gegen fischpathogene Bakterien und zum Einsatz in der Fischzucht.

32. Arzneimittel enthaltend eine oder mehrere der Verbindungen und/oder Extrakte gemäß einem der Ansprüche 1 bis 5, 10 bis 13.

33. Verwendung einer oder mehrerer der Verbindungen und/oder Extrakte gemäß einem der Ansprüche 1 bis 5, 10 bis 13 zur Herstellung eines Arzneimittels zur Behandlung von Bluthochdruck, Herz- Kreislauferkrankungen und Stoffwechselstörungen.

## Claims

1. Biologically active extracts, obtainable from *Ganoderma pfeifferi* DSM 13239 by treatment thereof with solvents.

2. The extracts according to claim 1, obtainable from the fruit body of naturally occurring fungi of the species *Ganoderma pfeifferi* and/or from the fruit bodies of fungi of the species *Ganoderma pfeifferi* DSM 13239 grown in mushroom farms.

3. The extracts according to claim 1, obtainable from the mycelium of the species *Ganoderma pfeifferi* DSM 13239, wherein the fungi are grown in fermenters.

4. Triterpenes of composition 3,26-dihydroxylanosta-8,24-diene-7-one, obtainable from the extracts according to at least one of claims 1 to 3.

5. Active substances of formula 1 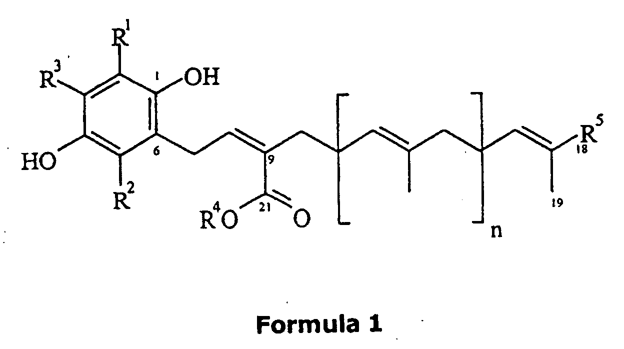 obtainable from the extracts according to at least one of claims 1 to 3, having residues R¹-R⁵, wherein R¹, R² and R³ represent hydrogen, R⁵ represents CH₃ and CH₂OH, and R⁴ is in the form of a free acid, and by chemical synthesis to obtain derivatives in which R¹, R² and R³ represent hydrogen, halogen, alkyl or alkoxy groups, R⁵ represents a -CH₂-aryl, -CH₂-alkyl, -CH₂-O-aryl, -CH₂-O-alkyl, -CH₂N₃, -CH₂-carboxylic acid, -CH₂-aldehyde or -CH₂-alcohol group, -CH₂NR⁹₂, -CX₃, -CHX₂, -CH₂X or -CH₃, or bound to a carbohydrate derivative through a heteroatom, wherein X = F, Cl, Br, and R⁹ is a hydrogen atom, an alkyl or aryl residue, and R⁴ is selected to yield the free acid, an acid halide, amide, a salt or an ester with aliphatic or aromatic alcohols of the compound, and n is a number of from 1 to 10.

6. A process for the preparation of novel substituted hydroquinones and their derivatives according to claim 5, **characterized in that** the coupling of the hydroquinone substituted with residues R¹-R³ to the terpenoid side chain is effected by starting from halogen-substituted hydroquinones via organometallic intermediates through the reaction with a terminal epoxide function of the terpenoid side chain substituted with R⁴ and R⁵.

7. A process for the preparation of novel substituted hydroquinones and their derivatives according to claim 5, **characterized in that** the coupling of the hydroquinone substituted with residues R¹-R³ to the terpenoid side chain is effected by starting from halomethyl-substituted hydroquinones via organometallic intermediates through the reaction with a carbonyl function of the terpenoid side chain substituted with R⁴ and R⁵.

8. A process for the preparation of novel substituted hydroquinones and their derivatives according to claim 5, **characterized in that** the coupling of the substituted hydroquinone to the terpenoid side chain is effected by starting from (2,5-dihydroxyphenyl)acetaldehydes substituted with residues R¹-R³ through reaction with carbanions intermediately produced from suitable terpenoids substituted with R⁴ and R⁵.

9. A process for the preparation of novel substituted hydroquinones and their derivatives according to claim 5, **characterized in that** the coupling of the hydroquinone substituted with residues R¹-R³ to the terpenoid side chain is effected by starting from substituted 4-(2,5-dihydroxyphenyl)-2-methylidenecarboxylic acid esters bearing an electron-withdrawing leaving group in position 3 through reaction with carbanions intermediately produced from suitable terpenoids substituted with R⁵.

10. Active substances according to claim 5, which are provided with the following substituents in formula 1: R¹, R² and R³ = H, F, Cl, Br, I or n-alkyl, R⁴ = MeI, MeII, ammonium, alkylammonium, aryl or alkyl, R⁵ = H, alkyl, aryl, R'OH, CHO, R'CHO, COOH or R'COOH (R' = aryl or alkyl).

11. Active substances according to claim 5 in the form of mono- and dihydroquinone ethers according to formula 2 or 3, 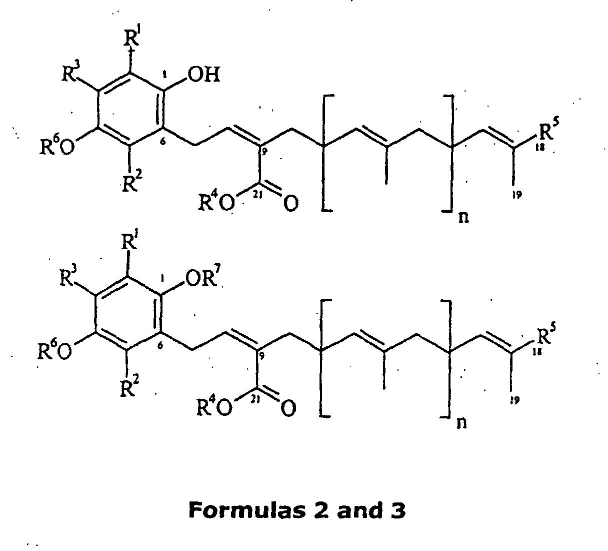 wherein R⁶, R⁷ = aryl, alkyl.

12. Active substances according to claim 5, **characterized in that** acid anhydrides, preferably phthalic anhydride, are introduced as substituents on carbon atom 18:

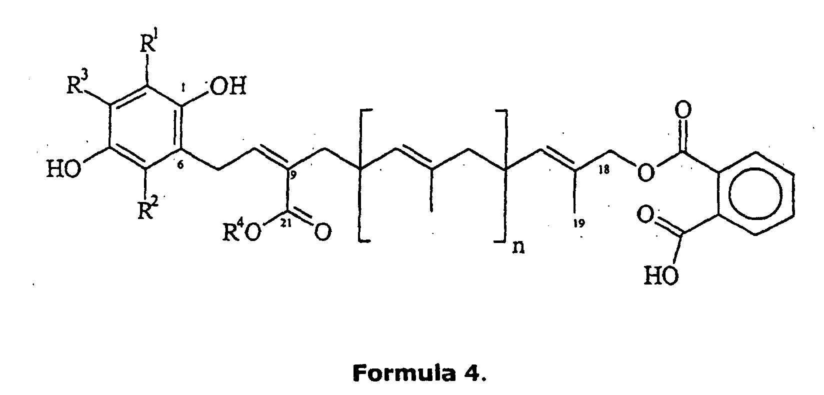

13. Active substances of general formula 1 to 12, obtainable by oxidation of the hydroquinones into the quinones:

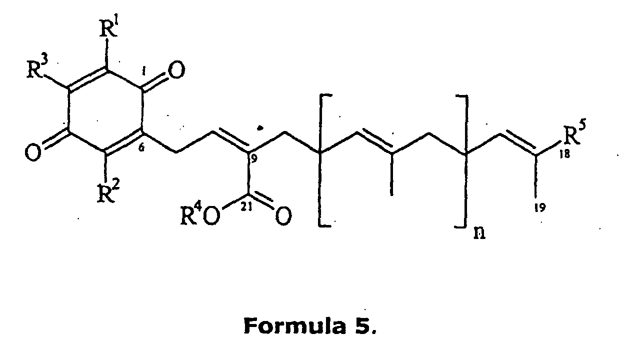

14. A process for the preparation of novel substituted hydroquinone ethers according to claim 5, **characterized in that** the corresponding quinones or hydroquinones are alkylated or arylated.

15. A process for the preparation of novel antimicrobially active substances of formula 5 to 12, **characterized in that** active substances of general formula 1 are linked by esterification with antibiotics bearing acid groups:

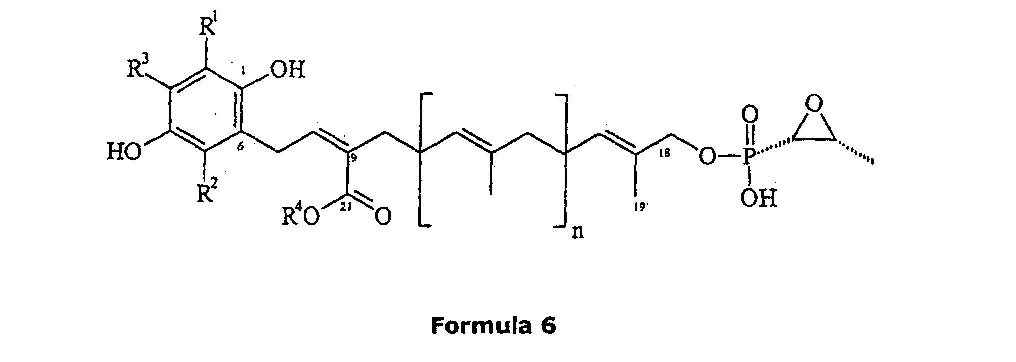

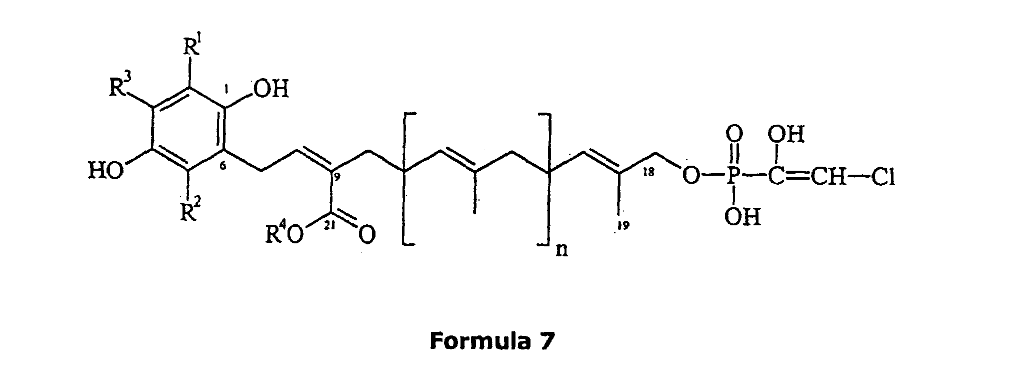

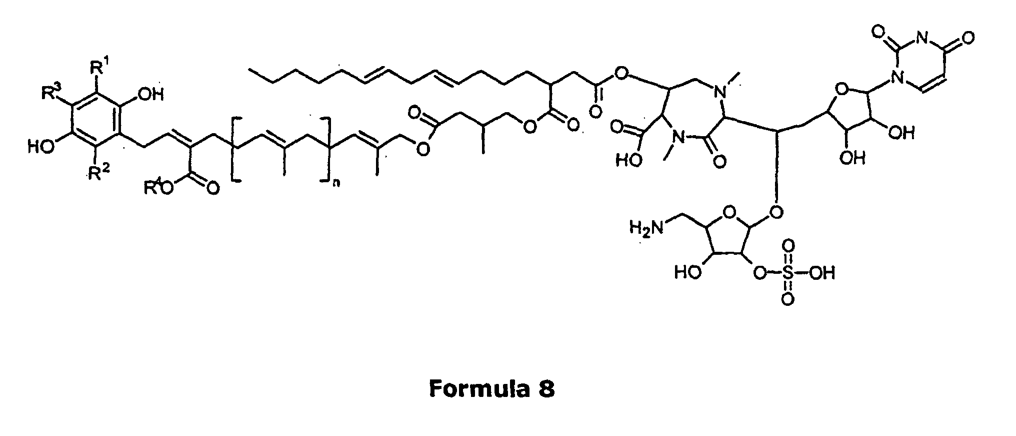

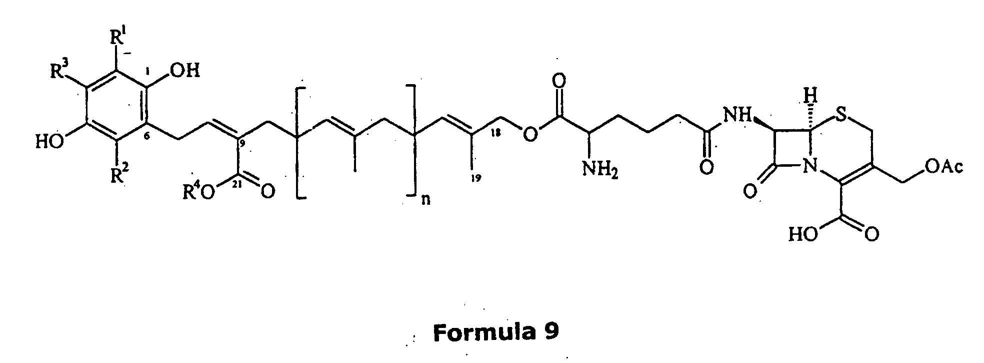

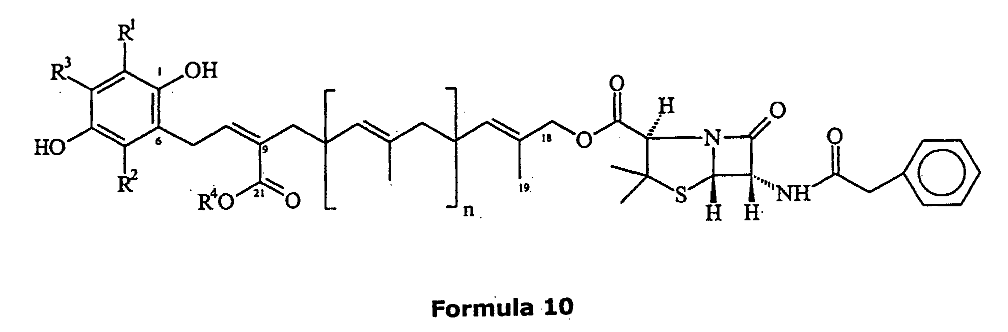

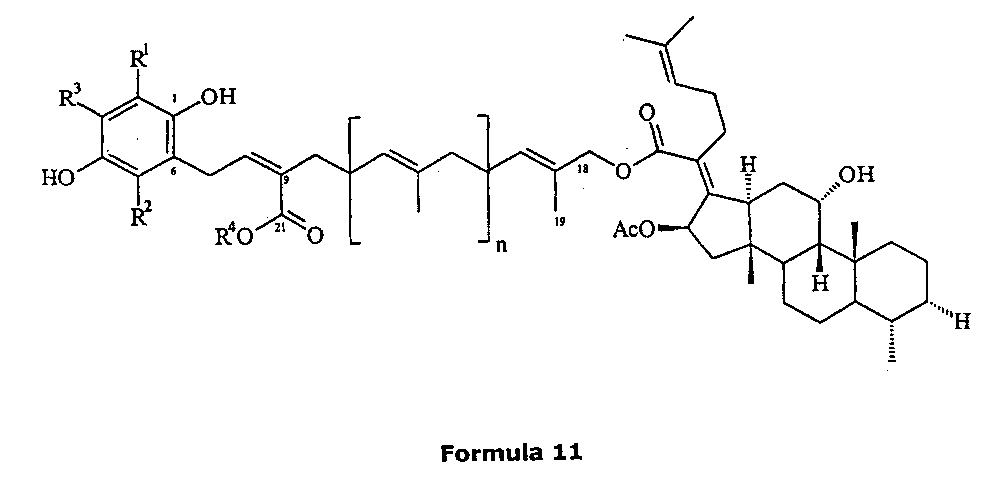

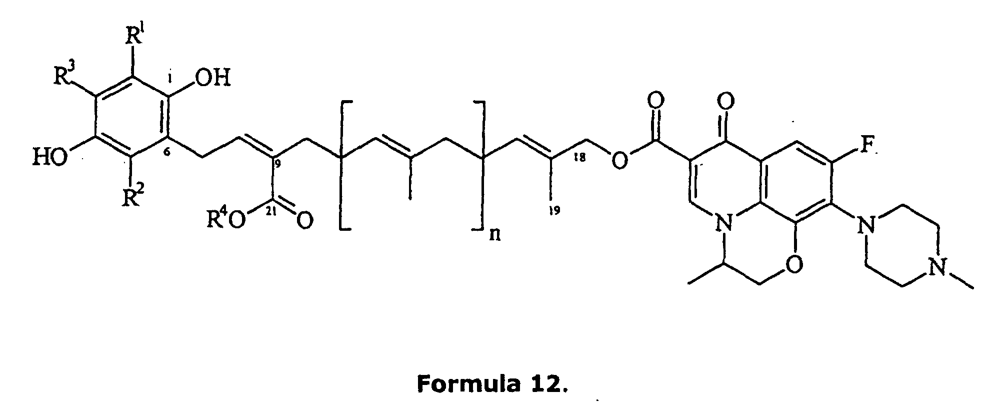

16. A process for the preparation of novel antimicrobially active substances, **characterized in that** active substances of general formula 1 are linked by coupling at carbon atom 11 with antibiotics bearing amino groups.

17. A process for the preparation of novel antimicrobially active substances of formula 5 to 12, **characterized in that** active substances of general formula 1 with R⁴ = CH₃ are used to be linked by coupling at carbon atom 11 with antibiotics bearing amino groups.

18. A process for the preparation of novel antimicrobially active substances of formula 1, **characterized in that** active substances of general formula 1 are bound at C18 to carbohydrate derivatives through a heteroatom.

19. Non-medical use of one or more of the compounds according to claims 4, 5, 10 to 13 and/or extracts according to any of claims 1 to 3 as free-radical scavengers.

20. Use of one or more of the compounds according to claims 4, 5, 10 to 13 and/or of the extracts according to claims 1 to 3 for preparing a medicament which can be employed as a free-radical scavenger.

21. Non-medical use of one or more of the compounds according to claims 4, 5, 10 to 13 and/or extracts according to any of claims 1 to 3 for inhibiting the activity of neutral endopeptidases.

22. Use of one or more of the compounds according to claims 4, 5, 10 to 13 and/or extracts according to any of claims 1 to 3 for preparing a medicament for inhibiting the activity of neutral endopeptidases, the activity of the angiotensin-converting enzyme, or for inhibiting the serum-mediated binding of lipopolysaccharides.

23. Non-medical use of one or more of the compounds according to claims 4, 5, 10 to 13 and/or extracts according to any of claims 1 to 3 for inhibiting the activity of the angiotensin-converting enzyme.

24. Use of biologically active extracts according to claims 1 to 3 and/or compounds according to claims 4, 5, 10 to 13 from fungi of the genus *Ganoderma* as substances and extracts for preparing a medicament having antimicrobial activity.

25. Use of biologically active extracts according to claims 1 to 3 and/or compounds according to claims 4, 5, 10 to 13 from fungi of the genus *Ganoderma* as additives in cosmetic formulations.

26. Use of biologically active extracts according to claims 1 to 3 and/or compounds according to claims 4, 5, 10 to 13 from fungi of the genus *Ganoderma* as a preservative for technical purposes or in pharmaceutical and cosmetic formulations.

27. Use of biologically active extracts according to claims 1 to 3 and/or compounds according to claims 4, 5, 10 to 13 from fungi of the genus *Ganoderma* as vitalizing and germ-reducing additives for foods and as food supplements.

28. Use of biologically active extracts according to claims 1 to 3 and/or compounds according to claims 4, 5, 10 to 13 from fungi of the genus *Ganoderma* for preparing a health care agent.

29. Use of biologically active extracts according to claims 1 to 3 and/or compounds according to claims 4, 5, 10 to 13 from fungi of the genus *Ganoderma* for preparing a medicament for the treatment of infections, as a sole active substance and in the form of combination preparations.

30. Use of the substituted hydroquinones or their derivatives according to claims 4, 5, 10 to 13 for preparing a medicament having antimicrobial activity.

31. Use of one or more compounds according to claims 4, 5, 10 to 13 and/or biologically active extracts according to claims 1 to 3 for preparing an agent against fish-pathogenic bacteria and for use in fish breeding.

32. A medicament containing one or more of the compounds and/or extracts according to any of claims 1 to 5, 10 to 13.

33. Use of one or more of the compounds and/or extracts according to any of claims 1 to 5, 10 to 13 for the preparation of a medicament for the treatment of hypertension, cardiovascular diseases and metabolic disorders.

## Revendications

1. Extraits biologiquement actifs pouvant être obtenus à partir de Ganoderma pfeifferi DSM 13239 par traitement avec des solvants.

2. Extraits selon la revendication 1, pouvant être obtenus à partir de corpuscules reproducteurs de champignons naturels de l'espèce Ganoderma pfeifferi et/ou des corpuscules reproducteurs de champignons, cultivés dans des champignonnières, de l'espèce Ganoderma pfeifferi DSM 13239.

3. Extraits selon la revendication 1, pouvant être obtenus à partir du mycélium de l'espèce Ganoderma pfeifferi DSM 13239, après culture dans des fermenteurs.

4. Triterpènes ayant la composition 3,26-dihydroxy-lanostat-8,24-diène-7-one, pouvant être obtenus à partir des extraits selon au moins l'une des revendications 1 à 3.

5. Principes actifs de formule 1 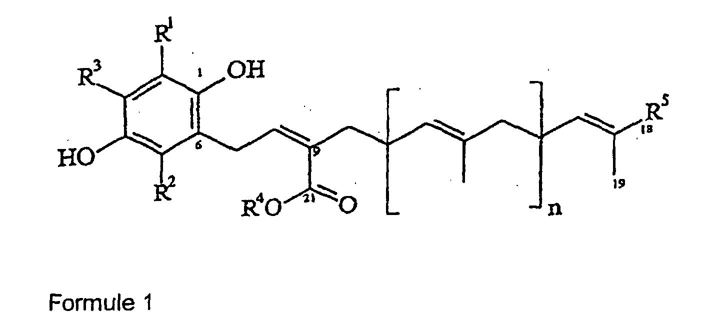 pouvant être obtenus à partir des extraits selon au moins l'une des revendications 1 à 3, portant les radicaux R¹-R⁵, où R¹, R² et R³ sont des atomes d'hydrogène, R⁵ est CH₃ et CH₂OH et R⁴ est un acide libre, et aussi par synthèse chimique, ce qui permet d'obtenir des dérivés dans lesquels R¹, R² et R³ sont des atomes d'hydrogène ou d'halogène ou des groupes alkyle ou alcoxy, R⁵ est un groupe -CH₂-aryle, -CH₂-alkyle, -CH₂-O-aryle, -CH₂-O-alkyle, acide -CH₂N₃-CH₂-carboxylique, -CH₂-aldéhyde ou -CH₂-alcool, -CH₂-NR⁹₂, -CX₃, -CHX₂, -CH₂X ou -CH₃, ou est lié par l'intermédiaire d'un hétéroatome à un dérivé d'hydrate de carbone, où X = F, Cl, Br, et R⁹ peut être un atome d'hydrogène, un groupe alkyle ou aryle, et R⁴ est choisi de façon à être en présence de l'acide libre, d'un halogénure d'acide, d'un amide d'acide, d'un sel ou d'un ester avec des alcools aliphatiques ou aromatiques du composé, n valant 1 à 10.

6. Procédé de préparation de nouvelles hydroquinones substituées et de leurs dérivés selon la revendication 5, **caractérisé en ce que** le couplage de l'hydroquinone substituée par les radicaux R¹-R³ à la chaîne latérale terpénoïde s'effectue, en partant d'hydroquinones halogénées, et en passant par des intermédiaires organométalliques, par la réaction avec une fonction époxyde terminale de la chaîne latérale terpénoïde substituée par R⁴ et R⁵.

7. Procédé de préparation de nouvelles hydroquinones substituées et de leurs dérivés selon la revendication 5, **caractérisé en ce que** le couplage de l'hydroquinone substituée par les radicaux R¹-R³ à la chaîne latérale terpénoïde s'effectue à partir d'hydroquinones halogénées, en passant par des intermédiaires organométalliques, par la réaction avec une fonction carbonyle de la chaîne latérale terpénoïde substituée par R⁴ et R⁵.

8. Procédé de préparation de nouvelles hydroquinones substituées et de leurs dérivés selon la revendication 5, **caractérisé en ce que** le couplage de l'hydroquinone substituée à la chaîne latérale terpénoïde est effectué à partir de (2,5-dihydroxyphényl)-acétaldéhydes substitués par les radicaux R¹-R³, par réaction avec des carbanions, obtenus d'une manière intermédiaire à partir de terpénoïdes appropriés substitués par R⁴ et R⁵.

9. Procédé de préparation de nouvelles hydroquinones substituées et de leurs dérivés selon la revendication 5, **caractérisé en ce que** le couplage de l'hydroquinone substituée par les radicaux R¹-R³ à la chaîne latérale terpénoïde s'effectue à partir d'esters d'acides 4-(2,5-dihydroxyphényl)-2-méthylidène-carboxyliques substitués, qui en position 3 portent un groupe éliminable attracteur d'électrons, par réaction avec des carbanions obtenus d'une manière intermédiaire à partir des terpénoïdes substitués par R⁵.

10. Principes actifs selon la revendication 5, qui dans la formule 1 portent les substituants suivants : R¹, R² et R³ = H, F, Cl, Br, I ou n-alkyle ; R⁴ = Me(I), Me(II), ammonium, alkylammonium, aryle ou alkyle ; R⁵ = H, alkyle, aryle, R'OH, CHO, R'CHO, COOH ou R'COOH (R' = aryle ou alkyle).

11. Principes actifs selon la revendication 5, sous forme de monoéthers et de diéthers de l'hydroquinone selon les formules 2 et 3, avec R⁶, R⁷ = aryle, alkyle.

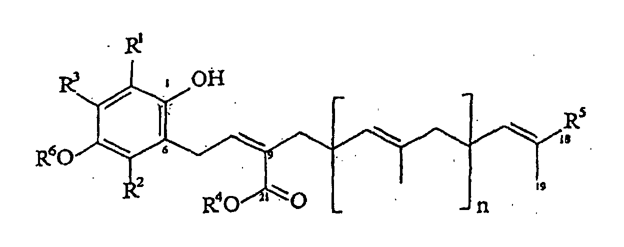

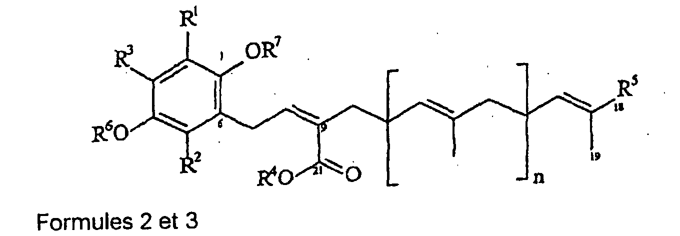

12. Principes actifs selon la revendication 5, **caractérisés en ce qu'**on introduit en tant que substituants sur l'atome de carbone 18 des anhydrides d'acide, de préférence de l'anhydride phtalique.

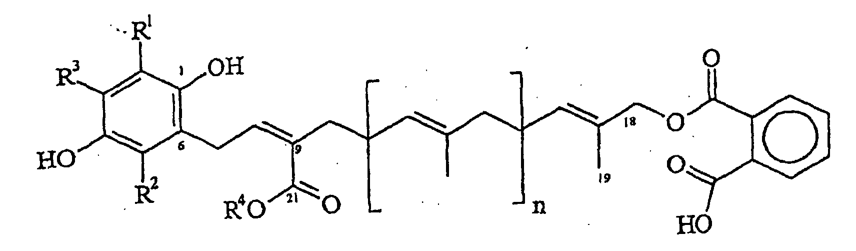

13. Principes actifs ayant les formules générales 1 à 12, pouvant être obtenus par oxydation des hydroquinones en les quinones

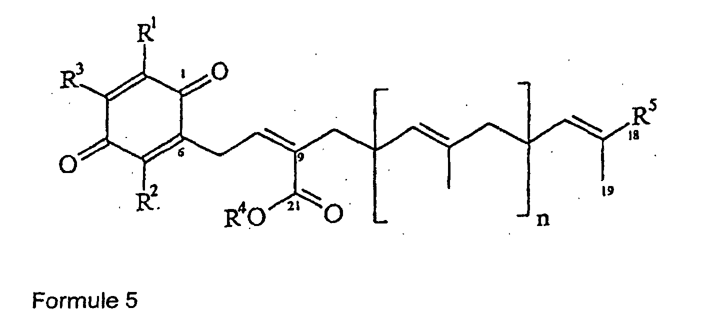

14. Procédé de préparation de nouveaux éthers substitués d'hydroquinone selon la revendication 5, **caractérisé en ce que** les quinones ou hydroquinones correspondantes sont alkylées ou arylées.

15. Procédé de préparation de nouveaux principes actifs antimicrobiens selon les formulas 5 à 12, **caractérisé en ce que** l'on relie des principes actifs de formule générale 1, par estérification, avec des antibiotiques portant des groupes acides.

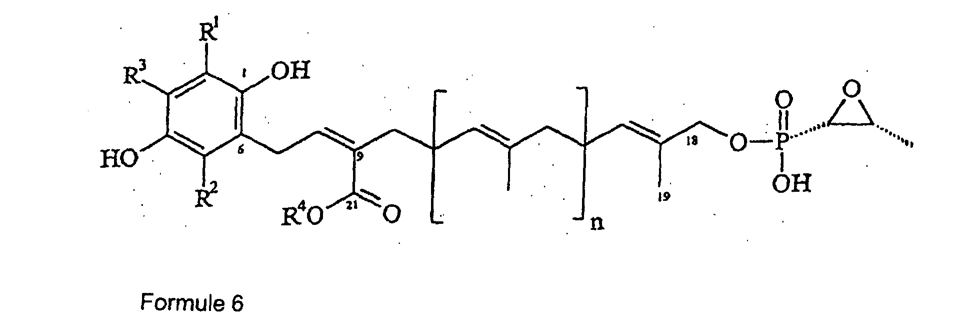

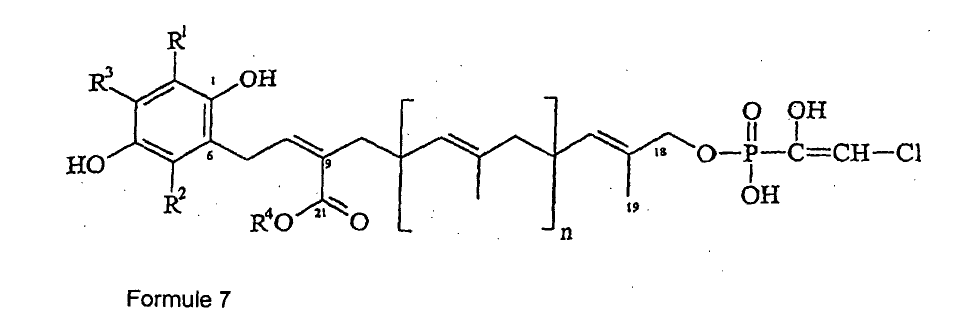

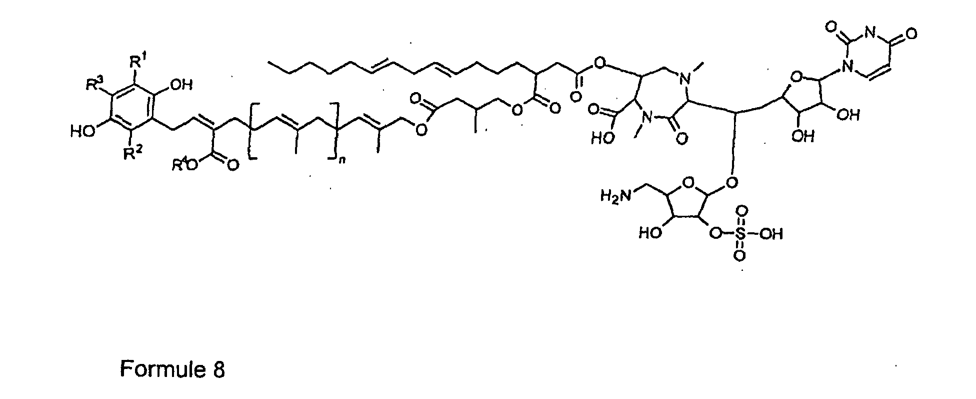

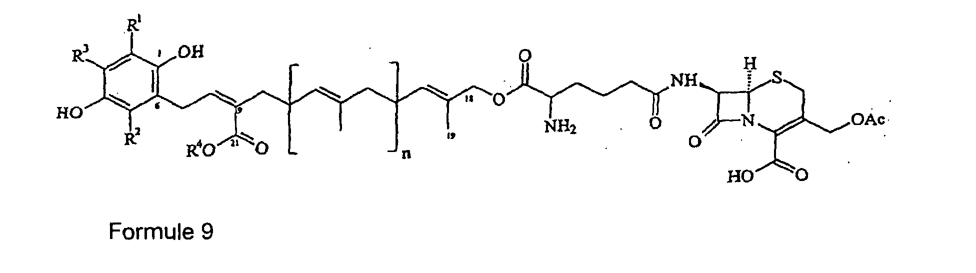

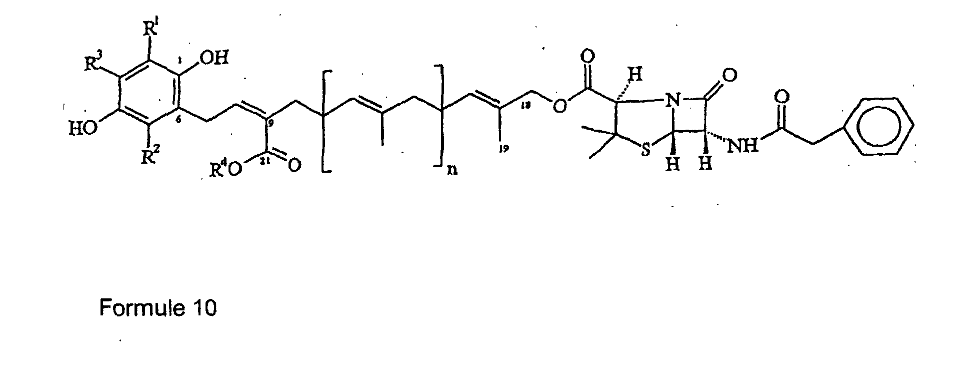

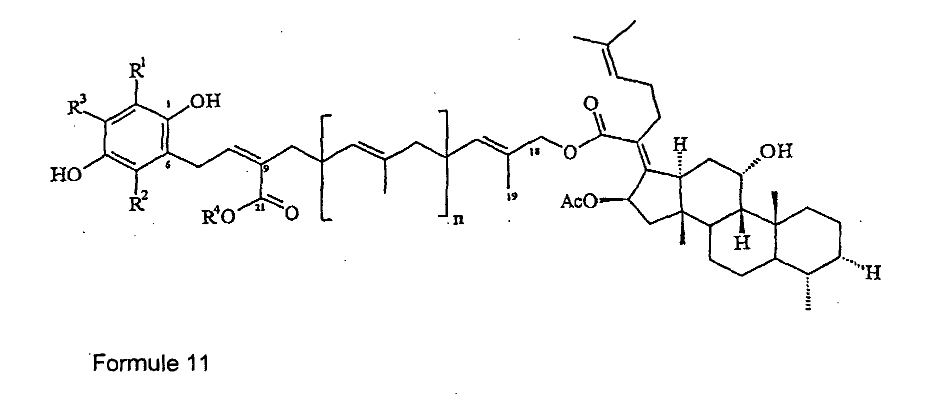

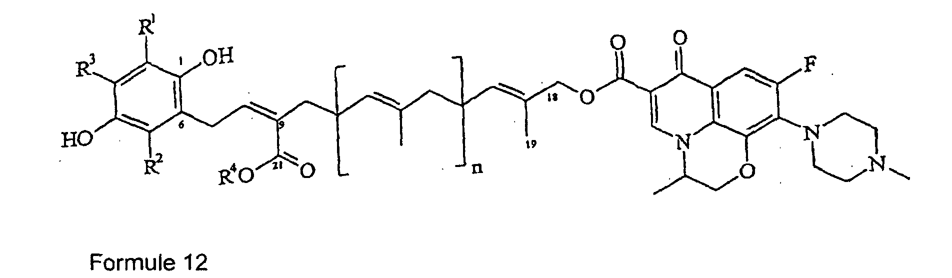

16. Procédé de préparation de nouveaux principes actifs antimicrobiens, **caractérisé en ce qu'**on relie des principes actifs de formule générale 1, par couplage sur l'atome de carbone 11, à des antibiotiques portant des groupes amino.

17. Procédé de préparation de nouveaux principes actifs antimicrobiens selon les revendications 5 à 12, **caractérisé en ce qu'**on utilise des principes actifs de formule générale 1 avec R⁴ = CH₃, et qu'on les relie sur l'atome C-11 à des antibiotiques portant des groupes amino.

18. Procédé de préparation de nouveaux principes actifs antimicrobiens selon la formule 1, **caractérisé en ce qu'**on relie des principes actifs de formule générale 1 sur l'atome C-18, par l'intermédiaire d'un hétéroatome, à des dérivés d'hydrates de carbone

19. Utilisation non médicinale d'un ou plusieurs des composés selon les revendications 4, 5, 10 à 13 et/ou des extraits selon l'une des revendications 1 à 3 en tant que fixateurs de radicaux.

20. Utilisation d'un ou plusieurs des composés selon les revendications 4, 5, 10 à 13 et/ou des extraits selon les revendications 1 à 3 pour préparer un médicament utilisable en tant que fixateur de radicaux.

21. Utilisation non médicinale d'un ou plusieurs des composés selon les revendications 4, 5, 10 à 13 et/ou des extraits selon l'une des revendications 1 à 3 pour inhiber l'activité d'endopeptidases neutres.

22. Utilisation d'un ou plusieurs des composés selon les revendications 4, 5, 10 à 13 et/ou des extraits selon l'une des revendications 1 à 3 pour préparer un médicament destiné à inhiber l'activité d'endopeptidases neutres, l'activité de l'enzyme de conversion de l'angiotensine, ou pour inhiber la liaison, médiée par le sérum, de lipopolysaccharides.

23. Utilisation non médicinale d'un ou plusieurs des composés selon les revendications 4, 5, 10 à 13 et/ou des extraits selon l'une des revendications 1 à 3 pour inhiber l'activité de l'enzyme de conversion de l'angiotensine.

24. Utilisation d'extraits biologiquement actifs selon les revendications 1 à 3 et/ou de composés selon les revendications 4, 5, 10 à 13 provenant de champignons du genre Ganoderma, en tant que substances et en tant qu'extraits pour préparer un médicament ayant une activité antimicrobienne.

25. Utilisation d'extraits biologiquement actifs selon les revendications 1 à 3 et/ou de composés selon les revendications 4, 5, 10 à 13 provenant de champignons du genre Ganoderma, en tant qu'additifs dans des préparations cosmétiques.

26. Utilisation d'extraits biologiquement actifs selon les revendications 1 à 3 et/ou de composés selon les revendications 4, 5, 10 à 13 provenant de champignons du genre Ganoderma, en tant que conservateurs pour des applications techniques ou dans des préparations pharmaceutiques et cosmétiques.

27. Utilisation d'extraits biologiquement actifs selon les revendications 1 à 3 et/ou de composés selon les revendications 4, 5, 10 à 13 provenant de champignons du genre Ganoderma, en tant qu'additifs vitalisants et stérilisants pour produits alimentaires, et en tant que complément alimentaire.

28. Utilisation d'extraits biologiquement actifs selon les revendications 1 à 3 et/ou de composés selon les revendications 4, 5, 10 à 13 provenant de champignons du genre Ganoderma, pour préparer un produit d'hygiène.

29. Utilisation d'extraits biologiquement actifs selon les revendications 1 à 3 et/ou de composés selon les revendications 4, 5, 10 à 13 provenant de champignons du genre Ganoderma, pour préparer un médicament destiné au traitement d'infections, en tant que seul principe actif, et aussi sous forme de préparations en combinaison.

30. Utilisation des hydroquinones substituées ou de leurs dérivés selon les revendications 4, 5, 10 à 13 pour préparer un médicament ayant une activité antimicrobienne.

31. Utilisation d'un ou plusieurs composés selon les revendications 4, 5, 10 à 13 et/ou d'extraits biologiquement actifs selon les revendications 1 à 3 pour préparer un produit contre les bactéries pathogènes vis-à-vis des poissons, et pour utilisation en pisciculture.

32. Médicament contenant un ou plusieurs des composés et/ou extraits selon l'une des revendications 1 à 5, 10 à 13.

33. Utilisation d'un ou plusieurs des composés et/ou extraits selon l'une des revendications 1 à 5, 10 à 13, pour préparer un médicament destiné au traitement de l'hypertension artérielle, des maladies cardiovasculaires et des troubles du métabolisme.
